# EUROPEAN PATENT SPECIFICATION

(11) **EP 4 340 807 B1**
(45) Date of publication and mention of the grant of the patent: **28.01.2026**
(21) Application number: 22729370.1
(22) Date of filing: 17.05.2022
(51) Int. Cl.: A61K 8/87, A61Q 5/06, A61Q 5/10

(54) **HAIR COLOR COMPOSITIONS FOR MITIGATING COLOR LOSS**
HAARFARBEZUSAMMENSETZUNGEN ZUR VERMINDERUNG DES FARBVERLUSTES
COMPOSITIONS DE COLORATION CAPILLAIRE POUR ATTÉNUER LA PERTE DE COULEUR

(30) Priority: 17.05.2021 US 202163189433 P
(43) Date of publication of application: 27.03.2024
(73) Proprietor: Lubrizol Advanced Materials, Inc., Cleveland, OH 44141-3247 (US)
(72) Inventor: ASKAR, Narjis A., Saint Charles, Illinois 60175 (US); LUBNIN, Alexander V., Cleveland, Ohio 44141-3247 (US); DAME, Tina R., Cleveland, Ohio 44141-3247 (US); XAVIER, Jean H., Plainsboro, New Jersey 08536 (US)
(74) Representative: Santas, Jonatan
(86) International application number: PCT/US2022/029534
(87) International publication number: WO 2022/245758

(56) References cited:
- WO-A1-2019/121501
- US-A1- 2006 005 326
- US-A1- 2008 025 933
- US-A1- 2016 175 233
- DATABASE GNPD [online] MINTEL; 8 February 2019 (2019-02-08), ANONYMOUS: "Double Paste Super Hard", XP055961496, retrieved from https://www.gnpd.com/sinatra/recordpage/6328851/ Database accession no. 6328851

## Description

### FIELD OF THE TECHNOLOGY

The present technology relates to hair color compositions for protecting dyed hair from fading or wash-out. More particularly, the technology concerns the use of a structured polymer in the hair color composition for dyed hair color protection against repeated shampoo treatments. In one aspect, the structured polymer comprises a polyurethane backbone with one or more tethered amino groups laterally attached to the polyurethane backbone, wherein the amino groups are tethered away (spaced) from the polymer backbone by at least two intervening atoms.

### BACKGROUND

The coloring of hair has become increasingly popular in recent years. However, fading of artificial hair color has become a common problem and a frequent complaint by consumers. Fading can occur during the shampoo washing treatment as color wash-out, or can be initiated by environmental circumstances, such as by exposure to UV radiation. The washing process is the most significant factor in the removal of hair color, while UV exposure had a significant impact only after 90 hours of intense irradiation. S. Marchioretto, "The Use of Silicones as a Color Lock Aid in Rinse-Off Hair Conditioners", J. of Cosmetic Science, 2003 Annual Scientific Meeting, pp. 130-131. Furthermore, the surfactants present in shampoo formulations provide a wetting function which brings moisture into the hair shaft, thus facilitating the removal of the dye molecules to exit during the water rinsing process.

Maintaining vibrant hair color and minimizing hair color fading is highly desirable in the hair care market. Hair dye is used most frequently when individuals age and wish to hide the gray hair that develops as a result of aging. Colorfastness of a dye can vary widely. There are generally three types of hair color: permanent, demi-permantent, semi-permanent, or temporary. The term "permanent" generally refers to oxidative hair color that bleaches the melanin found in the hair shaft as well as imparting color. In this process, a dye precusor is placed on the hair, and allowed to penetrate the hair shaft and become oxidized, most typically with an oxidizing agent, to produce the desired color in the hair. The dye composition is comprised of two main components: an oxidative dye precusor and a coupler. The oxidative dye precusors and couplers because of their low molecular weights and good water solubility diffuse easily into the hair where a coupling or condensation reaction takes place. The colored products developed by an oxidation agent, remain trapped in the hair by virtue of their higher molecular weights, relative insolubility in water and absorptive affinity to the internal hair surface. The chemical oxidation process is conducted in the presence of a base. The base is an alkaline material that can be, for example, ammonium hydroxide, sodium hydroxide, potassium hydroxide, and calcium hydroxide. It is well established that use of these materials can to some extent be damaging to the hair. Although permanence is the objective of such oxidative dyeing methods, in practice it is difficult to achieve. The color tends to fade over time and a contributory factor in fading is lack of wash-fastness. This means that the color tends to leach out of the hair after repeated washing. This leads to gradual fading or changing of the applied color. Other factors such as ultraviolet light, combing and perspiration also affects the color. Demi-permanent coloring compositions are a class of oxidation dye formulations that are less aggressive than permanent colors.

While oxidative hair color generally lasts from four to six weeks, within two to three weeks after the oxidative procedure, it is common to experience color fading in certain areas of the hair.

Semi-permanent hair color generally provides more lasting color than temporary dyes but without the permanence of oxidative color. The type of dye used in semi-permanent hair color has a larger molecular size and is too large to penetrate the hair shaft of normal virgin hair. However, such larger dye molecules easily penetrate porous and damaged hair where they are preferentially retained due to their larger size. Accordingly, the balancing of the small and large dye molecules found in many semi-permanent products provides uniform color. Semi-permanent hair colorants consist of dye stuffs and acid. The different dy stuffs are acid dyes, basic dyes, metalized dyes, and disperse dyes. However, only a temporary coloring effect will be produced lasting only for several shampoo cycles.

Temporary hair color is often found in a rinse form, and typically lasts for one shampoo. Such hair color is often used when special effects (such as on Halloween and on St. Patrick's Day) are desired. Temporary color is a leave-in formulation formulated with pigments and dyes that simply coat the hair shaft with colorants that are too large to penetrate its outer surface. Temporary hair colorants consist of dye stuffs and acid. The different dye stuffs are acid dyes, basic dyes, metalized dyes and disperse dyes. Minor penetration of the hair shaft may occur in individuals with damaged or porous hair, but such color application rarely lasts through more than one or two shampoo cycles.

As mentioned, the artificial coloring of hair afforded by permanent, demi-permanent, semi-permanent, and temporary hair coloring methodologies can gradually attenuate on repeated washing/shampooing leading over time to fading of the coloration of the hair. The prevention of color fading/leaching is a very important requirement in the hair coloring market today. There is an increasing demand for hair care products designed to prevent or mitigate color fading during washing. Several post (coloring) applied anti-fading products exist in the market including anti-fading treatments, shampoos, and conditioners. While these shampoos and conditioners help to prevent color fade, they are designed to be applied after the hair has been colored with a hair coloring product, thus requiring the consumer to purchase and use additional products to mitigate color loss or fading.

US2006005326 A1 discloses a hair dyeing composition comprising an elastomeric film-forming polymer such as a polyurethane, and a dyestuff, and addresses the need for colouration that withstands outside agents such as shampooing or sweat, while preserving the softness and untangling properties of the hair.

Accordingly, there is a need for a hair care composition that colors the hair and maintains color fastness throughout repeated wash cycles without the need for the use of post coloring anti-fading products. The present technology provides hair coloring compositions with good color fastness towards chemical agents (shampoos, conditioners, waving/straightening agents, etc.) and natural agents (UV, pollution, perspiration, etc.) by introducing into the hair color composition an effective quantity of at least one polyurethane polymer.

### SUMMARY OF THE TECHNOLOGY

In one aspect, the disclosed technology provides a hair color composition that optimizes color intensity and color saturation (e.g., deposition and/or diffusion of color onto and into the hair) resulting in brighter shades of color when applied to the hair.

In one aspect, the disclosed technology provides a hair color composition that has increased fade resistance and reduced wash-out compared to conventionally formulated hair color compositions.

In one aspect, the disclosed technology provides a hair color composition that protects hair color substantibility (e.g., against fading and wash-out) for at least 30 wash cycles.

In one aspect, the disclosed technology provides a hair color composition that maintains or improves the conditioning properties of the hair, including wet and dry feel and tangling resistance.

In one aspect, the hair care composition includes in a cosmetically acceptable carrier at least one color-imparting compound (hair coloring agent) and at least one polymer comprising a polyurethane backbone with one or more tethered tertiary amino groups laterally attached to said polyurethane backbone, wherein said tethered tertiary amino groups are situated away from said polyurethane backbone by a tethering moiety containing at least two intervening atoms.

In one aspect, the hair care composition includes in a cosmetically acceptable carrier at least one color-imparting compound (hair coloring agent) and at least one polymer comprising a polyurethane backbone with one or more tethered tertiary amino groups laterally attached to said polyurethane backbone, wherein said tethered tertiary amino groups are situated away from said polyurethane backbone by a tethering moiety containing at least two intervening atoms, and wherein said tertiary amino groups are neutralized.

In another aspect, the disclosed technology affords a process for coloring hair and retaining color in colored hair. The process includes applying to the hair a composition comprising at least one hair coloring agent and at least one polymer comprising a polyurethane backbone with one or more tethered tertiary amino groups laterally attached to said polyurethane backbone, wherein said tethered tertiary amino groups are situated away from said polyurethane backbone by a tethering moiety containing at least two intervening atoms. The method includes optionally rinsing the composition off of the hair after a contact time.

In still another aspect, the disclosed technology relates to the use of a polymer for imparting color fastness to a hair care coloring composition by introducing into the hair care coloring composition an effective quantity of at least one polyurethane polymer comprising at least one polyurethane backbone with one or more tethered tertiary amino groups laterally attached to said polyurethane backbone, wherein said tethered tertiary amino groups are situated away from said polyurethane backbone by a tethering moiety containing at least two intervening atoms.

In still another aspect, the disclosed technology relates to the use of a polymer for imparting color fastness to a hair care coloring composition by introducing into the hair care coloring composition an effective quantity of at least one polyurethane polymer comprising at least one polyurethane backbone with one or more tethered tertiary amino groups laterally attached to said polyurethane backbone, wherein said tethered tertiary amino groups are situated away from said polyurethane backbone by a tethering moiety containing at least two intervening atoms, and wherein said tertiary amino groups are neutralized.

### DETAILED DESCRIPTION

In all aspects of the disclosed technology all percentages are calculated by the weight of the total composition. All ratios are expressed as weight ratios. All numerical ranges of amounts are inclusive and combinable unless otherwise specified.

The term "cosmetically acceptable" means that the compositions, formulations, or components described for the disclosed technology are suitable for contact with human keratinous tissue (e.g., hair, skin, and nails) without toxicity, incompatability, instability, allergic response. All compositions, formulations, components, and ingredients described herein have the purpose of being directly applied to keratinous tissue are limited to those being cosmetically acceptable.

The term "coloring agent" means a material intended to impart a color to the hair. The coloring agent includes dyes, pigments, dye precursors, dye couplers, direct dyes, and mixtures thereof, and encompasses permanent, semi-permanent and temporary dyes commonly known in the art. Hair coloring agents are disclosed in the CTFA International Color Handbook, 2nd Edition, Micelle Press, England (1992) and Cosmetic Handbook, US Food and Drug Administration, FDA/IAS Booklet (1992).

The term "tethered" amine group refers to a location of the amine group situated away from the main polymer backbone. In such an arrangement, multiple amine groups are connected to the polymer backbone via a spacer. This topology can be represented by the following diagram: 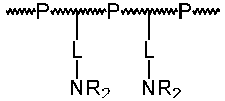 wherein P designates a urethane polymer backbone, L is a link or spacer (a tethering moiety), and NR₂ is a tethered tertiary amine which can be either neutralized with an acid or quaternized. Each R is generally independent of the other an alkyl group (e.g., 1 to 5 carbons atoms, preferably 1 or 2 carbon atoms) or an alkyl amine which can include another tertiary amine group. Each L can be a linking group (substituted, linear, branched, cycloalkyl, aromatic, or combinations thereof which can include urethane linkages, ester linkages, and in addition to carbon can contain heteroatoms such as oxygen and nitrogen). In a preferred and simple embodiment, L is generally ethylene, propylene or other alkylene groups of 2 to 6, preferably 2 to 4, and most preferably 2 or 3 carbon atoms.

While overlapping weight ranges for the various components and ingredients that can be contained in the disclosed compositions have been expressed for selected embodiments and aspects of the disclosed technology, the amount of each component in the disclosed compositions is selected from its disclosed range such that the sum of all components or ingredients in the composition will total 100 weight percent. The amounts employed will vary with the purpose and character of the desired product and can be readily determined by one skilled in the art.

As disclosed herein the hair care compositions contain various conventional additives and adjuvants known in the art, some of which can serve more than one function. For example, a particular component can be listed herein as an emollient but can also function as an emulsifier, humectant, and the like.

The hair care compositions of the disclosed technology may suitably comprise, consist essentially of, or consist of, the components, elements, and process delineations described herein. The disclosed technology illustratively disclosed herein suitably may be practiced in the absence of any element which is not specifically disclosed herein.

Embodiments and aspects described herein may be combinable with other embodiments and/or aspects despite not being expressly exemplified in combination.

Aspects of an exemplary embodiment of the disclosed technology relate to a hair coloring composition and method of use. In one embodiment, an exemplary composition comprises, consists essentionally of, or consists of: a) at least one hair coloring agent; and b) at least one polyurethane comprising a polyurethane backbone with one or more tethered tertiary amino groups laterally attached to said polyurethane backbone, wherein said tethered tertiary amino groups are situated away from said polyurethane backbone by a tethering moiety containing at least two intervening atoms.

In one aspect, the tethered tertiary amino group(s) present on the polyurethane backbone are optionally partially or fully neutralized and/or quaternized.

In one aspect, the hair color composition comprises at least one hair coloring agent (dye) (a). The colorfastness of a dye varies widely. Therefore, dyes used in the coloring of hair are categorized as permanent, semi-permanent, and temporary. selected from permanent dye, a semi-permanent dye, a temporary dye, and mixtures thereof. The type of dye(s) employed in a desired hair coloring formulation is contingent upon the desired class of hair coloring product on offer.

### Permanent Hair Dye

One of the most well known, and widely used coloring applications is the oxidative dyeing process. In this process, the dye is placed on the hair, allowed to penetrate the hair, and become oxidized, most typically with hydrogen peroxide to produce the desired color in the hair. The dye composition is comprised of two main components: oxidative dye precusor and dye coupler. Both components are low molecular weight, which enables them to penetrate the hair and be polymerized in the presence of a base and hydrogen peroxide, to form a final, larger molecular weight dye. The chemical reaction process in the presence of the base and the peroxide is a coupling or a condensation reaction. The base is an alkalizing material that can be, for example, ammonium hydroxide, sodium hydroxide, potassium hydroxide, and calcium hydroxide.

Permanent hair and demi-permanent dyes are generally marketed as two-component kits. One component contains the oxidation dye precursors and dye couplers in an alkaline liquid, gel, or creme base, and the other component is a stabilized solution of the oxidizing agent (e.g., hydrogen peroxide). The two components are mixed immediately prior to use. The mixture is then applied to hair for an appropriate period of time, generally 20 to 60 minutes, where the dye precursors and oxidizing agent diffuse into the hair shaft and following a sequence of chemical reactions color formation takes place.

Oxidative dye precusors are derived from an aromatic compound, e.g, benzene, which is substituted by at least two electron donor groups (e.g., NH₂ and OH) generally situated at the *para* and *ortho* positions of the ring. The oxidative hair dye may be any oxidative hair dye precursor commonly used in oxidative permanent hair dye product. Exemplary oxidative dye precursors include but are not limited to 4-amino-m-cresol, 1-hydroxyethyl-4-5-diaminophyrazole sulfate, N,N, bis [2-hydroxyethyl]-p-phenylene-di-amine sulfate, hydroxyethyl-p-phenylene-di-amine sulfate, p-aminophenol, m-aminophenol, 3-methyl-4-aminophenol, p-methyl amino phenol sulphate, p-phenylenediamine sulfate, tetraamino pyrimidine sulfate, toluene-2-5-diamine sulfate, and mixtures thereof.

In one aspect, the amount of oxidative dye precursors in the permanent hair color composition ranges from about 0.0005 to about 20 wt.%, or from about 0.005 to 10 wt.%, or from about 0.05 to about 6 wt.% based on the total weight of the composition.

Dye couplers are derived from an aromatic compound, e.g., benzene, and contains electron donating groups (e.g., NH₂ and OH) situated in the *meta* position of the ring. The dye coupler may be any dye coupler commonly used in oxidative permanent hair dye product. Exemplary dye couplers include but are not limited to resorcinol, 2-amino-3-hydroxypyridine, 4-amino-2-hydroxytoluene, 2-methyl-5-hydroxyethylaminophenol, 2-methyl resorcinol, 5-amino-6-chloro-o-cresol, 2-amino-4-hydroxyethylamino anisole sulfate, 1-napthol, 2,4,diaminophenoxy ethanol sulfate, m-amino phenol, phenyl methyl pyrazolone, 4-chloro resorcinol, and mixtures thereof.

In one aspect, the amount of dye coupler in the permanent hair color composition is present in an amount ranging from about 0.0001 to about 15 wt.%, or from about 0.0005 to about 10 wt.% based on the total weight of the composition.

In one aspect, the permanent hair dye composition comprises an alkalizing agent. The alkalizing agent may be any alkalizing agent commonly used in oxidative permanent hair dye products. Exemplary alkalizing agents include ammonia, ammonium hydroxide, sodium hydroxide, potassium hydroxide, calcium hydroxide, amino ethyl propanol, ethanolamines (e.g., monoethanolamine, diethanolamine), and mixtures thereof. The alkalizing agent is utilized in an amount sufficient to maintain the pH of the composition in a range of from about 7 to about 12.

In one aspect, the permanent hair dye composition comprises a reducing agent. Reducing agents assist in preventing the premature reaction of the oxidative dye precursors and dye couplers during storage thus enhancing the shelf-life of these oxidative dye components. The reducing agent may be any reducing agent commonly used in oxidative permanent hair dye products. Suitable reducing agents include sodium sulfite, sodium hydrosulfite, ascorbic acid, sodium metabisulfite, and mixtures thereof.

In one aspect, the amount of reducing agent employed ranges from about 0.0005 to about 6 wt.% based on the total weight of the composition.

In one aspect, the permanent hair dye composition comprises an antioxidant. Antioxidants along with the reducing agents mitigate the premature reaction between the oxidative dye precusor and the dye coupler before the addition of the oxidizing agent prior to use. The antioxidant may be any anitoxidant commonly used in oxidative permanent hair dye products. Exemplary antioxidants include sodium sulfite, sodium ascorbate, thioglycolic acid, ammonium thioglycolate, ascorbic acid, erythorbic acid, and mixtures thereof.

In one aspect, the amount of antioxidant utilized ranges from about 0.1 to about 3 wt.%, based on the weight of the composition.

In one aspect, the permanent hair dye composition comprises an oxidizing agent. The oxidizing agent facilitates the reaction between the oxidative dye precursor and dye coupling agent allowing for color development. The oxidizing agent may be any oxidizing agent commonly used in oxidative permanent hair dye products. Exemplary oxidizing agents include organic peroxides (e.g., hydrogen peroxide, urea peroxide, melamine peroxide), inorganic peroxides (e.g., sodium peroxide, sodium periodate, calcium peroxide, barium persilicates, persulfates,sodium bromate, perborate salts, melamin peroxide), alkali metal bromates and ferricyanides, and mixtures thereof.

In one aspect, the amount of oxidizing agent present in the permanent hair color composition ranges from about 0.0001 to about 25 wt.% based on the total weight of the composition.

In one aspect, the permanent hair color composition in addition to the oxidative dye precursor and dye coupler, can also contain other hair dye components. In one aspect the other hair dye component is a direct dye. The direct dye may be any direct dye commonly used in oxidative permanent hair dye products. Exemplary direct dyes include 4-[4'-aminophenyl)-(4"-imino-2",5"-cyclohexadien-1"-yliden) methyl]-2-methylaminobenzene monohydrochloride (C.I. 42 510), 4-[4'-amino-3'-methylphenyl)-(4"-imino-3"-methyl-2",5"-cyclohexadien-1"-yliden)methyl]-2-methylaminobenzene monohydrochloride (C.I. 42 520), 4-hydroxypropylamino-3-nitrophenol, 4-amino-3-nitrophenol, 2-amino-6-chloro-4-nitrophenol, HC Blue 2, HC Yellow 4, HC Red 3, Disperse Violet 4, Disperse Black 9, HC Blue 7, HC Blue 12, HC Yellow 2, HC Yellow 12, Disperse Blue 3, Disperse violet 1, and mixtures thereof.

In one aspect, the amount of direct dye present in the permanent hair color composition ranges from about 0.0001 to about 20 wt.% based on the total weight of the composition.

In one aspect, the permanent hair dye is formulated as a two-component system. Each component is packaged separately and mixed together immediately prior to use. One component (dye component) comprises the oxidative dye precursor, the dye coupling agent, and the alkalizing agent (e.g., ammonia), and the other component (oxidizing component) comprises the oxidizing agent.

The dye component and the oxidizing component can be formulated in any manner commonly used in oxidative permanent hair dye products. In one aspect, the oxidative dye component is formulated in the form of an emulsion, but other vehicles such as gels, pastes, solutions, and powders are utilized. In one aspect, the oxidizing component can be formulated as an aqueous solution or powder form as long as the oxidizing component is compatible for mixing with the dye component. Stabilizers can be used to stabilize the oxidizing component depending on the type of stabilizer employed. Generally, peroxides are inherently unstable, particularly in an aqueous medium. The stabilizer can be any stabilizer commonly used in an oxidative permanent hair dye product. Exemplary stablizers include sodium stannate, pentasodium pentetate, phenacetin and ethylenediamine tetraacetic acid.

The at least one polyurethane having tethered tertiary amino groups which are optionally partially or fully neutralized and/or quaternized can be formulated into the dye component, formulated into the oxidizing component or formulated into both of the dye and oxidizing components of the two-part permanent dye system.

### Semi-permanent Hair Dye

The semi-permanent" hair dyes directly color hair without requiring an oxidation reaction to develop color. The semi-permanent dye may be any dye commonly used in semi-permanent dyeing products. Suitable semi-permanent dyes can be selected from basic dyes, HC dyes, acid dyes, direct dyes, disperse dyes, and mixtures thereof. Other nitro anilines suitable as a semi-permanent dye include 4-hydroxypropylamino-3-nitrophenol and N,N'-bis-(2-hydroxyethyl)-2-nitrophenylenediamine. Mixtures of dyes from any one of the foregoing dye classes can be employed to achieve a desired color or tint.

Suitable basic dyes include blues, browns, greens, oranges, reds, and yellows. Suitable blues include Basic Blue 3, 6, 7, 9, 26, 41, 47, and 99. Suitable browns include Basic Browns 4, 16, and 17. Suitable greens include Basic Green 1 and 4. Suitable oranges include Basic Orange 1 and 2. Suitable Reds include Basic Red 1, 2, 22, 46, 51, 76, and 118. Suitable violets include Basic Violet 1, 3, 4, 10, 11, 14, and 16. Suitable yellows include Basic Yellow 11, 28, and 57.

HC dyes include blue, brown, green, orange, red, violet, and yellow. Suitable blues include HC Blue 2, 4, 5, 6, 7, 8, 9, 10, 11, 12, 13, and 14. Suitable browns include HC Brown 1 and 2. Suitable greens include HC Green 1. Suitable oranges include HC Orange 1, 2, 3, and 5. Suitable reds include HC Red 1, 3, 7, 8, 9, 10, 11, 13, and 14. Suitable violets include HC Violet 1 and 2. Suitable yellows include HC Yellow 2, 4, 5, 6, 7, 8, 9, 10, 11, 13, 14, and 15.

The acid dyes are selected from black, blue, brown, green, orange, red, violet, and yellow. Examples of Acid Black are numbers 1 and 52. Suitable blues include Acid Blue 1, 3, 9, 62, and 74. Examples of browns and greens include Acid Brown 13 and Acid Green 1, 25, and 50, respectively. Suitable oranges include Acid Orange 3, 6, 7, and 24. Suitable reds include Acid Red 14, 18, 27, 33, 35, 51, 52, 73, 87, 92, 95, 184, and 195. Suitable violets include Acid Violet 9 and 43. Suitable yellows include Acid Yellow 1, 3, 23, and 73. Lakes of the foregoing acid dyes are also useful in the present technology.

Suitable direct dyes include Direct Black 51, Direct Blue 86, Direct Red 23, 80, and 81; Direct Violet 48, and Direct Yellow 12.

Suitable disperse dyes include Disperse Black 9, Disperse Blue 1, 3, and 7; Disperse Brown 1, Disperse Orange 3, Disperse Red 11, 15, and 17; and Disperse Violet 1, 4, and 15.

In one aspect, the semi-permanent dye is present in an amount ranging from about 0.0005 to about 20 wt.%, or from about 0.005 to 10 wt.%, or from about 0.05 to about 6 wt.%, based on the total weight of the composition.

The semi-permanent coloring compositions may be formulated in any manner commonly used in semi-permanent hair dye products. In one aspect, the semi-permanent dye composition is formulated in the form of lotions, shampoos, mousses, and emulsions. These product forms must have a viscosity so that they do not flow during application. The hair dyeing process is relatively simple requiring a contact time from about 5 to about 40 minutes, followed by rinsing.

While a good number of the semi-permanent dyes have good water solubility, select nitro aniline derived dyes may be less soluble in aqueous media and may require formulation with specific solvents such as benzyl alcohol, glycol or a glycol derivative.

In one aspect, the at least one polyurethane having tethered tertiary amino groups which are optionally partially or fully neutralized and/or quaternized can be formulated directly into the semi-permanent dye composition.

### Temporary Hair Dye

Temporary hair colorants include color rinses, which provide color that lasts until the first shampooing. Ingredients which impart temporary color generally have a fairly high molecular weight and are unable to penetrate the hair shaft. These materials are simply deposited onto the hair fiber and are removed by subsequent shampooing. Traditionally, temporary hair coloring compositions are used when a cosmetic effect is desired for one day.

The temporary hair coloring agent may be any dye commonly used in temporary dyeing products. In one aspect, the termporary coloring agent is selected from at least one pigment. As used herein, the term "pigment" means any pigment that imparts a color to the hair.

The at least one pigment that may be used can be chosen from the organic and/or mineral pigments known in the art, such as those described in Kirk-Othmer's Encyclopaedia of Chemical Technology and in Ullmann's Encyclopaedia of Industrial Chemistry.

The at least one pigment may be in the form of powder or of pigmentary paste. The pigment may be coated or uncoated.

The at least one pigment may be chosen, for example, from mineral pigments, organic pigments, lakes, pigments with special effects such as nacres or glitter flakes, and mixtures thereof.

The at least one pigment may be a mineral pigment. As used herein, the term "mineral pigment" means any pigment that satisfies the definition in Ullimann's encyclopedia in the chapter on inorganic pigments. The mineral pigments that may be useful in the present disclosure include iron oxides, chromium oxides, manganese violet, ultramarine blue, chromium hydrate, and ferric blue.

Examples colored mineral pigments include red pigments of red oxide, iron oxide, iron hydroxide, and iron titanate, inorganic brown pigments such as γ-iron oxide, inorganic yellow pigments such as yellow iron oxide and yellow soil, inorganic black pigments such as black iron oxide and carbon black, inorganic violet pigments such as manganese violet and cobalt violet, inorganic green pigments such as chromium hydroxide, chromium oxide, cobalt oxide, and cobalt titanate, inorganic blue pigments such as iron blue and ultramarine blue, pigments obtained by flaking tar-based dyes such as red No. 3, red No. 104, red No. 106, red No. 201, red No. 202, red No. 204, red No. 205, red No. 220, red No. 226, red No. 227, red No. 228, red No. 230, red No. 401, red No. 505, yellow No. 4, yellow No. 5, yellow No. 202, yellow No. 203, yellow No. 204, yellow No. 401, blue No. 1, blue No. 2, blue No. 201, blue No. 404, green No. 3, green No. 201, green No. 204, green No. 205, orange No. 201, orange No. 203, orange No. 204, orange No. 206, and orange No. 207, ones obtained by flaking natural pigments such as carminic acid, laccaic acid, carthamin, and brazilin, and the like, titanium oxide-coated mica, titanated mica, iron oxide-treated titanated mica, titanium oxide-coated mica, bismuth oxychloride, titanium oxide-coated bismuth oxychloride, titanium oxide-coated talc, titanium oxide-coated colored mica, and metal powder pigments such as aluminum, gold, silver, copper, platinum, and stainless steel; and mixtures thereof.

The at least one pigment may be an organic pigment. As used herein, the term "organic pigment" means any pigment that satisfies the definition in Ullmann's encyclopedia in the chapter on organic pigments. For instance, the at least one organic pigment may be chosen from nitroso, nitro, azo, xanthene, quinoline, anthraquinone, phthalocyanin, metal-complex, isoindolinone, isoindoline, quinacridone, perinone, perylene, diketopyrrolopyrrole, thioindigo, dioxazine, triphenylmethane, and quinophthalone compounds.

White or colored organic pigments may be chosen from carmine, carbon black, aniline black, melanin, azo yellow, quinacridone, phthalocyanin blue, sorghum red, the blue pigments codified in the Color Index under the references CI 42090, 69800, 69825, 73000, 74100, and 74160, the yellow pigments codified in the Color Index under the references CI 11680, 11710, 15985, 19140, 20040, 21100, 21108, 47000, and 47005, the green pigments codified in the Color Index under the references CI 61565, 61570, and 74260, the orange pigments codified in the Color Index under the references CI 11725, 15510, 45370, and 71105, the red pigments codified in the Color Index under the references CI 12085, 12120, 12370, 12420, 12490, 14700, 15525, 15580, 156820, 156830, 15800, 15850, 15865, 15880, 17200, 26100, 45380, 45410, 58000, 73360, 73915, and 75470, and the pigments obtained by oxidative polymerization of indole or phenolic derivatives as described in French Patent Publication No. FR 2 679 771.

Exemplary pigmentary pastes of organic pigments include Pigment Yellow 3 (CI 11710), Pigment Yellow 1 (CI 11680), Pigment Orange 43 (CI71105), Pigment Red 4 (CI 12085), Pigment Red 5 (CI 12490), Pigment Violet 23 (CI 51319), Pigment Blue 15.1 (CI 74160), Pigment Green 7 (CI 74260), Pigment Black 7 (CI 77266), and mixtures thereof.

The at least one pigment in accordance with the present technology may also be in the form of at least one composite pigment as described in European Patent Publication No. EP 1 184 426. These composite pigments may be, for example, compounds of particles comprising a mineral core, at least one binder for ensuring the binding of the organic pigments to the core, and at least one organic pigment at least partially covering the core.

The organic pigment may also be a lake. As used herein, the term "lake" means dyes adsorbed onto insoluble particles, the assembly thus obtained remaining insoluble during use. The insoluble particles onto which the dyes are adsorbed can be, for example, alumina, silica, calcium sodium borosilicate, calcium aluminum borosilicate, and aluminum. A non-limiting example of a lake is D&C Red 7 (CI 15 850:1).

Exemplary dyes include cochineal carmine, D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), and D&C Blue 1 (CI 42 090).

The at least one pigment may also be a pigment with special effects. As used herein, the term "pigments with special effects" means pigments that generally create a non-uniform colored appearance (characterized by a certain shade, a certain vivacity, and a certain lightness) that changes as a function of the conditions of observation (light, temperature, observation angles, etc.). They thus contrast with white or colored pigments that afford a standard uniform opaque, semi-transparent, or transparent shade Several types of pigments with special effects exist those with a low refractive index, such as fluorescent, photochromic, or thermochromic pigments, and those with a high refractive index, such as nacres or glitter flakes.

Examples of pigments that impart special effects include nacreous pigments such as mica/red iron oxide, mica coated with titanium or with bismuth oxychloride, colored nacreous pigments such as titanium mica with iron oxides, titanium mica with ferric blue or with chromium oxide, titanium mica with an organic pigment as set forth above, and nacreous pigments based on bismuth oxychloride. Exemplary nacreous pigments include mica-TiO₂-lake, mica-TiO₂, mica-Fe₂O₃, and mica-TiO₂-Fe₂O₃.

In addition to nacres on a mica support, multilayer pigments based on synthetic substrates such as alumina, silica, sodium calcium borosilicate, calcium aluminum borosilicate, and aluminum, may be utilized.

Pigments with an interference effect that are not fixed onto a substrate, for instance, liquid crystals (Helicones^{™} HC from Material District) and holographic interference flakes. Pigments with special effects also include fluorescent pigments, phosphorescent pigments, photochromic pigments, thermochromic pigments, and quantum dots.

The variety of pigrnents that may be used in the present disclosure makes it possible to obtain a wide range of colors, and also optical effects such as metallic effects or interference effects.

In one aspect, the particle size of the at least one pigment used in the cosmetic composition according to the present technology ranges from 10 nm to 200 µm, or from 20 nm to 80 µm, or from 30 nm to 50 µm.

In one aspect, the at least one pigment may be dispersed in the temporary hair product via at least one dispersant. The at least one dispersant serves to protect the dispersed particles against agglomeration or flocculation. The at least one dispersant may be a surfactant, an oligomer, a polymer, or a mixture of several thereof, bearing at least one functional group with strong affinity for the surface of the particles to be dispersed. For example, they can physically or chemically attach to the surface of the at least one pigment. These dispersants also contain at least one functional group that is compatible with or soluble in the continuous medium. For example, 12-hydroxystearic acid esters and C₈ to C₂₀ fatty acid esters of polyols such as glycerol or diglycerol, polyglyceryl-2 dipolyhydroxystearate commercially available under the trade name Dehymyls^{™} PGPH marketed by BASF, or polyhydroxystearic acid such commercially available under the trade name Arlacel^{™} P100 marketed by Croda, and mixtures thereof.

The pigment (coloring agent) is present in the temporary dyeing compositions in an effective amount to dye hair. In one aspect, the amount of pigment present in the temporary dyeing composition ranges from about 0.0005 to about 20 wt.%, or from about 0.005 to 10 wt.%, or from about 0.05 to about 6 wt.%, based on the total weight of the composition.

The temporary coloring compositions may be formulated in any manner commonly used in temporary hair dye products. In one aspect, the temporary dye composition is formulated in the form of lotions, creams, gels, shampoos, mousses, and emulsions or other appropriate vehicles known to the skilled hair color formulator.

In one aspect, the at least one polyurethane having tethered tertiary amino groups which are optionally partially or fully neutralized and/or quaternized can be formulated directly into the temporary dye composition.

The at least one polyurethane component (b) comprises the reaction product of:
(i) at least one polyisocyanate;
(ii) at least one main chain polyamine, polyol, polymercaptan, and mixtures thereof having about two isocyanate reactive hydrogens; and
(iii) at least one compound containing a tertiary nitrogen having two isocyanate reactive hydrogen-containing substituents and a tethered tertiary amino group substituent, wherein said tertiary amino group is situated away from said polyurethane backbone by a tethering moiety containing at least two intervening atoms.

In one aspect, the tethered tertiary amino group(s) of the at least one polyurethane component b) is optionally partially or fully neutralized and/or quaternized.

In one aspect, the at least one polyurethane b) having tethered tertiary amino groups which are optionally partially or fully neutralized and/or quaternized is dispersed in water to form a polyurethane prepolymer dispersion.

In one aspect, said partially or fully neutralized and/or quaternized prepolymer aqueous dispersion is chain extended with a chain extender selected from water, an inorganic or organic polyamine, low molecular weight polyols, and mixtures thereof.

### Polyurethane Component

The polyurethanes of this technology are formed from at least one polyisocyanate and at least one NCO-reactive compound (isocyanate reative compound). Any compound that provides a source of active hydrogen for reacting with isocyanate groups via the following reaction: -NCO+H-X → -NH-C(=O)-X, can be used as the NCO-reactive compound in the present technology. Examples of active hydrogen compounds include but are not limited to polyols, polythiols and polyamines. In addition, tethered tertiary amine groups are introduced into the polymer via monomers containing at least two active hydrogen groups for reaction with isocyanates. Optionally, isocyanate reactive chain extenders and water dispersability monomers can be reacted into the polyurethane backbone.

In one aspect, the polyurethanes of the present technology are prepared as aqueous polyurethane dispersions utilizing the well-known prepolymer method.

### Isocyanates

Suitable polyisocyanates have an average of about two or more isocyanate groups, preferably an average of about two to about four isocyanate groups per molecule and include aliphatic, cycloaliphatic, araliphatic, aromatic, and heterocyclic polyisocyanates, as well as products of their oligomerization, used alone or in mixtures of two or more. In one aspect the polyisocyanate is selected from diisocyanates, but even monofunctional isocyanates can be used, for example, as molecular weight controlling agents.

Specific examples of suitable aliphatic polyisocyanates include alpha, omega-alkylene diisocyanates having from 5 to 20 carbon atoms, such as hexamethylene-1,6-diisocyanate, 1,12-dodecane diisocyanate, 2,2,4-trimethyl-hexamethylene diisocyanate, 2,4,4-trimethyl-hexamethylene diisocyanate, 2-methyl-1,5-pentamethylene diisocyanate, lysine diisocyanate and the like. Polyisocyanates having fewer than 5 carbon atoms can be used but are less preferred because of their high volatility and toxicity. Preferred aliphatic polyisocyanates include hexamethylene-1,6-diisocyanate, 2,2,4-trimethyl-hexamethylene-diisocyanate, and 2,4,4-trimethyl-hexamethylene diisocyanate.

Specific examples of suitable cycloaliphatic polyisocyanates include dicyclohexylmethane diisocyanate, isophorone diisocyanate, cyclohexane diisocyanate, bis-(isocyanatomethyl) cyclohexane, methylcyclohexane diisocyanate, cyclohexane triisocyanate, their isomers and the like. Preferred cycloaliphatic polyisocyanates include dicyclohexylmethane diisocyanate and isophorone diisocyanate.

Specific examples of suitable araliphatic polyisocyanates include m-tetramethyl xylylene diisocyanate, p-tetramethyl xylylene diisocyanate, 1,4-xylylene diisocyanate, 1,3-xylylene diisocyanate, and the like. A preferred araliphatic polyisocyanate is tetramethyl xylylene diisocyanate.

Examples of suitable aromatic polyisocyanates include diphenylmethylene diisocyanate, toluene diisocyanate, phenylene diisocyanate, naphthalene diisocyanate, tetrahydronaphthalene diisocyanate, biphenylene diisocyanate, dimethyl biphenylene diisocyanate, dichloro biphenylene diisocyanate, triphenyl methane triisocyanate, their isomers, and the like. In one aspect, the aromatic polyisocyanate include 4,4'-diphenylmethylene diisocyanate and toluene diisocyanate.

Examples of suitable heterocyclic isocyanates include 5,5'-methylenebisfurfuryl isocyanate and 5,5'-isopropylidenebisfurfuryl isocyanate.

### Active-Hydrogen Containing Compounds

The term "active-hydrogen containing" refers to compounds that are a source of active hydrogen and can react with isocyanate groups via the following reaction:

-NCO+H-X → -NH-C(=O)-X

Such compounds typically range widely in molecular weight from 18 g/mol for water and 17 g/mol for ammonia to about 10,000 g/mol. They are customary divided into two subclasses depending on their molecular weight: Polyols with number-average molecular weight from about 500 to 10,000 g/mol and chain extenders with molecular weight from 18 to 500 g/mol. The extremes of the scale represent physical reality: High molecular weight polyols contribute to the soft segment and low molecular weight short chain extenders contribute to the hard segment of polyurethane.

### Polyols and Active Hydrogen Containing Compounds

The term "polyol" in the context of the present technology means any high molecular weight product (*M*ₙ > 500 g/mol), typically referred to as a long-chain polyol, which has an active hydrogen that can be reacted with isocyanates and includes materials having an average of about two or more hydroxyl or other NCO-reactive groups per molecule. Examples of moieties which contain active hydrogens that react with NCO-groups (isocyanate groups) are hydroxyl, amino, thiol groups.

Such long-chain polyols include polyether, polyester, polycarbonate and polycaprolactone polyols. Other examples of active hydrogen group containing polymers include polyamide, polyester amide, polyacetal, polythioether, polysiloxane, ethoxylated polysiloxane, halogenated polyester and polyether, polybutadiene, hydrogenated polybutadiene, polyisoprene, polyisobutylene, alkyd-modified and polythioether polyols, hydroxyl-containing acrylic and methacrylic polymers and copolymers, hydroxyl-containing epoxies, and the like, and mixtures thereof. Combinations of different types of polyols may be used. In one aspect, the molecular weight (*M*ₙ ) > 500 g/mol, or ranges from about 650 g/mol to about 8000 g/mol, or from about 800 to about 4000 g/mol, or from about 1000 to about 3000 g/mol, or from about 1200 to about 2500 g/mol, or from about 1400 to about 2000 g/mol.

### Polyether Polyol

Polyether polyols are obtained in known manner by reaction of starting compounds that contain reactive hydrogen atoms, such as water or the diols set forth for preparing the polyester polyols, with alkylene oxides, such as ethylene oxide, propylene oxide, 1,2-propane diol, 1,3-propane diol, butylene oxide, styrene oxide, tetrahydrofuran, epichlorohydrin, and mixtures thereof. In one aspect, the polyethers include polytetrahydrofuran (PTHF) and poly(propylene glycol) (PPG derived from 1,2-propane diol or 1,3-propane diol). Examples include Terathane^{®} PTHF polyols from Invista and Acclaim^{™} PPG diols with lower monol contents from Arco Chemical, and Cerenol^{™} bio-based PPG from DuPont. Cerenol^{™} bio-based PPG is derived from 1,3-propane diol.

In one aspect, polyether polyols provide less than about 25 wt.%, more preferably less than about 15 wt.% and most preferably less than about 5 wt.% poly(ethylene oxide) units in the backbone (main chain) based upon the dry weight of final polyurethane, since such main-chain poly(ethylene oxide) units tend to cause swelling of polyurethane particles in the waterborne polyurethane dispersion and also contribute to lower in-use (under wet or high-humidity conditions) tensile strength of articles made from the polyurethane dispersion.

### Polyester Polyol

The polyester polyols typically are esterification products prepared by the reaction of organic polycarboxylic acids or their anhydrides with a stoichiometric excess of a diol. Examples of suitable polyols for use in the reaction include polyglycol adipates, polyethylene terephthalate polyols, polycaprolactone polyols, orthophthalic polyols, sulfonated polyols, and the like, and mixtures thereof.

The diols used in making the polyester polyols can be aliphatic, cycloaliphatic or aromatic and include alkylene glycols, e.g., ethylene glycol, 1,2- and 1,3-propylene glycols, 1,2-, 1,3-, 1,4-, and 2,3-butylene glycols, hexane diols, neopentyl glycol, 1,6-hexanediol, 1,8-octanediol, and other glycols such as bisphenol-A, cyclohexane diol, cyclohexane dimethanol (1,4-bis-hydroxymethylcycohexane), 2-methyl-1,3-propanediol, 2,2,4-trimethyl-1,3-pentanediol, 2-butyl-2-ethyl prorane-1,3-diol, Versatic^{™} alcohols produced from CARDURA^{®} E10P (Hexion), triethylene glycol, tetraethylene glycol, polyethylene glycol, dipropylene glycol, polypropylene glycol, dibutylene glycol, polybutylene glycol, caprolactone diol, dimerate diol, hydroxylated bisphenols, polyether glycols, halogenated diols, and the like, and mixtures thereof. In one aspect, the diols include ethylene glycol, butylene glycol, hexane diol, and neopentyl glycol.

Suitable carboxylic acids used in making the polyester polyols include dicarboxylic acids and tricarboxylic acids and anhydrides, e.g., maleic acid, maleic anhydride, succinic acid, glutaric acid, glutaric anhydride, adipic acid, suberic acid, pimelic acid, azelaic acid, sebacic acid, chlorendic acid, 1,2,4-butane-tricarboxylic acid, phthalic acid, the isomers of phthalic acid, phthalic anhydride, fumaric acid, tetrabromophthalic anhydride and acid dimeric fatty acids such as oleic acid, and the like, and mixtures thereof. In one aspect, the polycarboxylic acids used in making the polyester polyols include aliphatic or aromatic dibasic acids.

In one aspect, the polyester polyol is a diol. Exemplary polyester diols include hexane diol neopentyl glycol adipic acid polyester diol, e.g., Piothane^{™} 67-3000HNA (Panolam Industries) and Piothane 67-1000HNA; as well as propylene glycol maleic anyhydride adipic acid polyester diols, e.g., Piothane 50-1000OPMA; and hexane diol neopentyl glycol fumaric acid polyester diols, e.g., Piothane 67-500HNF. Other preferred polyester diols include Rucoflex^{™} S1015-35, S1040-35, and S-1040-110 (RUCO Polymer Corp.).

### Polycarbonate Polyol

Polycarbonate polyols include those obtained from the reaction of diols such 1,3-propanediol, 1,4-butanediol, 1,6-hexanediol, diethylene glycol, triethylene glycol, tetraethylene glycol, and the like, and mixtures thereof with diarylcarbonates such as diphenylcarbonate or phosgene.

### Polysiloxane Polyol

The polysiloxane polyols are characterized by the presence of the - R₁R₂SiO- repeat units which can contain alkyl or aryl groups such as polydimethylsiloxanes, poly(dimethysiloxane-co-diphenylsiloxane)s, polydiphenylsiloxanes, poly(methylphenyl)siloxanes and the like, and combinations thereof. Examples include ethoxylated poly(dimethylsiloxane) (PDMS) Y-17256 from Momentive Performance Materials and side-chain PDMS diol MCR-C61 from Gelest.

### Polyacetals

Polyacetals include the compounds that can be prepared from the reaction of (A) aldehydes, such as formaldehyde and the like, and (B) glycols such as diethylene glycol, triethylene glycol, ethoxylated 4,4'-dihydroxy-diphenyldimethylmethane, 1,6-hexanediol, and the like. Polyacetals can also be prepared by the polymerization of cyclic acetals.

### Polyester Amides and Polyamides

In lieu of long-chain polyols, long-chain amides may also be used to prepare the isocyanate terminated prepolymer. Suitable long-chain amides include polyester amides and polyamides, such as the predominantly linear condensates obtained from reaction of polybasic saturated and unsaturated carboxylic acids or their anhydrides and polyvalent saturated or unsaturated aminoalcohols, diamines, polyamines, and mixtures thereof.

Diamines and polyamines are among the preferred compounds useful in preparing the aforesaid polyester amides and polyamides. Suitable diamines and polyamines include 1,2-diaminoethane, 1,6-diaminohexane, 2-methyl-1,5-pentanediamine, 2,2,4-trimethyl-1,6-hexanediamine, 1,12-diaminododecane, 2-aminoethanol, 2-[(2-aminoethyl)amino]-ethanol, piperazine, 2,5-dimethylpiperazine, 1-amino-3-aminomethyl-3,5,5-trimethylcyclohexane (isophorone diamine or IPDA), bis-(4-aminocyclohexyl)-methane, bis-(4-amino-3-methyl-cyclohexyl)-methane, 1,4-diaminocyclohexane, 1,2-propylenediamine, hydrazine, urea, amino acid hydrazides, hydrazides of semicarbazidocarboxylic acids, bis-hydrazides and bis-semicarbazides, diethylene triamine, triethylene tetramine, tetraethylene pentamine, pentaethylene hexamine, N,N,N-tris-(2-aminoethyl)amine, N-(2-piperazinoethyl)-ethylene diamine, N,N'-bis-(2-aminoethyl)-piperazine, N,N,N'tris-(2-aminoethyl)ethylene diamine, N-[N-(2-aminoethyl)-2-aminoethyl]-N'-(2-aminoethyl)-piperazine, N-(2-aminoethyl)-N'-(2-piperazinoethyl)-ethylene diamine, N,N-bis-(2-aminoethyl)-N-(2-piperazinoethyl)amine, N,N-bis-(2-piperazinoethyl)-amine, polyethylene imines, iminobispropylamine, guanidine, melamine, N-(2-aminoethyl)-1,3-propane diamine, 3,3'-diaminobenzidine, 2,4,6-triaminopyrimidine, polyoxypropylene amines, tetrapropylenepentamine, tripropylenetetramine, N,N-bis-(6-aminohexyl)amine, N,N'-bis-(3-aminopropyl)ethylene diamine, and 2,4-bis-(4'-aminobenzyl)-aniline, and the like, and mixtures thereof. Preferred diamines and polyamines include 1-amino-3-aminomethyl-3,5,5-trimethyl-cyclohexane (isophorone diamine or IPDA), bis-(4-aminocyclohexyl)-methane, bis-(4-amino-3-methylcyclohexyl)-methane, ethylene diamine, diethylene triamine, triethylene tetramine, tetraethylene pentamine, and pentaethylene hexamine, and mixtures thereof. Other suitable diamines and polyamines include Jeffamine^{™} D-2000 and D-4000, which are amine-terminated polypropylene glycols, differing only by molecular weight, and which are available from Huntsman Chemical Company.

### Polythiol

In one aspect, polythiol compounds may also be used to prepare the isocyanate terminated prepolymer. Typical examples of polythiol compounds represented b include 1,2-ethanedithiol, propane-1,2-dithiol, n-hexane 1,6-dithiol, n-decane-1,10-dithiol, 1,3-cyclohexanedithiol, 1,4-cyclohexanedithiol, bis(2-mercaptoethyl)ether, 1,2-bis(mercaptomethylthio)benzene, 1,4-dimercaptobenzene, and bis(2-mercaptoethyl) sulfide.

### NCO:OH Ratio

In one aspect, the prepolymer produced in the present technology will be isocyanate-terminated. For this purpose, the ratio of isocyanate equivalents to active hydrogen in the prepolymer typically ranges from about 1.3/1 to about 2.5/1, or from about 1.5/1 to about 2.1/1, or from about 1.7/1 to about 2/1.

An OH-terminated prepolymer can also be made if desired. In this case, an access of OH equivalents over NCO is used.

### Tethering Monomer

The polyurethanes of the present technology contain cationic groups tethered to the backbone. Such cationic groups include ammonium, phosphonium and sulfonium groups. These groups can be incorporated into the polymer in the ionic form or, optionally, they can be generated by post-neutralization (e.g., with an acid) and/or post-quaternization of corresponding nitrogen (e.g., with an alkyl halide), phosphorous, or sulfur moieties tethed from the polyurethane backbone. The combination of all of the above groups can be used as well as their combination with nonionic stabilization. Anionic groups also can be incorporated into the polymer producing zwitterionic compositions.

The tethered cationic group containing monomer reacted with the other reactants forming the polyurethane through conventional isocyanate reactions with active hydrogen groups present on the tethered tertiary amine monomer such that, when the monomer is incorporated into the polyurethane, the tertiary nitrogen atom is tethered away from the polyurethane backbone by at least two atoms in one aspect, and by at least three atoms in another aspect. In one aspect, the tethering atoms comprise, consist essentially of, or consist of carbon atoms.

The monomer having a tethered tertiary amino group contains on average two active hydrogen groups which participate in building the polyurethane of the present technology.

Examples of tethered amine monomers include 2,2'-((3-dimethylamino)propyl)azanediyl)bis(ethan-1-ol) and 1,1'-((3-dimethylamino)propyl)azanediyl)bis(propan-2-ol) (Jeffcat^{®} DPA from Hunstman) and are represented by the structures: 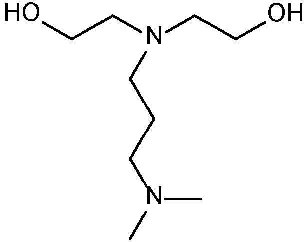 2,2'-((3-(dimethylamino)propyl)azanediyl)bis(ethan-1-ol) 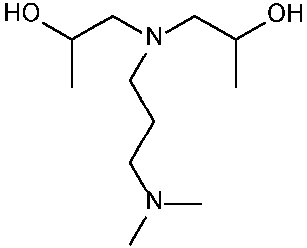 1,1'-((3-(dimethylamino)propyl)azanediyl)bis(propan-2-ol)

In one aspect, the monomer having a tethered tertiary amino group may contain only one active hydrogen group. These monofunction active hydrogen group containing tethered monomers may be used in polyurethane synthesis as chain extenders or as components of polyester or polyether polyols These monofunctional active hydrogen group containing monomers can be used either in combination with difunctional tethered monomers describe above, or by themselves.

In one aspect, a variant of Michael addition reaction can produce monofunction active hydrogen group tethered amine monomers when hydroxyalkyl acrylates react with N,N-dialkyl alkylenediamines. Thus, reaction of 2-hydroxyethyl acrylate with N,N-dimethyl propylenediamine yields the following monomer:

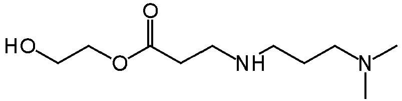

In another embodiment, aminoalcohol monomers with tethered tertiary amino groups can be synthesized by reacting oxiranes (epoxides) with the aforementioned asymmetric dialkyl diaminoalkylenes:

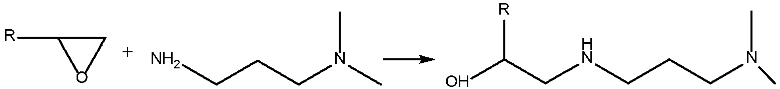

Tertiary amines can be neutralized to make cationic salts with virtually any acid. Examples of acids include acetic acid, formic acid, hydrochloric acid, phosphoric acid, sulfuric acid, nitric acid, nitrous acid, boric acid, carbonic acid, perchloric acid, acrylic acid, methacrylic acid, itaconic acid, maleic acid, 2-carboxyethyl acrylate, lactic acid, ascorbic acid, glycine, alanine, leucine, norleucine, phenylalanine, serine, taurine, valine, alpha-aminobutyric acid, palmitic acid, stearic acid, benzoic acid, mercaptoacetic acid, salicylic acid, pivalic acid, chloroacetic acid, dichloroacetic acid, trichloroacetic acid, citric acid, propionic acid, glycolic acid, 1-sulfonaphthalene, tartaric acid, phthalic acid, isophthalic acid, terephthalic acid, 5-sulfosalicylic acid, benzenesulfonic acid, cyclohexanecarboxylic acid, o-, m-, and p-toluic acid, o-, m-, and p-aminobenzoic acid, p-hydroxybenzoic acid, phenylacetic acid, methylbenzenesulfonic acid, butyric acid, valeric acid, oxalic acid, maleic acid, fumaric acid, malonic acid, succinic acid, glutaric acid, oleic acid, o-, m-, and p-chlorobenzoic acid, o-, m-, and p-bromobenzoic acid, anthranilic acid, o-, m-, and p-nitrobenzoic acid, adipic acid, caprylic acid, caproic acid, lauric acid, fluoroacetic acid, capric acid, myristic acid, methoxyacetic acid, dodecanesulfonic acid, dodecylbenzenesulfonic acid, ethylbenzenesulfonic acid, octanesulfonic acid, hexanesulfonic acid, polyacrylic acid, copolymers of acrylic and methacrylic acid.

In one aspect, the tertiary amine groups are neutralized prior to or during dispersion of the polyurethane in water to some extent. In one embodiment, the extent of neutralization of the tethered and/or terminal tertiary amine group is >10%, in another embodiment > 20, ≥25 or >30 mole %, and in more preferred embodiments the extent of neutralization is >45 or >60 mole %. In one embodiment, at least 80, at least 85, at least 90 or at least 95 mole % of the tertiary amino groups are neutralized. Since multiple tertiary amino groups sometime inhibit the neutralization or quaternization of closely spaced adjacent tertiary amino groups, when we specify percent quaternization or neutralization, we will refer to the percent of groups quaternized or neutralized at one or more nitrogen atoms of the tethered or terminal group (not reducing the percent quaternization or neutralization due to inhibition of quaternization or neutralization due to close physical proximity). In another embodiment, excess acid over amine can be used.

The tertiary amine may be quaternized with any known quaternizing agent. In one aspect the quaternizing agents are alkyl halides, aralkyl halides, dialkyl carbonates, dialkyl sulphates and epoxides. In one aspect, quaternizing agents include methyl chloride, ethyl chloride, benzyl chloride, methyl bromide, ethyl bromide, benzyl bromide, dimethyl sulfate, diethyl sulfate, ethylene oxide, propylene oxide, butylene oxide, styrene oxide and epichlorohydrin.

In one aspect, the tertiary amine groups are quaternized to some extent. In one embodiment, the extent of quaternization of the tethered and/or terminal tertiary amine group is >10 mol %, or > 20, ≥25 or >30, or >45 or >60 mole %. In one apect, at least 80, or at least 85, or at least 90 or at least 95 mole % of the tertiary amino groups are quaternized.

Combinations of quaternization and neutralization can be used.

The number of tethered tertiary amino groups may be from 0.1 to about 15 or 20 milliequivalents per gram of urethane polymer. In one aspect, the lower limit is 0.2, 0.3, 0.4, 0.5, or 0.6 milliequivalents/gram and the upper limit is about 10, 8, 5, 4, 3, 2, 1 or less than 1 milliequivalents per gram of urethane polymer. The number of tethered tertiary amino groups goes down as the groups are quaternized or neutralized with an acid (which makes them more effective in colloidally stabilizing the urethane dispersion in water). The number of tethered tertiary amino groups also goes down as nonionic and/or zwitterionic groups are added to the urethane polymer supplement the cationic stabilization effect for colloidal stabilization for the urethane dispersion in water. For the purpose of facilitating the calculation of the amount of tethered tertiary amino groups for the above ranges, if there are multiple tethered tertiary amino groups in one group tethered or terminal location from the urethane backbone, all of the tethered tertiary amino groups together will be counted as a single tethered tertiary amino group. Tethered tertiary amino groups will be counted the same irrespective of whether they are quaternized or neutralized with an acid component. In one example without any nonionic colloidal stabilization moieties, we found very effective colloidal stabilization using just 0.87 milliequivalents of tethered tertiary amino groups per gram of urethane polymer.

### Chain Extenders

Chain extenders with the molecular weight from 18 to 500 g/mole such as aliphatic, cycloaliphatic, or aromatic diols or amines optionally can be used during the formation of the prepolymer and during the dispersion step of the process. Because the prepolymer is formed at elevated temperatures and in the general absence of water, the less reactive alcohol functionality is preferred for the prepolymer chain extension to provide for better control over the temperature and mixing.

On the other hand, during the dispersion stage of the process, chain extenders are competing with water for the reaction with the remaining NCO. In this case, a more reactive amine functionality is desired.

In one aspect, no chain extender is used.

In one aspect, a chain extender selected from water, inorganic or organic polyamines having an average of about 2 or more primary and/or secondary amine groups or combinations thereof is suitable for use in this technology. Suitable organic amines for use as a chain extender are the same diamines and polyamines described above as monomers for preparing polyester amides and polyamides.

In one aspect, the chain extender used to extend the prepolymer is selected from water, ethylene diamine, and mixtures thereof.

The amount of chain extender typically ranges from about 0.3 to about 1.1 equivalents based on available isocyanate.

### Water-Dispersability Enhancing Compounds

In one aspect, at least one water-dispersability enhancing compound (i.e., monomer), which has at least one, hydrophilic, ionic or potentially ionic group is optionally included in the polyurethane polymers and prepolymers of this technology to assist dispersion of the polymer/prepolymer in water. In one aspect, the tethered amine monomer or its salt is this water-dispersability enhancing compound and its content is sufficient to prepare a stable dispersion without additional means.

In another aspect, an additional water-dispersability enhancing compound can be used especially if the content of the tethered amine monomer or its salt is insufficient to prepare a stable dispersion without additional means. These compounds may be of a nonionic, anionic, cationic or zwitterionic nature or the combination thereof. For example, anionic groups such as carboxylic acid groups can be incorporated into the prepolymer in an inactive form and activated by a salt-forming compound, such as tethered amine compound or an additional tertiary amine. Normally, carboxylic groups are introduced by hydroxy-carboxylic acids having the general formula (HO)ₓQ(COOH)_{y}, wherein Q is a straight or branched hydrocarbon radical containing 1 to 12 carbon atoms, and x and y are 1 to 3. Examples of such hydroxy-carboxylic acids include dimethylolpropanoic acid, dimethylol butanoic acid (DMBA), citric acid, tartaric acid, glycolic acid, lactic acid, malic acid, and the like, and mixtures thereof. Dihydroxy-carboxylic acids are more preferred with dimethylolproanoic acid and dimethylol butanoic acid (DMBA) being most preferred. Carboxylic acids can be converted into cationic centers by post-polymerization reactions such as, for example, reaction of epoxy quaternary ammonium compounds with carboxylic group of dimethylolpropanoic acid.

Water-dispersability enhancing compounds of particular interest are side chain hydrophilic monomers. Some examples include alkylene oxide polymers and copolymers in which the alkylene oxide groups have from 2-10 carbon atoms as shown, for example, in U.S. Patent No. 6,897,281.

The amount of such side chain hydrophilic monomers can be as little as 10, or 6, or 3, or 2 or even 1% or less based on the weight of final polyurethane if enhanced colloidal stability is desired and as high as 20, or 30, or 40, or even 50% if water- or polar solvent-absorbing characteristics are required.

Commercially available side chain hydrophilic monomers include Tegomer^{®} D-3403 from Evonik and Ymer N90, N120, and N180 from Perstorp.

### Catalysts

Urethane prepolymer may be formed without the use of a catalyst but catalysis may be employed in some instances to reduce synthesis time or temperature. Examples of catalysts include organo-tin compounds, tertiary amines and transition metal compounds. Specific examples of suitable catalysts include stannous octoate, dibutyl tin dilaurate, and tertiary amine compounds such as triethylamine and bis-(dimethylaminoethyl) ether, morpholine compounds such as beta,beta-dimorpholinodiethyl ether, bismuth carboxylates, zinc bismuth carboxylates, iron (III) chloride, potassium octoate, potassium acetate and zirconium catalysts K-KAT^{®} XC-9213 and K-KAT^{®} 6212 from King Industries.

In one aspect, the amount of catalyst used to form the prepolymer will typically be from about 5 to about 200 parts per million of the total weight of prepolymer reactants.

### Solvents

Optional solvents which are nonreactive to any significant extent in the context of the urethane-making reactions, may be used in the present technology, but are not preferred because they introduce volatile organic component (VOC). The use of a solvent may be desirable to reduce the prepolymer viscosity, provide a heat sink, serve as refluxing medium, and assist with film formation. Examples of solvents include substituted pyrrolidinones, amides, esters, ethers, ketoesters, ketones, glycolether-esters, hydrogenated furans, tertiary alcohols, aromatic and aliphatic hydrocarbons, chlorinated hydrocarbons, and the like, and mixtures thereof.

Specific examples include N-methyl-2-pyrrolidinone, N-ethyl-2-pyrrolidinone dimethylformamide, dimethylacetamide, acetone, methylethyl ketone, diisobutyl ketone, isobutylheptyl ketone, dimethylsulfoxide, N-methyl caprolactam, N-methyl valerolactam, ethylene glycol monomethyl ether formal, and dipropylene glycol dimethyl ether.

The amount of solvent can vary in a broad range depending on the specifics of the polymer to be produced. About 0.1 to 30 parts by weight of solvent by weight of solvent per 100 parts by weight of the prepolymer can be used.

In some cases, it is desirable to remove at least a portion of the solvent from the dispersion. It can be done with solvents which have a lower boiling point than water. These solvents can be removed from the dispersion by, for example, distillation, vacuum distillation, isotropic distillation, and thin-film evaporation.

### Polymer Preparation

Aqueous dispersions of polyurethane particles are made in accordance with the present technology by forming a polyurethane prepolymer in the substantial absence of water and then dispersing this prepolymer in aqueous medium. This can be done in any fashion. Typically, prepolymer formation will be done by bulk or solution polymerizing the ingredients of the prepolymer.

Once the polyurethane prepolymer mixture is formed, it is dispersed in an aqueous medium to form a dispersion or a solution. Dispersing the prepolymer in aqueous medium can be done by any conventional technique, in the same way that other polyurethane prepolymers made by bulk or solution polymerization are dispersed in water. Normally, this will be done by combining the prepolymer blend with water with mixing. The prepolymer may be neutralized and/or quaternized prior to or immediately after being dispersed in water. Following neutralization and dispersion in water, the prepolymer is chain extended by reaction with at least one of water, inorganic or organic polyamines having an average of about 2 or more primary and/or secondary amine groups, polyalcohols, ureas, or combinations thereof.

In one aspect, the neutralized and dispersed prepolymer is chain extended with the water present in the dispersion medium.

In one aspect, the tethered polyurethanes of the disclosed technology are prepared by a process comprising:
(A) reacting (1) at least one polyisocyanate having an average of about two or more isocyanate groups selected from an aliphatic diisocyanate, an aromatic diisocyanate, an araliphatic diisocyanate, and mixtures thereof; (2) at least one active hydrogen group containing compound selected from polyether polyols; polyester polyols, polycarbonate polyols, polysiloxane polyols, polyacetals, polyester amides; polyester amides; polythiols, and mixtures thereof; (3) at least one active hydrogen group containing monomer having a tethered tertiary amine group to form a prepolymer; and
(B) dispersing said prepolymer in water, optionally neutralizing said prepolymer if necessary, and chain extending said prepolymer by reaction with at least one of water, inorganic or organic polyamine having an average of about 2 or more primary and/or secondary amine groups, polyalcohols, ureas, or combinations thereof.

Where solvent polymerization is employed, the solvent and other volatile components can optionally be distilled off from the final dispersion, if desired. Where the prepolymer includes enough water-dispersability enhancing compound (such as cationic and optional nonionic monomers) to form a stable dispersion without added emulsifiers (surfactants), the dispersion can be made without such compounds, i.e., substantially free of surfactants, if desired. The advantage of this approach is that the hair care products made from the polyurethane exhibit less water sensitivity, better film formation and less foaming.

An additional benefit of the compositions of the present technology over those disclosed in the prior art is the significantly higher solids content possible. In one aspect, the dispersions of this technology typically have total solids (i.e., polyurethane solids) of at least about 20 wt.%, or at least about 25 wt.%, or at least about 30, 31.25, 35, or 40 wt.%.

Aqueous dispersions of the at least one polyurethane having tethered tertiary amino groups that are partially or fully neutralized and/or quaternized are relatively hydrophilic when dispersed in a continuous hair color formulation phase, and when the polymer dries the neutralizing agent evaporates and the polymer reverts to the unneutralized form redering the polymer hydrophobic. Without wishing to be bound by theory, it is believed that the polymer forms a hydrophobic film on/in the colored hair substrate) which locks the coloring agent onto or into the hair thus preventing the coloring agent from leaching.

The at least one polyurethane having tethered tertiary amino groups that are partially or fully neutralized and/or quaternized is compatible with coloring agents commonly used in permanent, semi-permanent and temporary hair coloring compositions.

In one aspect, the at least one polyurethane having tethered tertiary amino groups is a polyurethane dispersion prepared from at least one cycloaliphtic diisocyanate, at least one polyether polyol, at least one tethered tertiary amino group monomer containing two active hydrogen groups, wherein said tertiary amino groups are neutralized with an acid.

In one aspect, the at least one polyurethane having tethered tertiary amino groups is a polyurethane dispersion prepared from a first cycloaliphtic diisocyanate, a second cycloaliphatic diisocyanate different from the first cycloaliphatic diisocyanate, at least one polyether polyol, at least one tethered tertiary amino group monomer containing two active hydrogen groups, wherein said tertiary amino groups are neutralized with an acid.

In one aspect, the at least one polyurethane having tethered tertiary amino groups is a polyurethane dispersion prepared from dicyclohexylmethane diisocyanate (H₁₂MDI) and isophorone diisocyanate (IPDI), polytetrahydrofuran, at least one tethered tertiary amino group monomer selected from 2,2'-((3-dimethylamino)propyl)azanediyl)bis(ethan-1-ol) and 1,1'-((3-dimethylamino)propyl)azanediyl)bis(propan-2-ol), and mixtures thereof, wherein said tertiary amino groups are neutralized with acetic acid.

The amount of the at least one polyurethane having tethered tertiary amino groups which are optionally partially or fully neutralized and/or quaternized ranges from about 0.004 to about 4 wt.%, or from about 0.2 to about 2.4 wt.%, or from about 0.3 to about 1.2 wt.% (active polymer basis), based on the total weight of the composition. Additionally, the cationic nature of the polymer conveys conditioning properties to the polymer.

The hair color compositions of the present technology can be formulated to be delivered as a shampoo, conditioner, rinse, lotion, emulsion, cream, foam, gel, spray, mousse, pomade, oil, highlighters, powder, paste, tablet, and wax. The ingredients and techniques to formulate the permanent, semi-permanent and temporary hair color products of the present technology are well-known to the formulator of hair color products.

### Auxiliary Components, Additives and Adjuvants

Product formulations comprising the hair coloring agent(s) and the at least one polyurethane having tethered tertiary amino groups which are optionally partially or fully neutralized and/or quaternized of the disclosed technology may contain various auxiliar components, additives and cosmetic adjuvants, conventionally or popularly included in hair coloring compositions including, without being limited thereto, acidifying or alkalizing pH adjusting agents (neutralizing agents) and buffering agents; auxiliary fixatives and film formers, such as nonionic, anionic, cationic, or amphoteric polymers of synthetic or natural origin, and the like; auxiliary rheology modifiers, such as viscosity-increasing polymeric natural and derivatized gums, resin thickeners or gellants; additives, such as emulsifiers, emulsion stabilizers, waxes, dispersants, and the like, and viscosity control agents, such as solvents, electrolytes, and the like; auxiliary conditioning agents, such as antistatic agents, synthetic oils, ester oils, vegetable or animal oils, ceramides, cholesterol, lecithin, silicone oils, monomeric or polymeric quaternized ammonium compounds and derivatives thereof, sheen enhancers, moisturizers, emollients, humectants, lubricants, sunscreen agents, and the like; surfactants, such as anionic, cationic, nonionic, amphoteric, zwitterionic surfactants, and silicone derivatives thereof; polymer film modifying agents (e.g., plasticizers), hair swelling agents (e.g., urea, isopropyl alcohol, propylene carbonate, ethylene carbonate), tackifiers, detackifiers, wetting agents, and the like; product stabilizing and finishing agents, such as chelating agents, opacifiers, pearlescing agents, proteinaceous materials and derivatives thereof, vitamins and derivatives thereof, preservatives, fragrances, solubilizers, colorants (temporary or permanent), such as pigments and dyes, UV absorbers and filters, and the like; propellants (water-miscible or water-immiscible), such as fluorinated hydrocarbons, liquid volatile hydrocarbons, compressed gases, and the like; and mixtures thereof.

Auxiliary components, additives and adjuvant ingredients, products, or materials, which may be employed in the hair coloring compositions disclosed herein will be referred to by the international nomenclature commonly referred to as INCI name assigned them in the International Cosmetic Ingredient Dictionary, published by the Personal Care Products Council (formally the Cosmetic, Toiletry, and Fragrance Association), Washington D.C. (hereafter INCI Dictionary), such as can be found in any edition thereof, for example, Volumes 1 and 2, Sixth Edition, (1995) or Volumes 1-3, Seventh and Eighth Editions, (1997, 2000), or by their commonly used chemical names. Numerous commercial suppliers of materials listed by INCI name, trade name or both can be found in the INCI Dictionary and in numerous commercial trade publications, including but not limited to the 2001 McCutcheon's Directories, Volume 1: Emulsifiers & Detergents and Volume 2: Functional Materials, published by McCutcheon's Division, The Manufacturing Confectioner Publishing Co., Glen Rock, NJ (2001); and 2001 Cosmetic Bench Reference, edition of Cosmetics & Toiletries®, 115 (13), published by Allured Publishing Corporation, Carol Stream IL (2001). Such components and the formulation of compositions are also described in detail in well-known references, such as Cosmetics Science and Technology, First Edition (Sagarin (ed)), published 1957, and Second Edition (Balsam, et al. (eds)), published 1972-74; and The Chemistry and Manufacture of Cosmetics, Second Edition (deNavarre (ed)), published 1975, and Third Edition (Schlossman (ed)), published 2000, both available from Allured Publishing Corporation; Rieger (ed), Harry's Cosmeticology, 8th Edition, Chemical Publishing, Co., Inc., New York, NY (2000); and various formularies available to those skilled in the pharmaceutical arts, such as Remington's Pharmaceutical Sciences, Fourteenth Edition, Mack Publishing Company, Easton, PA (1970).

### Solvent

The composition can be prepared as water-free or water-based formulation. The composition may include a solvent in which the hair coloring agent is soluble/dispersible. The solvent may be selected from water, and organic solvent, and combinations of water and an organic solvent. Examples of organic solvents, other than water, include linear and branched alcohols, such as ethanol, propanol, isopropanol, hexanol, and the like; glycols (e.g.,1,2-propane diol, bio-derived 1,3-propane diol), and aromatic alcohols, such as benzyl alcohol, cyclohexanol, and the like. Other examples of nonaqueous solvents or diluents include silicones, and silicone derivatives, such as cyclomethicone, and the like, ketones such as acetone and methylethyl ketone; natural and synthetic oils and waxes, such as vegetable oils, plant oils, animal oils, essential oils, mineral oils, C₇ to C₄₀ isoparaffins, alkyl carboxylic esters, such as ethyl acetate, amyl acetate, ethyl lactate, and the like, jojoba oil, shark liver oil, and the like. Some of the foregoing nonaqueous auxiliary solvents or diluents may also serve as conditioners and emulsifiers. For purposes of computing a weight basis in the composition, however, all the liquids listed in this section are considered as solvents/diluents.

### Rheology Modifiers (Thickening Agents)

To provide a composition which adheres well to the hair fibers, the composition can include a rheology modifier or thickening agent which increases the overall viscosity of the composition. The Brookfield viscosity of the composition measured at 20rpm and room temperature (20-25°C), when applied to the hair, can be at least 400 mPa.s, or at least 1000 mPa.s, or at least 2000 mPa.s, or at least 3000 mPa.s, and can be up to 10,000 mPa.s.

To increase the viscosity, the composition may include one or more rheology modifiers, which can be synthetic or natural.

Examples include fatty alcohols, such as C₁₀-C₃₂ alcohols, e.g., C₁₂-C₂₂ alcohols, natural oils, organo clay materials, and polymers of acrylic acid and/or methacrylic acid, such as carbomers. Exemplary natural oils include mineral oils (mainly C₁₅-C₄₀ linear and branched aliphatic alkanes, with minor amounts of cycloalkanes), which may be sold as paraffinum liquidum. An exemplary organo clay thickening agent is disteardimonium hectorite.

Exemplary synthetic rheology modifiers include acrylic based polymers and copolymers. One class of acrylic based rheology modifiers are the carboxyl functional alkali-swellable and alkali-soluble thickeners (ASTs) produced by the free-radical polymerization of acrylic acid alone or in combination with other ethylenically unsaturated monomers. The polymers can be synthesized by solvent/precipitation as well as emulsion polymerization techniques. Exemplary synthetic rheology modifiers of this class include homopolymers of acrylic acid or methacrylic acid and copolymers polymerized from one or more monomers of acrylic acid, substituted acrylic acid, and salts and C₁-C₃₀ alkyl esters of acrylic acid and substituted acrylic acid. As defined herein, the substituted acrylic acid contains a substituent positioned on the alpha and/or beta carbon atom of the molecule, wherein in one aspect the substituent is independently selected from C₁₋₄ alkyl, -CN, and -COOH. Optionally, other ethylenically unsaturated monomers such as, for example, styrene, vinyl acetate, ethylene, butadiene, acrylonitrile, as well as mixtures thereof can be copolymerized into the backbone. The foregoing polymers are optionally crosslinked by a monomer that contains two or more moieties that contain ethylenic unsaturation. In one aspect, the crosslinker is selected from a polyalkenyl polyether of a polyhydric alcohol containing at least two alkenyl ether groups per molecule. Other Exemplary crosslinkers are selected from allyl ethers of sucrose and allyl ethers of pentaerythritol, and mixtures thereof. These polymers are more fully described in U.S. Patent No. 5,087,445; U.S. Patent No. 4,509,949; and U.S. Pat. No. 2,798,053.

In one aspect, the AST rheology modifier or thickener is a crosslinked homopolymer polymerized from acrylic acid or methacrylic acid and is generally referred to under the INCI name of Carbomer. Commercially available Carbomers include Carbopol^{™} polymers 934, 940, 941, 956, 980, and 996, as well as Carbopol Ultrez 10 and 30, Carbopol Clear, and Carbopol Style 2.0 polymers available from Lubrizol Advanced Materials, Inc. In a further aspect, the rheology modifier is selected from a crosslinked copolymer polymerized from a first monomer selected from one or more monomers of acrylic acid, substituted acrylic acid, salts of acrylic acid and salts of substituted acrylic acid and a second monomer selected from one or more C₁₀-C₃₀ alkyl acrylate esters of acrylic acid or methacrylic acid. In one aspect, the monomers can be polymerized in the presence of a steric stabilizer such as disclosed in U.S. Patent No. 5,288,814. Some of the forgoing polymers are designated under INCI nomenclature as Acrylates/C10-30 Alkyl Acrylate Crosspolymer and are commercially available under the trade names Carbopol^{®} 1342 and 1382, Carbopol^{®} Ultrez 20 and 21, Carbopol^{®} ETD 2020 and Pemulen^{®} TR-1 and TR-2 from Lubrizol Advanced Materials, Inc.

In another aspect, the auxiliary rheology modifier can be a crosslinked, linear poly(vinyl amide/acrylic acid) copolymer as disclosed in U.S. Patent No. 7,205,271.

Another class of synthetic rheology modifier suitable for use in the composition includes hydrophobically modified ASTs, commonly referred to as hydrophobically modified alkali-swellable and alkali-soluble emulsion (HASE) polymers. Typical HASE polymers are free radical addition polymers polymerized from pH sensitive or hydrophilic monomers (e.g., acrylic acid and/or methacrylic acid), hydrophobic monomers (e.g., C₁-C₃₀ alkyl esters of acrylic acid and/or methacrylic acid, acrylonitrile, styrene), an "associative monomer", and an optional crosslinking monomer. The associative monomer comprises an ethylenically unsaturated polymerizable end group, a non-ionic hydrophilic midsection that is terminated by a hydrophobic end group. The non-ionic hydrophilic midsection comprises a polyoxyalkylene group, e.g., polyethylene oxide, polypropylene oxide, or mixtures of polyethylene oxide/polypropylene oxide segments. The terminal hydrophobic end group is typically a C₈-C₄₀ aliphatic moiety. Exemplary aliphatic moieties are selected from linear and branched alkyl substituents, linear and branched alkenyl substituents, carbocyclic substituents, aryl substituents, aralkyl substituents, arylalkyl substituents, and alkylaryl substituents. In one aspect, associative monomers can be prepared by the condensation (e.g., esterification or etherification) of a polyethoxylated and/or polypropoxylated aliphatic alcohol (typically containing a branched or unbranched C₈-C₄₀ aliphatic moiety) with an ethylenically unsaturated monomer containing a carboxylic acid group (e.g., acrylic acid, methacrylic acid), an unsaturated cyclic anhydride monomer (e.g., maleic anhydride, itaconic anhydride, citraconic anhydride), a monoethylenically unsaturated monoisocyanate (e.g., α,α-dimethyl-m-isopropenyl benzyl isocyanate) or an ethylenically unsaturated monomer containing a hydroxyl group (e.g., vinyl alcohol, allyl alcohol). Polyethoxylated and/or polypropoxylated aliphatic alcohols are ethylene oxide and/or propylene oxide adducts of a monoalcohol containing the C₈-C₄₀ aliphatic moiety. Non-limiting examples of alcohols containing a C₈-C₄₀ aliphatic moiety are capryl alcohol, iso-octyl alcohol (2-ethyl hexanol), pelargonic alcohol (1-nonanol), decyl alcohol, lauryl alcohol, myristyl alcohol, cetyl alcohol, cetearyl alcohol (mixture of C₁₆-C₁₈ monoalcohols), stearyl alcohol, isostearyl alcohol, elaidyl alcohol, oleyl alcohol, arachidyl alcohol, behenyl alcohol, lignoceryl alcohol, ceryl alcohol, montanyl alcohol, melissyl, lacceryl alcohol, geddyl alcohol, and C₂-C₂₀ alkyl substituted phenols (e.g., nonyl phenol), and the like.

Exemplary HASE polymers are disclosed in U.S. Patent Nos. 3,657,175; 4,384,096; 4,464,524; 4,801,671; and 5,292,843. In addition, an extensive review of HASE polymers is found in Gregory D. Shay, Chapter 25, "Alkali-Swellable and Alkali-Soluble Thickener Technology A Review", Polymers in Aqueous Media - Performance Through Association, Advances in Chemistry Series 223, J. Edward Glass (ed.), ACS, pp. 457-494, Division Polymeric Materials, Washington, DC (1989). Commercially available HASE polymers are sold under the trade names, Aculyn^{®} 22 (INCI Name: Acrylates/Steareth-20 Methacrylate Copolymer), Aculyn^{®} 44 (INCI Name: PEG-150/Decyl Alcohol/SMDI Copolymer), Aculyn 46^{®} (INCI Name: PEG-150/Stearyl Alcohol/SMDI Copolymer), and Aculyn^{®} 88 (INCI Name: Acrylates/Steareth-20 Methacrylate Crosspolymer) from Dow Chemical, Chromapol^{™} 5 polymer and Novethix^{™} L-10 (INCI Name: Acrylates/Beheneth-25 Methacrylate Copolymer) from Lubrizol Advanced Materials, Inc.

In another embodiment, acid swellable associative polymers can be used with the hydrophobically modified, cationic polymers of the disclosed technology. Such polymers generally have cationic and associative characteristics. These polymers are free radical addition polymers polymerized from a monomer mixture comprising an acid sensitive amino substituted hydrophilic monomer (e.g., dialkylamino alkyl (meth)acrylates or (meth)acrylamides), an associative monomer (defined hereinabove), a lower alkyl (meth)acrylate or other free radically polymerizable comonomers selected from hydroxyalkyl esters of (meth)acrylic acid, vinyl and/or allyl ethers of polyethylene glycol, vinyl and/or allyl ethers of polypropylene glycol, vinyl and/or allyl ethers of polyethylene glycol/polypropylene glycol, polyethylene glycol esters of (meth)acrylic acid, polypropylene glycol esters of (meth)acrylic acid, polyethylene glycol/polypropylene glycol esters of (meth)acrylic acid), and combinations thereof. These polymers can optionally be crosslinked. By acid sensitive is meant that the amino substituent becomes cationic at low pH values, typically ranging from 0.5 to 6.5. Exemplary acid swellable associative polymers are commercially available under the trade name Structure^{®} Plus (INCI Name: Acrylates/Aminoacrylates/C₁₀-C₃₀ Alkyl PEG-20 Itaconate) from Nouryon, and Carbopol^{®} Aqua CC (INCI Name: Polyacrylates-1 Crosspolymer) from Lubrizol Advanced Materials, Inc. In one aspect, the acid swellable polymer is a copolymer of one or more C₁-C₅ alkyl esters of (meth)acrylic acid, C₁-C₄ dialkylamino C₁-C₆ alkyl methacrylate, PEG/PPG-30/5 allyl ether, PEG 20-25 C₁₀-C₃₀ alkyl ether methacrylate, hydroxy C₂-C₆ alkyl methacrylate crosslinked with ethylene glycol dimethacrylate. Other useful acid swellable associative polymers are disclosed in U.S. Patent No. 7,378,479.

Hydrophobically modified alkoxylated methyl glucosides, such as, for example, PEG-120 Methyl Glucose Dioleate, PEG-120 Methyl Glucose Trioleate, and PEG-20 Methyl Glucose Sesquistearate, available from Lubrizol Advanced Materials, Inc., under the trade names, Glucamate^{®} DOE-120, Glucamate^{™} LT, Glucamate^{™} VLT and Glucamate^{™} SSE-20, respectively, are also suitable as rheology modifiers.

Polysaccharides obtained from tree and shrub exudates, such as gum Arabic, gum gahatti, and gum tragacanth, as well as pectin; seaweed extracts, such as alginates and carrageenans (e.g., lambda, kappa, iota, and salts thereof); algae extracts, such as agar; microbial polysaccharides, such as xanthan, gellan, and wellan; cellulose ethers, such as ethylhexylethylcellulose, hydroxybutylmethylcellulose, hydroxyethylmethylcellulose, hydroxypropylmethylcellulose, methylcellulose, carboxymethylcellulose, hydroxyethylcellulose, and hydroxypropylcellulose; polygalactomannans, such as fenugreek gum, cassia gum, locust bean gum, tara gum, and guar gum; starches, such as corn starch, tapioca starch, rice starch, wheat starch, potato starch and sorghum starch can also be employed in the compositions herein as suitable rheology modifiers.

In one aspect, a suitable rheology modifier is fermentation derived cellulose (FDC). Fermentation derived cellulose (diutan gum *Sphingomonas* ferment extract) is a bio-based material obtained from microbial fermentation. Another naturally derived cellulose material suitable for use as a rheology modifier is microfibrous cellulose (MFC).

The rheology modifier(s) can be used alone or in combination and may be present in the composition, on an actives basis, at a total concentration of 0.001-50 wt.%, e.g., at least 0.1 wt. %, or at least 1 wt.%, such as up to 20 wt.%, or up to 10 wt.%, or up to 3 wt.%, based on the total weight of the composition.

### Surfactants

The hair care composition may also include one or more surfactants, such as anionic, cationic, amphoteric, and nonionic surfactants, as well as mixtures thereof.

In one aspect of the present technology, suitable anionic surfactants include but are not limited to alkyl sulfates, alkyl ether sulfates, alkyl sulphonates, alkaryl sulfonates, α-olefin-sulphonates, alkylamide sulphonates, alkarylpolyether sulphates, alkylamidoether sulfates, alkyl monoglyceryl ether sulfates, alkyl monoglyceride sulfates, alkyl monoglyceride sulfonates, alkyl succinates, alkyl sulfosuccinates, alkyl ether sulfosuccinates, alkyl sulfosuccinamates, alkyl amidosulfosuccinates; alkyl sulfoacetates, alkyl phosphates, alkyl ether phosphates, alkyl ether carboxylates, alkyl amidoethercarboxylates, acyl lactylates, alkyl isethionates, acyl isethionates, carboxylate salts and amino acid derived surfactants such as N-alkyl amino acids, N-acyl amino acids, as well as alkyl peptides. Mixtures of these anionic surfactants are also useful.

In one aspect, the cation moiety of the forgoing surfactants is selected from sodium, potassium, magnesium, ammonium, and alkanolammonium ions such as monoethanolammonium, diethanolammonium triethanolammonium ions, as well as monoisopropylammonium, diisopropylammonium and triisopropylammonium ions. In one embodiment, the alkyl and acyl groups of the foregoing surfactants contain from about 6 to about 24 carbon atoms in one aspect, from 8 to 22 carbon atoms in another aspect and from about 12 to 18 carbon atoms in a further aspect and may be unsaturated. The aryl groups in the surfactants are selected from phenyl or benzyl. The ether containing surfactants set forth above can contain from 1 to 10 ethylene oxide and/or propylene oxide units per surfactant molecule in one aspect, and from 1 to 3 ethylene oxide units per surfactant molecule in another aspect.

Examples of suitable anionic surfactants include the sodium, potassium, lithium, magnesium, and ammonium salts of laureth sulfate, trideceth sulfate, myreth sulfate, C₁₂-C₁₃ pareth sulfate, C₁₂-C₁₄ pareth sulfate, and C₁₂-C₁₅ pareth sulfate, ethoxylated with 1, 2, and 3 moles of ethylene oxide; the sodium potassium, lithium, magnesium, ammonium, and triethanolammonium salts of lauryl sulfate, coco sulfate, tridecyl sulfate, myristyl sulfate, cetyl sulfate, cetearyl sulfate, stearyl sulfate, oleyl sulfate, and tallow sulfate, disodium lauryl sulfosuccinate, disodium laureth sulfosuccinate, sodium cocoyl isethionate, sodium lauroyl isethionate, sodium lauroyl methyl isethionate, sodium C₁₂-C₁₄ olefin sulfonate, sodium laureth-6 carboxylate, sodium dodecylbenzene sulfonate, triethanolamine monolauryl phosphate, and fatty acid soaps, including the sodium, potassium, ammonium, and triethanolamine salts of a saturated and unsaturated fatty acids containing from about 8 to about 22 carbon atoms.

In one aspect, the amino acid surfactants are selected from a N-acyl amino acid of the formula: 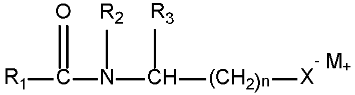 wherein R₁ is a saturated or unsaturated, straight or branched alkyl chain containing 7 to 17 carbon atoms, R₂ is H or a methyl group, R₃ is H, COO⁻M⁺, CH₂COO⁻M⁺ or COOH, n is 0 to 2, X is COO⁻ or SO₃⁻ and M independently represents H, sodium, potassium, ammonium or triethanolammonium.

In one aspect, the N-acyl amino acid surfactants represented by the formula immediately above are derived from taurates, glutamates, alanine, alaninates, sacosinates, aspartates, glycinates, and mixtures thereof.

Representative taurate surfactants conform to the formula: 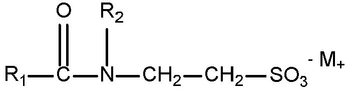 wherein R₁ is a saturated or unsaturated, straight or branched alkyl chain containing 7 to 17 carbon atoms in one aspect and 9 to 13 carbon atoms in another aspect, R₂ is H or methyl, and M is H, sodium, potassium, ammonium or triethanolammonium.

Non-limiting examples of taurate surfactants are potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, sodium methyl palmitoyl taurate, sodium methyl stearoyl taurate, and mixtures thereof.

Representative glutamate surfactants conform to the formula: 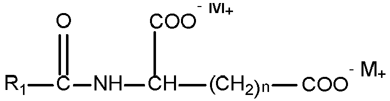 wherein R₁ is a saturated or unsaturated, straight or branched alkyl chain containing 7 to 17 carbon atoms in one aspect and 9 to 13 carbon atoms in another aspect, n is 0 to 2, and M independently is H, sodium, potassium, ammonium or triethanolammonium.

Non-limiting examples of glutamate surfactants are di-potassium capryloyl glutamate, di-potassium undecylenoyl glutamate, di-sodium capryloyl glutamate, di-sodium cocoyl glutamate, di-sodium lauroyl glutamate, di-sodium stearoyl glutamate, di-sodium undecylenoyl glutamate, potassium capryloyl glutamate, potassium cocoyl glutamate, potassium lauroyl glutamate, potassium myristoyl glutamate, potassium stearoyl glutamate, potassium undecylenoyl glutamate, sodium capryloyl glutamate, sodium cocoyl glutamate, sodium lauroyl glutamate, sodium myristoyl glutamate, sodium olivoyl glutamate, sodium palmitoyl glutamate, sodium stearoyl glutamate, sodium undecylenoyl glutamate, and mixtures thereof.

Representative alanine and alaninate surfactants conform to the formula: 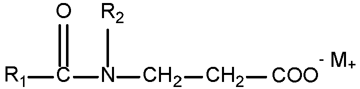 wherein R₁ is a saturated or unsaturated, straight or branched alkyl chain containing 7 to 17 carbon atoms in one aspect and 9 to 13 carbon atoms in another aspect, R₂ is H or methyl, and M is H, sodium, potassium, ammonium or triethanolammonium.

Non-limiting examples of alanine and alaninate surfactants are cocoyl methyl β-alanine, lauroyl β-alanine, lauroyl methyl β-alanine, myristoyl β-alanine, potassium lauroyl methyl β-alanine, sodium cocoyl alaninate, sodium cocoyl methyl β-alanine, sodium myristoyl methyl β-alanine, and mixtures thereof.

Representative glycinate surfactants conform to the formula: 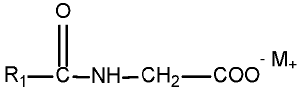 wherein R₁ is a saturated or unsaturated, straight or branched alkyl chain containing 7 to 17 carbon atoms in one aspect and 9 to 13 carbon atoms in another aspect, and M is H, sodium, potassium, ammonium or triethanolammonium.

Non-limiting examples of glycinate surfactants are sodium palmitoyl glycinate, sodium lauroyl glycinate, sodium cocoyl glycinate, sodium myristoyl glycinate, potassium lauroyl glycinate, potassium cocoyl glycinate, sodium stearoyl glycinate, and mixtures thereof.

Representative sarcosinate surfactants conform to the formula: 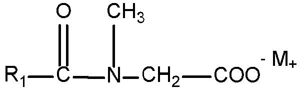 wherein R₁ is a saturated or unsaturated, straight or branched alkyl chain containing 7 to 17 carbon atoms in one aspect and 9 to 13 carbon atoms in another aspect, and M is H, sodium, potassium, ammonium or triethanolamine.

Non-limiting examples of sarcosinate surfactants are potassium lauroyl sarcosinate, potassium cocoyl sarcosinate, sodium cocoyl sarcosinate, sodium lauroyl sarcosinate, sodium myristoyl sarcosinate, sodium palmitoyl sarcosinate, and mixtures thereof.

Representative aspartate surfactants conform to the formula: 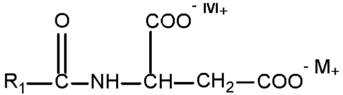 wherein R₁ is a saturated or unsaturated, straight or branched alkyl chain containing 7 to 17 carbon atoms in one aspect and 9 to 13 carbon atoms in another aspect, and M independently is H, sodium, potassium, ammonium or triethanolammonium.

Non-limiting examples of aspartate surfactants are sodium lauroyl aspartate, sodium myristoyl aspartate, sodium cocoyl aspartate, sodium caproyl aspartate, di-sodium lauroyl aspartate, di-sodium myristoyl aspartate, di-sodium cocoyl aspartate, di-sodium caproyl aspartate, potassium lauroyl aspartate, potassium myristoyl aspartate, potassium cocoyl aspartate, potassium caproyl aspartate, di-potassium lauroyl aspartate, di-potassium myristoyl aspartate, di-potassium cocoyl aspartate, di-potassium caproyl aspartate, and mixtures thereof.

Cationic surfactants present may act as conditioning agents and assist in the heating step by ensuring that the heating device runs smoothly over the hair fibers. While the surfactants may also help to increase viscosity, they are not considered as rheology modifiers for purposes of describing the exemplary embodiment herein.

The cationic surfactants can be any of the cationic surfactants known or previously used in the art of aqueous surfactant compositions. Suitable classes of cationic surfactants alkyl amines, alkyl imidazolines, ethoxylated amines, quaternary compounds, and quaternized esters. In addition, alkyl amine oxides can function as a cationic surfactant at a low pH.

Alkyl amine surfactants can be salts of primary, secondary and tertiary fatty C₁₂-C₂₂ alkylamines, substituted or unsubstituted, and substances sometimes referred to as "amidoamines". Examples of alkylamines and salts thereof include dimethyl cocamine, dimethyl palmitamine, dioctylamine, dimethyl stearamine, dimethyl soyamine, soyamine, myristyl amine, tridecyl amine, ethyl stearylamine, N-tallowpropane diamine, ethoxylated stearylamine, dihydroxy ethyl stearylamine, arachidylbehenylamine, dimethyl lauramine, stearylamine hydrochloride, soyamine chloride, stearylamine formate, N-tallowpropane diamine dichloride, and amodimethicone (INCI name for a silicone polymer and blocked with amino functional groups, such as aminoethylamino propylsiloxane).

Examples of amidoamines and salts thereof include stearamido propyl dimethyl amine, stearamidopropyl dimethylamine citrate, palmitamidopropyl diethylamine, and cocamidopropyl dimethylamine lactate.

Examples of alkyl imidazoline surfactants include alkyl hydroxyethyl imidazoline, such as stearyl hydroxyethyl imidazoline, coco hydroxyethyl imidazoline, ethyl hydroxymethyl oleyl oxazoline, and the like.

Examples of ethyoxylated amines include PEG-cocopolyamine, PEG-15 tallow amine, quaternium-52, and the like.

Among the quaternary ammonium compounds useful as cationic surfactants, some correspond to the general formula: (R⁵R⁶R⁷R⁸N⁺) E⁻, wherein R⁵, R⁶, R⁷, and R⁸ are independently selected from an aliphatic group having from 1 to about 22 carbon atoms, or an aromatic, alkoxy, polyoxyalkylene, alkylamido, hydroxyalkyl, aryl or alkylaryl group having 1 to about 22 carbon atoms in the alkyl chain; and E⁻ is a salt-forming anion such as those selected from halogen, (e.g., chloride, bromide), acetate, citrate, lactate, glycolate, phosphate, nitrate, sulfate, and alkylsulfate. The aliphatic groups can contain, in addition to carbon and hydrogen atoms, ether linkages, ester linkages, and other groups such as amino groups. The longer chain aliphatic groups, e.g., those of about 12 carbons, or higher (C₁₀-C₃₂ in the alkyl chain), can be saturated or unsaturated. In one aspect, the aryl groups are selected from phenyl and benzyl.

Exemplary quaternary ammonium surfactants include, but are not limited to cetyl trimethylammonium chloride (cetrimonium chloride), cetylpyridinium chloride, dicetyl dimethyl ammonium chloride, dihexadecyl dimethyl ammonium chloride, stearyl dimethyl benzyl ammonium chloride, dioctadecyl dimethyl ammonium chloride, dieicosyl dimethyl ammonium chloride, didocosyl dimethyl ammonium chloride, dihexadecyl dimethyl ammonium chloride, dihexadecyl dimethyl ammonium acetate, behenyl trimethyl ammonium chloride (behentrimonium chloride), benzalkonium chloride, benzethonium chloride, and di(coconutalkyl) dimethyl ammonium chloride, ditallowdimethyl ammonium chloride, di(hydrogenated tallow) dimethyl ammonium chloride, di(hydrogenated tallow) dimethyl ammonium acetate, ditallowdimethyl ammonium methyl sulfate, ditallow dipropyl ammonium phosphate, and ditallow dimethyl ammonium nitrate.

At low pH, amine oxides can protonate and behave similarly to N-alkyl amines. Examples include, but are not limited to, dimethyl- dodecylamine oxide, oleyldi(2-hydroxyethyl) amine oxide, dimethyltetradecylamine oxide, di(2-hydroxyethyl)-tetradecylamine oxide, dimethylhexadecylamine oxide, behenamine oxide, cocamine oxide, decyltetradecylamine oxide, dihydroxyethyl C₁₂-C₁₅ alkoxypropylamine oxide, dihydroxyethyl cocamine oxide, dihydroxyethyl lauramine oxide, dihydroxyethyl stearamine oxide, dihydroxyethyl tallowamine oxide, hydrogenated palm kernel amine oxide, hydrogenated tallowamine oxide, hydroxyethyl hydroxypropyl C₁₂-C₁₅ alkoxypropylamine oxide, lauramine oxide, myristamine oxide, cetylamine oxide, oleamidopropylamine oxide, oleamine oxide, palmitamine oxide, PEG-3 lauramine oxide, dimethyl lauramine oxide, potassium trisphosphonomethylamine oxide, soyamidopropylamine oxide, cocamidopropylamine oxide, stearamine oxide, tallowamine oxide, and mixtures thereof.

At low pH, amine oxides can protonate and behave similarly to N-alkyl amines. Examples include, but are not limited to, dimethyl- dodecylamine oxide, oleyldi(2-hydroxyethyl) amine oxide, dimethyltetradecylamine oxide, di(2-hydroxyethyl)-tetradecylamine oxide, dimethylhexadecylamine oxide, behenamine oxide, cocamine oxide, decyltetradecylamine oxide, dihydroxyethyl C₁₂-₁₅ alkoxypropylamine oxide, dihydroxyethyl cocamine oxide, dihydroxyethyl lauramine oxide, dihydroxyethyl stearamine oxide, dihydroxyethyl tallowamine oxide, hydrogenated palm kernel amine oxide, hydrogenated tallowamine oxide, hydroxyethyl hydroxypropyl C₁₂-C₁₅ alkoxypropylamine oxide, lauramine oxide, myristamine oxide, cetylamine oxide, oleamidopropylamine oxide, oleamine oxide, palmitamine oxide, PEG-3 lauramine oxide, dimethyl lauramine oxide, potassium trisphosphonomethylamine oxide, soyamidopropylamine oxide, cocamidopropylamine oxide, stearamine oxide, tallowamine oxide, and mixtures thereof.

In one aspect of the present technology, suitable amphoteric surfactants include but are not limited to alkyl betaines, e.g., lauryl betaine; alkylamido betaines, e.g., cocamidopropyl betaine and cocohexadecyl dimethyl betaine; alkylamido sultaines, e.g., cocamidopropyl hydroxysultaine; (mono- and di-) amphocarboxylates, e.g., sodium cocoamphoacetate, sodium lauroamphoacetate, sodium capryloamphoacetate, disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, and disodium capryloamphodipropionate; and mixtures thereof.

The foregoing amphoteric surfactants (i.e., the betaines and sultaines are disclosed without a counter ion, as one of ordinary skill in the art will recognize that the under the pH conditions of the compositions containing the amphoteric surfactants, these surfactants are either electrically neutral by virtue of having balancing positive and negative charges, or they contain counter ions such as alkali metal, alkaline earth or ammonium ions as a charge balancing moiety.

The nonionic surfactant can be any of the nonionic surfactants known or previously used in the art of aqueous surfactant compositions. Suitable nonionic surfactants include, but are not limited to, aliphatic (C₆-C₁₈) primary or secondary linear or branched chain acids, alcohols or phenols; alkyl ethoxylates; alkyl phenol alkoxylates (especially ethoxylates and mixed ethoxy/propoxy moieties); block alkylene oxide condensates of alkyl phenols; alkylene oxide condensates of alkanols; and ethylene oxide/propylene oxide block copolymers. Other suitable nonionic surfactants include mono- or dialkyl alkanolamides; alkyl polyglucosides (APGs); sorbitan fatty acid esters; polyoxyethylene sorbitan fatty acid esters; polyoxyethylene sorbitol esters; polyoxyethylene acids, and polyoxyethylene alcohols. Other examples of suitable nonionic surfactants include coco mono- or diethanolamide, coco glucoside, decyl diglucoside, lauryl diglucoside, coco diglucoside, polysorbate 20, 40, 60, and 80, ethoxylated linear alcohols, cetearyl alcohol, lanolin alcohol, stearic acid, glyceryl stearate, PEG-100 stearate, laureth 7, and oleth 20.

In another embodiment, non-ionic surfactants include alkoxylated methyl glucosides such as, for example, methyl gluceth-10, methyl gluceth-20, PEG-20 methyl glucose ether, PPG-10 methyl glucose ether, and PPG-20 methyl glucose ether, available from Lubrizol Advanced Materials, Inc., under the trade names, Glucam^{®} E10, Glucam^{®} E20, Glucam^{®} P10, and Glucam^{®} P20, respectively; and hydrophobically modified alkoxylated methyl glucosides, such as PEG 120 methyl glucose dioleate, PEG-120 methyl glucose trioleate, and PEG-20 methyl glucose sesquistearate, available from Lubrizol Advanced Materials, Inc., under the trade names, Glucamate^{®} DOE-120, Glucamate^{™} LT, Glucamate^{™} VLT and Glucamate^{™} SSE-20, respectively, are also suitable. Other exemplary hydrophobically modified alkoxylated methyl glucosides are disclosed in U.S. Patent Nos. 6,573,375 and 6,727,357.

Other surfactants which can be utilized in the composition are set forth in more detail in WO 99/21530, U.S. Patent Nos. 3,929,678, 4,565,647, 5,720,964, and 5,858,948. In addition, suitable surfactants are also described in McCutcheon's Emulsifiers and Detergents (North American and International Editions, by Schwartz, Perry and Berch).

While the amounts of the surfactant utilized in a composition comprising the exemplary thermally activated agent can vary widely depending on a desired application, the amounts which are often utilized generally range from 1 wt.% to 80 wt.%, on an actives basis. For example, the surfactant may be present in the composition, on an actives basis, at a total concentration of 0.001-20 wt.%, e.g., at least 0.1 wt.%.

### Conditioning agents

Conditioning agents include any material which is used to give a particular conditioning benefit to hair, scalp and/or skin. In hair treatment compositions, suitable conditioning agents are those which deliver one or more benefits relating to shine, softness, combability, antistatic properties, wet-handling, damage, manageability, body, and greasiness. Suitable conditioning agents for use in the hair coloring compositions of the present technology include relatively low molecular weight cationic compounds and cationic polymers, ampholytic polymers, silicones (e.g., silicone oils, cationic silicones, silicone gums, high refractive silicones, and silicone resins), organic conditioning oils (e.g., hydrocarbon oils, polyolefins, Natural oils, and ester oils), and combinations thereof.

Cationic Compounds are non-polymeric compounds that contain at least one cationic moiety or at least one moiety that can be ionized to form a cationic moiety. Typically, these cationic moieties are nitrogen containing groups such as quaternary ammonium salts or protonated amino groups. The cationic protonated amines can be primary, secondary, or tertiary amines. In one aspect, the cationic conditioning agents include quaternary nitrogen containing non-polymeric and polymeric materials that well known in the art for hair conditioning. In one aspect, the auxiliary conditioning agent different than (a) is a dialkyl quaternary ammonium compound corresponding to the general formula: (R⁷⁵)(R⁷⁶)(R⁷⁷)(R⁷⁸)N⁺CA⁻ wherein two of R⁷⁵, R⁷⁶, R⁷⁷ and R⁷⁸ are selected from an alkyl group containig from 12 to 22 carbon atoms or an aromatic, alkoxy, polyoxyalkylene, alkylamido, hydroxyalkyl, aryl or alkylaryl group having up to about 30 carbon atoms with or without an ester group; and the remainder of R⁷⁵, R⁷⁶, R⁷⁷ and R⁷⁸ are independently selected from an alkyl group containing from 1 to about 4 carbon atoms or an alkoxy, polyoxyalkylene, alkylamido, hydroxyalkyl, aryl or alkylaryl group having up to about 4 carbon atoms; and CA⁻ is a salt-forming anion such as those selected from halogen, (e.g. chloride, bromide), acetate, citrate, lactate, glycolate, phosphate, nitrate, sulfonate, sulfate, alkylsulfate, and alkyl sulfonate (e.g., methosulfate and ethosulfate) moieties. The alkyl groups can contain, in addition to carbon and hydrogen atoms, ether and/or ester linkages, and other groups such as amino groups. The longer chain alkyl groups, e.g., those of about 12 carbons, or higher, can be saturated or unsaturated or branched. In one embodiment, two of R⁷⁵, R⁷⁶, R⁷⁷ and R⁷⁸ are selected from an alkyl group containing from 12 to 22 carbon atoms in one aspect, from 14 to 20 carbon atoms in another aspect, and from 16 to 18 carbon atoms in a further aspect; the remainder of R⁷⁵, R⁷⁶, R⁷⁷ and R⁷⁸ are independently selected from CH₃, C₂H₅, C₂H₄OH, and mixtures thereof. Any two of R⁷⁵, R⁷⁶, R⁷⁷, and R⁷⁸ together with the nitrogen atom to which they are attached can be taken together to form a ring structure containing 5 to 6 carbon atoms, one of said carbon atoms can optionally be replaced with a heteroatom selected from nitrogen, oxygen or sulfur. CA⁻ is a salt-forming anion selected from halogen, (e.g. chloride, bromide), acetate, citrate, lactate, glycolate, phosphate, nitrate, sulfate, and alkylsulfate (e.g., methosulfate, ethosulfate).

Non-limiting examples of dialkyl quaternized ammonium compounds include dicocodimonium chloride; dicocodimonium bromide; dimyristyldimonium chloride; dimyristyldimonium bromide; dicetyldimonium chloride; dicetyldimonium bromide; dicetylmethylbenzylmonium chloride; distearyldimonium chloride; distearyldimonium bromide; dimetyldi(hydrogenated tallow)monium chloride; hydroxypropylbisstearylmonium chloride; distearylmethylbenzylmonium chloride; dibehenyl/diarachidyldimonium chloride; dibehenyl/diarachidyldimonium bromide; dibehenyldimonium chloride; dibehenyldimonium bromide; dibehenyldimonium methosulfate; dibehenylmethylbenzylmonium chloride; dihydrogenated tallow benzylmonium chloride; dihydrogenated tallowethyl hydroxyethylmonium methosulfate; dihydrogenated tallow hydroxyethylmonium methosulfate; di-C₁₂-C₁₅ alkyldimonium chloride; di-C₁₂-C₁₈ alkyldimonium chloride; di-C₁₄-C₁₈ alkyldimonium chloride; dicocoylethyl hydroxyethylmonium methosulfate; disoyoylethyl hydroxyethylmonium methosulfate; dipalmitoylethyldimonium chloride; dihydrogenated palmoylethyl hydroxyethylmonium methosulfate; dihydrogenated tallowamidoethyl hydroxyethylmonium chloride; dihydrogenated tallowamidoethyl hydroxyethylmonium methosulfate; dihydrogenated tallowoylethyl hydroxyethylmonium methosulfate; distearoylethyl hydroxyethylmonium methosulfate; and Quaternium-82.

In one aspect, the cationic compound is an asymmetric dialkyl quaternary ammonium compound corresponding to the general formula: (R⁸⁰)(R⁸¹)(R⁸²)(R⁸³)N⁺CA⁻ wherein R⁸⁰ is selected from an alkyl group containing from 12 to 22 carbon atoms or an aromatic, alkoxy, polyoxyalkylene, alkylamido, hydroxyalkyl, aryl or alkylaryl group containing up to about 22 carbon atoms; R⁸¹ is selected from an alkyl group containing from 5 to 12 carbon atoms or an aromatic, alkoxy, polyoxyalkylene, alkylamido, hydroxyalkyl, aryl or alkylaryl group containing up to about 12 carbon atoms; R⁸² and R⁸³ are independently selected from an alkyl group containing from 1 to about 4 carbon atoms or an aromatic, alkoxy, polyoxyalkylene, alkylamido, hydroxyalkyl, aryl or alkylaryl group containing up to about 4 carbon atoms; and CA⁻ is a salt-forming anion such as, for example, halogen, (e.g. chloride, bromide), acetate, citrate, lactate, glycolate, phosphate, nitrate, sulfate, and alkylsulfate (e.g., methosulfate, ethosulfate). The alkyl groups can contain, in addition to carbon and hydrogen atoms, ether linkages, ester linkages, and other moieties such as amino groups. The longer chain alkyl groups, e.g., those of about 12 carbons, or higher, can be saturated or unsaturated and/or straight or branched. In one embodiment, R⁸⁰ is selected from a non-functionalized alkyl group containing from 12 to 22 carbon atoms in one aspect, from 14 to 20 carbon atoms in another aspect, and from 16 to 18 carbon atoms in a further aspect; R⁸¹ is selected from a non-functionalized alkyl group containing from 5 to 12 carbon atoms in one aspect, from 6 to 10 carbon atoms in another aspect, and 8 carbon atoms in a further aspect; R⁸² and R⁸³ are independently selected from CH₃, C₂H₅, C₂H₄OH, and mixtures thereof; and CA⁻ is selected from Cl, Br, CH₃OSO₃, C₂H₅OSO₃, and mixtures thereof. In one aspect, R⁸⁰ is a straight, saturated non-functionalized alkyl group, and R⁸¹ is a branched, saturated non-functionalized alkyl group. In one aspect, the branched group of R⁸¹ is a straight, saturated alkyl group containing from 1 to 4 carbon atoms, and in another aspect, R⁸¹ is an alkyl group containing 2 carbon atoms.

Non-limiting examples of asymmetric dialkyl quaternized ammonium salt compounds include: stearylethylhexyldimonium chloride, stearylethylhexyldimonium bromide; stearyl ethylhexyl dimonium methosulfate; cetearyl ethylhexyldimonium methosulfate.

A number of quaternary nitrogen containing compounds their manufacturers and general descriptions of their chemical characteristics are found in the CTFA Dictionary and in the International Cosmetic Ingredient Dictionary, Vol. 1 and 2, 5th Ed., published by the Cosmetic Toiletry and Fragrance Association, Inc. (CTFA) (1993). The name assigned to the ingredients by the CTFA or by the manufacturer is used for convenience.

Other non-limiting examples of quaternary ammonium compounds useful as auxiliary conditioning agents different than (a) include acetamidopropyl trimonium chloride, behenamidopropyl ethyldimonium ethosulfate, cetethyl morpholinium ethosulfate, cocoamidopropyl ethyldimonium ethosulfate, dicetyldimonium chloride, hydroxyethyl behenamidopropyl dimonium chloride, Quaternium-18, Quaternium-26, Quaternium-27, Quaternium-53, Quaternium-63, Quaternium-70, Quaternium-72, Quaternium-76, Quaternium-87, PPG-9 diethylmonium chloride, PPG-25 diethylmonium chloride, PPG-40 stearalkonium chloride, isostearamidopropyl ethyldimonium ethosulfate, and mixtures thereof.

In one aspect, the auxiliary conditioning agent different than (a) is a quaternary nitrogen containing ether substituted, ethoxylated alkyl glucoside compound represented by the formula: 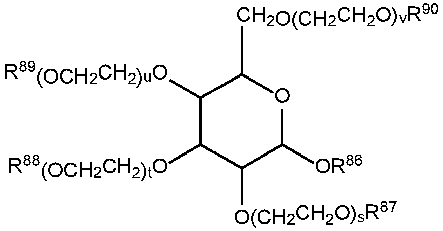 wherein R⁸⁶ represents C₁ to C₅ alkyl, e.g., methyl, ethyl, propyl; R⁸⁷, R⁸⁸, R⁸⁹ and R⁹⁰ independently represent hydrogen; a C₁ to C₂₂ alkyl group; a C₂ to C₂₂ alkenyl group; an acyl substituent represented by -C(O)R⁹⁵, where R⁹⁵ is selected from C₅ to C₂₁ alkyl or C₅ to C₂₁ alkenyl; and where at least one of R⁸⁷, R88, R⁸⁹ and R⁹⁰ represents a quaternary nitrogen moiety represented by the formula: 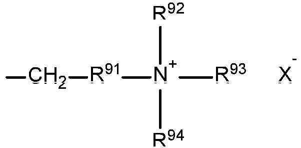 wherein R⁹¹ is a C₁ to C₅ alkylene, e.g., methylene, ethylene, propylene, or a C₁ to C₅ hydroxy substituted alkylene, e.g., hydroxyemethylene, hydroxyethylene, hydroxypropylene; and R⁹², R⁹³, and R⁹⁴ independently represent C₁ to C₂₂ alkyl, e.g., methyl, ethyl, propyl, butyl, decyl, dodecyl, hexadecyl, octadecyl, behenyl; C₆ to C₁₀ aryl, e.g, phenyl, tolyl, benzyl; and X⁻ is a salt forming anion, such as, for example, halogen, (e.g. chloride, bromide), acetate, citrate, lactate, glycolate, phosphate, nitrate, sulfate, and alkylsulfate (e.g., methosulfate, ethosulfate); and wherein the sum of s + t + u + v ranges from about 1 to about 200, or from about 5 to about 100, or from about 8 to about 50, or from about 10 to about 25.

In one aspect, R⁸⁶ is a methyl group; at least one of the R⁸⁷ to R⁹⁰ substituents is a quaternary nitrogen containing moiety, with the remaining R⁸⁷ to R⁹⁰ substituent(s) not substituted with the quaternary nitrogen moiety being selected from hydrogen; R⁹¹ is hydroxyalkylene, with two of R⁹² to R⁹⁴ representing methyl and the remaining substituent of R⁹² to R⁹⁴ that is not methyl is selected from a C₁₀ to C₂₂ alkyl or C₁₀ to C₂₂ alkenyl group.

The quaternary nitrogen containing ether substituted, ethoxylated alkyl glucoside compounds set forth under the structure immediately above are disclosed in U.S. Patent No. 5,1 38,043. In one aspect, a suitable quaternary nitrogen containing ether substituted, ethoxylated alkyl glucoside compound is Lauryl Methyl Gluceth-10 Hydroxypropyldimonium Chloride which is commercially available under the Gluquat^{™} 125 trade name from Lubrizol Advanced Materials, Inc.

Cationic polymers are also useful as conditioning agents alone or in combination with the other conditioning agents described herein. Suitable cationic polymers can be synthetically derived or natural polymers can be synthetically modified to contain cationic moieties. Polymeric quaternary ammonium moiety salt containing polymers can be prepared by the polymerization of a diallylamine such as dialkyldiallylammonium salt or copolymer thereof in which the alkyl group contains 1 to about 22 carbon atoms in one aspect and methyl or ethyl in another aspect. Copolymers containing a quaternary moiety derived from a dialkyldiallylammonium salt and an anionic component derived from anionic monomers of acrylic acid and methacrylic acid are suitable conditioning agents. Also suitable are, polyampholyte terpolymers having a cationic component prepared from a derivative of diallylamine, such as a dimethyldiallylammonium salt, an anionic component derived from anionic monomers of acrylic acid or 2-acrylamido-2-methylpropane sulfonic acid and a nonionic component derived from nonionic monomers of acrylamide. The preparation of such quaternary ammonium salt moiety containing polymers can be found, for example, in U.S. Patent. Nos. 3,288,770; 3,412,019; 4,772,462 and 5,275,809.

Non-limiting examples of such polymers are described in the CTFA International Cosmetic Ingredient Dictionary/Handbook via the CTFA website as well as the CTFA Cosmetic Ingredient Handbook, Ninth Ed., Cosmetic and Fragrance Assn., Inc., Washington D.C. (2002).

In one aspect, suitable cationic polymers include the chloride salts of the foregoing quaternized homopolymers and copolymers in which the alkyl group is methyl or ethyl, and are commercially available under the Merquat^{®} series of trademarks from Lubrizol Advanced Materials, Inc.

A homopolymer prepared from diallyl dimethyl ammonium chloride (DADMAC) having the CTFA name, Polyquaternium-6, is available under the Merquat 100 and Merquat 106 trademark. A copolymer prepared from DADMAC and acrylamide having the CTFA name, Polyquaternium-7, is sold under the Merquat 550 trademark. Another copolymer prepared from DADMAC and acrylic acid having the CTFA name, Polyquaternium-22, is sold under the Merquat 280 trademark. The preparation of Polyquaternium-22 and its related polymers is described in U.S. Patent. No. 4,772,462.

Also useful is an ampholytic terpolymer prepared from a nonionic component derived from acrylamide or methyl acrylate, a cationic component derived from DADMAC or methacrylamidopropyl trimethyl ammonium chloride (MAPTAC), and an anionic component derived from acrylic acid or 2-acrylamido-2-methylpropane sulfonic acid or combinations of acrylic acid and 2-acrylamido-2-methylpropane sulfonic acid. An ampholytic terpolymer prepared from acrylic acid, DADMAC and acrylamide having the CTFA name, Polyquarternium-39, is available under the Merquat Plus 3330 trademark. Another ampholytic terpolymer prepared from acrylic acid, methacrylamidopropyl trimethyl ammonium chloride (MAPTAC) and methyl acrylate having the CTFA name, Polyquarternium-47, is available under the Merquat 2001 trademark. Still another ampholytic terpolymer prepared from acrylic acid, MAPTAC and acrylamide having the CTFA name, Polyquarternium-53, is available under the Merquat 2003PR trademark. The preparation of such terpolymers is described in U.S. Patent. No. 5,275,809.

Other cationic polymers and copolymers suitable as conditioners in the hair coloring compositions of the disclosed technology have the CTFA names Polyquaternium-4, Polyquaternium-11, Polyquarternium-16, Polyquaternium-22, Polyquaternium-28, Polyquaternium-29, Polyquaternium-30, Polyquaternium-32, Polyquaternium-33, Polyquaternium-35, Polyquaternium-36, Polyquaternium-37, Polyquaternium-44, Polyquaternium-46, Polyquaternium-47, Polyquaternium-52, Polyquaternium-53, Polyquarternium-55, Polyquaternium-59, Polyquaternium-61, Polyquaternium-64, Polyquaternium-65, Polyquaternium-67, Polyquaternium-69, Polyquaternium-70, Polyquaternium-71, Polyquaternium-72, Polyquaternium-73, Polyquaternium-74, Polyquaternium-76, Polyquaternium-77, Polyquaternium-78, Polyquaternium-79, Polyquaternium-80, Polyquaternium-81, Polyquaternium-82, Polyquaternium-84, Polyquaternium-85, Polyquaternium-87, and PEG-2-cocomonium chloride.

Exemplary cationically modified natural polymers suitable for use in the hair coloring composition include cationically modified polysaccharide polymers, such as cationically modified cellulose and cationically modified starch derivatives modified with a quaternary ammonium halide moiety. Exemplary cationically modified cellulose polymers are salts of hydroxyethyl cellulose reacted with trimethyl ammonium substituted epoxide (CTFA, Polyquaternium-10). Other suitable types of cationically modified cellulose include the polymeric quaternary ammonium salts of hydroxyethyl cellulose reacted with lauryl dimethyl ammonium substituted epoxide (CTFA, Polyquaternium-24). Cationically modified potato starch having the CTFA name, Starch Hydroxypropyltrimonium Chloride, is available under the Sensomer^{™} CI-50 trademark, from Lubrizol Advanced Materials, Inc.

Other suitable cationically modified natural polymers include cationic polygalactomannan derivatives such as guar gum derivatives and cassia gum derivatives, e.g., CTFA: Guar Hydroxypropyltrimonium Chloride, Hydroxypropyl Guar Hydroxypropyltrimonium Chloride, and Cassia Hydroxypropyltrimonium Chloride. Guar hydroxypropyltrimonium chloride is commercially available under the Jaguar^{™} trade name series and the N-Hance trade name series from Ashland Inc. Cassia Hydroxypropyltrimonium Chloride is commercially available under the Sensomer^{™} CT-250 and Sensomer^{™} CT-400 trademarks from Lubrizol Advanced Materials, Inc.

In one aspect, the polymeric cationic an ampholytic polymers can be present from about 0.05 to about 5 wt.% or, from about 0.1 to about 3 wt.%, or from about 0.5 to about 2.0 wt.%, based on the total weight of the composition.

### Silicones

The silicone conditioning agent may comprise volatile silicones, non-volatile silicones, and mixtures thereof. If volatile silicones are present, they are typically employed as a solvent or carrier for commercially available forms of non-volatile silicone fluid conditioning agents such as oils and gums and resins. Volatile silicone fluids are often included in the conditioning package to improve silicone fluid deposition efficacy or to enhance the shine, sheen, or glossiness of the hair. Volatile silicone materials are frequently included in formulations to enhance sensory attributes (e.g., feel) on the hair, scalp, and skin.

In one aspect, the silicone conditioning agent is non-volatile and includes silicone oils, gums, resins, and mixtures thereof. By non-volatile is meant that the silicone has a very low vapor pressure at ambient temperature conditions (e.g., less than 2 mm Hg at 20°C). The non-volatile silicone conditioning agent has a boiling point above about 250°C in one aspect, above about 260°C in another aspect, and above about 275°C in a further aspect. Background information on silicones including sections discussing silicone oils, gums, and resins, as well as their manufacture, are found in Encyclopedia of Polymer Science and Engineering, vol. 15, 2d ed., pp 204-308, John Wiley & Sons, Inc. (1989).

### Silicone Oil

In one aspect, the silicone conditioning agent is silicone oil selected from a polyorganosiloxane material. In one aspect, the polyorganosiloxane material can be selected from polyalkylsiloxanes, polyarylsiloxanes, polyalkylarylsiloxanes, hydroxyl terminated polyalkylsiloxanes, polyarylalkylsiloxanes, amino functional polyalkylsiloxanes, quaternary functional polyalkylsiloxanes, and mixtures thereof.

In one aspect, the silicone oil conditioning agent includes polyorganosiloxanes represented by the formula: 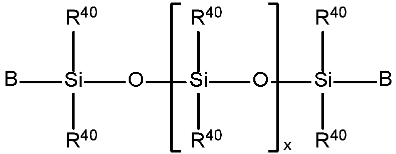 wherein B independently represents hydroxy, methyl, methoxy, ethoxy, propoxy, and phenoxy; R⁴⁰ independently represents methyl, ethyl, propyl, phenyl, methylphenyl, phenylmethyl, a primary, secondary, or tertiary amine, a quaternary group selected from a group selected from:

-R⁴¹-N(R⁴²)CH₂CH₂N(R⁴²)₂;

-R⁴¹-N(R⁴²)₂;

-R⁴¹-N⁺(R⁴²)₃CA⁻;

and

-R⁴¹-N(R⁴²)CH₂CH₂N⁺(R⁴²)H₂ CA⁻;

wherein R⁴¹ is a linear or branched, hydroxyl substituted or unsubstituted alkylene or alkylene ether moiety containing 2 to 10 carbon atoms; R⁴² is hydrogen, C₁-C₂₀ alkyl (e.g, methyl), phenyl or benzyl; q is an integer ranging from about 2 to about 8; CA⁻ is a halide ion selected from chlorine, bromine, iodine and fluorine; and x is an integer ranging from about 7 to about 8000, or from about 50 to about 5000, or from about 100 to about 3000, or from about 200 to about 1000.

In one aspect, the amino functional polyalkylsiloxane can be represented by the formula: 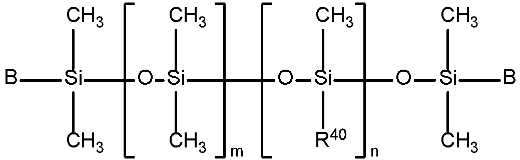 wherein B independently represents hydroxy, methyl, methoxy, ethoxy, propoxy, and phenoxy; and R⁴⁰ is selected from:

-R⁴¹-N(R⁴²)CH₂CH₂N(R⁴²)₂;

-R⁴¹-N(R⁴²)₂;

-R⁴¹-N⁺(R⁴²)₃CA⁻;

and

-R⁴¹-N(R⁴²)CH₂CH₂N⁺(R⁴²)H₂ CA⁻

wherein R⁴¹ is a linear or branched, hydroxyl substituted or unsubstituted alkylene or alkylene ether moiety containing 2 to 10 carbon atoms; R⁴² is hydrogen, C₁-C₂₀ alkyl (e.g, methyl), phenyl or benzyl; CA⁻ is a halide ion selected from chlorine, bromine, iodine and fluorine; and the sum of m+n ranges from about 7 to about 1000, or from about 50 to about 250, or from about 100 to about 200, subject to the proviso that m or n is not 0. In one aspect B is hydroxy and R⁴⁰ is -(CH₂)₃NH(CH₂)₃NH₂. In another aspect B is methyl and R⁴⁰ is -(CH₂)₃NH(CH₂)₃NH₂. In still another aspect B is methyl and R⁴⁰ is a quaternary ammonium moiety represented by -(CH₂)₃OCH₂CH(OH)CH₂N⁺(R⁴²)₃ CA⁻; wherein R⁴² and CA⁻ are as previously defined.

The silicone oil conditioning agents can have a viscosity ranging from about above about 25 to about 1,000,000 mPa·s at 25°C, or from about 100 to about 600,000 mPa·s, or from about 1000 to about 100,000 mPa·s, or from about 2,000 to about 50,000 mPa·s, or from about 4,000 to about 40,000 mPa·s. The viscosity is measured by means of a glass capillary viscometer as described by Dow Corning Corporate Test Method CTM004, dated July 20, 1970. In one aspect the silicone oils have an average molecular weight below about 200,000 daltons. The average molecular weight can typically range from about 400 to about 199,000, or from about 500 to about 150,000 daltons, or from about 1,000 to about 100,000 daltons, or from about 5,000 to about 65,000 daltons.

Exemplary silicone oil conditioning agents include, but are not limited to, polydimethylsiloxanes (dimethicones), polydiethylsiloxanes, polydimethyl siloxanes having terminal hydroxyl groups (dimethiconols), polymethylphenylsiloxanes, phenylmethylsiloxanes, amino functional polydimethylsiloxanes (amodimethicones), and mixtures thereof.

### Silicone Gum

Another silicone conditioning agent useful in the disclosed technology is a silicone gum. A silicone gum is a polyorganosiloxane material of the same general structure of the silicone oils set forth above wherein B independently represents hydroxy, methyl, methoxy, ethoxy, propoxy, and phenoxy; R⁴⁰ independently represents methyl, ethyl, propyl, phenyl, methylphenyl, phenylmethyl, and vinyl. Silicone gums have a viscosity measured at 25°C of greater than 1,000,000 mPa·s. The viscosity can be measured by means of a glass capillary viscometer as described above for the silicone oils. In one aspect the silicone gums have an average molecular weight about 200,000 daltons and above. The molecular weight can typically range from about 200,000 to about 1,000,000 daltons. It is recognized that the silicone gums described herein can also have some overlap with the silicone oils described previously. This overlap is not intended as a limitation on any of these materials.

Suitable silicone gums for use in the silicone component of the hair coloring compositions of the disclosed technology are polydimethylsiloxanes (dimethicones), optionally having terminal end groups such as hydroxyl (dimethiconols), polymethylvinylsiloxane, polydiphenylsiloxane, and mixtures thereof.

### Silicone Resins

Silicone resins can be included as a silicone conditioning agent suitable for use in the compositions of the disclosed technology. These resins are crosslinked polysiloxanes. The crosslinking is introduced through the incorporation of trifunctional and tetrafunctional silanes with monofunctional and/or difunctional silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetra-functional siloxane monomer units (and hence, a sufficient level of crosslinking) such that they form a rigid or hard film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. In one aspect, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and tetrachlorosilane, with the methyl substituted silanes being most commonly utilized.

Silicone materials and silicone resins can be identified according to a shorthand nomenclature system known to those of ordinary skill in the art as "MDTQ" nomenclature. Under this naming system, the silicone is described according to the presence of various siloxane monomer units which make up the silicone. The "MDTQ" nomenclature system is described in the publication entitled *"*Silicones: Preparation, Properties and Performance"; Dow Corning Corporation, 2005, and in U.S. Patent. No. 6,200,554.

Silicone materials and silicone resins can be identified according to a shorthand nomenclature system known to those of ordinary skill in the art as "MDTQ" nomenclature. Under this naming system, the silicone is described according to the presence of various siloxane monomer units which make up the silicone. The "MDTQ" nomenclature system is described in the publication entitled *"*Silicones: Preparation, Properties and Performance"; Dow Corning Corporation, 2005, and in U.S. Patent. No. 6,200,554.

### Volatile Silicones

The optional volatile silicones referred to above include linear polydimethylsiloxanes and cyclic polydimethylsiloxanes (cyclomethicones), and mixtures thereof. Volatile linear polydimethylsiloxanes (dimethicones) typically contain about 2 to about 9 silicon atoms, alternating with oxygen atoms in a linear arrangement. Each silicon atom is also substituted with two alkyl groups (the terminal silicon atoms are substituted with three alkyl groups), such as, for example, methyl groups. The cyclomethicones typically contain about 3 to about 7 dimethyl substituted silicon atoms in one aspect and from about 3 to about 5 in another aspect, alternating with oxygen atoms, in a cyclic ring structure. The term "volatile" means that the silicone has a measurable vapor pressure, or a vapor pressure of at least 2 mm of Hg at 20°C. The volatile silicones have a viscosity of 25 mPa·s or less at 25°C, or from about 0.65 about to about 10 mPa·s, or from about 1 to about 5 mPa·s, or from about 1.5 to about 3.5 mPa·s. A description of linear and cyclic volatile silicones is found in Todd and Byers, "Volatile Silicone Fluids for Cosmetics", Cosmetics and Toiletries, Vol. 91(1), pp. 27-32 (1976), and in Kasprzak, "Volatile Silicones", Soap/Cosmetics/Chemical Specialities, pp. 40-43 (December 1986).

Exemplary volatile linear dimethicones include, but are not limited to, hexamethyldisiloxane, octamethyltrisiloxane, decamethyltetrasiloxane, dodecamethylpentasiloxane and blends thereof. Volatile linear dimethicones and dimethicone blends are commercially available from Dow Corning Corporation as Dow Corning 200^{®} Fluid (e.g., product designations 0.65 CST, 1 CST, 1.5 CST, and 2 CST) and Dow Corning^{®} 2-1184 Fluid.

Exemplary volatile cyclomethicones are D4 cyclomethicone (octamethylcyclotetrasiloxane), D5 cyclomethicone (decamethylcyclopentasiloxane), D6 cyclomethicone, and blends thereof (e.g., D4/D5 and D5/D6). Volatile cyclomethicones and cyclomethicone blends are commercially available from Momentive Performance Materials Inc. as SF1173, SF1202, SF1256, and SF1258 silicone fluids, and Dow Corning Corporation as Dow Corning^{®} 244, 245, 246, 345, and 1401 silicone fluids. Blends of volatile cyclomethicones and volatile linear dimethicones also can be employed.

### Dimethicone Copolyols

Other suitable silicone oils include the dimethicone copolyols, which are linear or branched copolymers of dimethylsiloxane (dimethicone) modified with alkylene oxide units. The alkylene oxide units can be arranged as random or block copolymers. A generally useful class of dimethicone polyols are block copolymers having terminal and/or pendent blocks of polydimethylsiloxane and blocks of polyalkylene oxide, such as blocks of polyethylene oxide, polypropylene oxide, or both. The polyalkylene oxide terminal hydroxyl groups may be esterified with a C10-C22 fatty acid. An example of an esterified dimethicone copolyol is Dimethicone PEG-7 Isostearate marketed by Lubrizol Advanced Materials, Inc. under the Silsence DW18 trade name. Dimethicone copolyols can be water soluble or insoluble depending on the amount of polyalkylene oxide present in the dimethicone polymer and can be anionic, cationic, or nonionic in character.

In one aspect, the amount of silicone conditioner(s) in the compositions of the present technology should be sufficient to provide the desired conditioning performance to the hair, and generally ranges from about 0.01 to about 20 wt.%,or from about 0.05 to about 15 wt.%,or from about 0.1% to about 10 wt.%, or from about 1 to about 5 wt.%, based on the total weight of the composition.

### Hydrocarbon Oils

The conditioning component of the compositions of the disclosed technology can also contain hydrocarbon oil conditioners. Suitable hydrocarbon oils for use as conditioning agents in the compositions of the disclosed technology include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils typically contain about 12 to 19 carbon atoms. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2,2,4,4,6,6,8,8-dimethyl-10-methylundecane and 2,2,4,4,6,6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from BP Chemical Company.

Liquid polyolefin conditioning oils can be used in the hair coloring compositions of the present technology. The liquid polyolefin conditioning agents are typically poly-α-olefins that have been hydrogenated. Polyolefins for use herein can be prepared by the polymerization of C₄ to about C₁₄ olefinic monomers. Non-limiting examples of olefinic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. In one aspect of the disclosed technology, hydrogenated α-olefin monomers include, but are not limited to: 1-hexene to 1-hexadecenes, 1-octene to 1-tetradecene, and mixtures thereof.

Fluorinated or perfluorinated oils are also contemplated within the scope of the present technology. Fluorinated oils include perfluoropolyethers described in European Patent 0 486 135 and the fluorohydrocarbon compounds described in WO 93/11103. The fluoridated oils may also be fluorocarbons such as fluoramines, e.g., perfluorotributylamine, fluoridated hydrocarbons, such as perfluorodecahydronaphthalene, fluoroesters, and fluoroethers.

### Natural Oils

Natural oil conditioners are also useful in the practice of the disclosed technology and include but are not limited to peanut, sesame, avocado, coconut, calendula, cocoa butter, shey butter, almond, safflower, corn, cotton seed, sesame seed, walnut oil, castor, mango, olive, jojoba, palm, palm kernel, soybean, wheat germ, linseed, sunflower seed, grape seed, eucalyptus, lavender, vetiver, litsea, cubeba, lemon, sandalwood, rosemary, chamomile, savory, nutmeg, cinnamon, hyssop, caraway, orange, geranium, cade, and bergamot oils, musk rose oil fish oils, candelilla wax, carnauba wax, glycerol tricaprocaprylate; and mixtures thereof.

### Ester Oils

Ester oil conditioners include, but are not limited to, fatty esters having at least 10 carbon atoms. These fatty esters include esters derived from fatty acids or alcohols (e.g., mono-esters, polyhydric alcohol esters, and di- and tri-carboxylic acid esters). The fatty esters hereof may include or have covalently bonded thereto other compatible functionalities, such as amides and alkoxy moieties (e.g., ethoxy or ether linkages, etc.).

Exemplary fatty esters include, but are not limited to isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate. Other fatty esters suitable for use in the compositions of the disclosed technology are mono-carboxylic acid esters of the general formula R⁶⁰C(O)OR⁶¹, wherein R⁶⁰ and R⁶¹ are alkyl or alkenyl radicals, and the sum of carbon atoms in R⁶⁰ and R⁶¹ is at least 10 in one aspect, and at least 22 in another aspect of the disclosed technology.

Still other fatty esters suitable for use in the compositions of the disclosed technology are di- and tri-alkyl and alkenyl esters of carboxylic acids, such as esters of C₄ to C₈ dicarboxylic acids (e.g., C₁ to C₂₂ esters, preferably C₁ to C₆, of succinic acid, glutaric acid, adipic acid). Specific non-limiting examples of di- and tri-alkyl and alkenyl esters of carboxylic acids include isocetyl stearyol stearate, diisopropyl adipate, and tristearyl citrate. Other fatty esters suitable for use in the compositions of the disclosed technology are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono-and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Specific non-limiting examples of suitable synthetic fatty esters include: P-43 (C₈ to C₁₀ triester of trimethylolpropane), MCP-684 (tetraester of 3,3 diethanol-1,5 pentadiol), MCP 121 (C₈ to C₁₀ diester of adipic acid), all of which are available from ExxonMobil Chemical Company.

The amount of hydrocarbon and natural conditioning oils and ester oil conditioning agents can range from about 0.05 to about 10 wt.%, in one aspect, from about 0.5 to about 5 wt.% in another aspect, and from about 1 to about 3 wt.% in a further aspect, based on the total weight of the composition.

Other oily material conditioning agents that are useful in combination with the polymers of the disclosed technology include, for example, acetylated lanolin alcohols; lanolin alcohol concentrates; esters of lanolin fatty acids such as the isopropyl esters of lanolin fatty acid; polyol fatty acids; ethoxylated alcohols, such as ethoxylate and castor oils; sterols; sterol esters; sterol ethoxylates; and like materials.

### Preservatives

In one aspect, any preservative suitable for use in personal care can be used in the composition for modifying hair. Suitable preservatives include polymethoxy bicyclic oxazolidine, methyl paraben, propyl paraben, ethyl paraben, butyl paraben, benzyltriazole, DMDM hydantoin (also known as 1,3-dimethyl-5,5-dimethyl hydantoin), imidazolidinyl urea, phenoxyethanol, phenoxyethylparaben, methylisothiazolinone, methylchloroisothiazolinone, benzoisothiazolinone, triclosan, and suitable polyquaternium compounds as disclosed above (e.g., Polyquaternium-1).

In another aspect, acid-based preservatives are useful in the exemplary compositions. The use of acid-based preservatives facilitates the formulation of products in the low pH range. Lowering the pH of a formulation inherently provides an inhospitable environment for microbial growth in addition to being suited to the modifying process. Moreover, formulating at low pH enhances the efficacy of acid-based preservatives, and affords a personal care product which maintains an acidic pH balance on the skin. Any acid-based preservative that is useful in personal care products can be used in the exemplary compositions. In one aspect the acid preservative is a carboxylic acid compound represented by the formula: R³C(O)OH, wherein R³ represents hydrogen, a saturated and unsaturated hydrocarbyl group containing 1 to 8 carbon atoms or C₆ to C₁₀ aryl. In another aspect, R³ is selected from a hydrogen, a C₁ to C₈ alkyl group, a C₂ to C₈ alkenyl group, or phenyl. Exemplary acids are, but are not limited to, formic acid, acetic acid, propionic acid, sorbic acid, caprylic acid, and benzoic acid, and mixtures thereof.

In another aspect, suitable acids include but are not limited to, oxalic acid, succinic acid, glutaric acid, adipic acid, azelaic acid, fumaric acid, lactic acid, glyceric acid, tartronic acid, malic acid, tartaric acid, gluconic acid, citric acid, ascorbic acid, salicylic acid, phthalic acid, mandelic acid, benzoic acid, benzilic acid, and mixtures thereof.

Salts of the foregoing acids are also useful as long as they retain efficacy at low pH values. Suitable salts include the alkali metal (e.g., sodium, potassium, calcium) and ammonium salts of the acids enumerated above.

The acid-based preservatives and/or their salts can be used alone or in combination with non-acidic preservatives typically employed in personal care, home care, health care, and institutional and industrial care products.

The preservatives may comprise from 0.01 wt.% to 3.0 wt.% in one aspect, or from 0.1 wt.% to 1 wt. %, or from 0.3 wt.% to 1 wt. %, of the total weight of the hair modifying composition.

### Chelating Agents

Chelating agents can be employed to stabilize the composition against the deleterious effects of metal ions. When utilized, suitable chelating agents include tetrasodium EDTA (ethylene diamine tetraacetic acid) and salts thereof such as disodium EDTA, citric acid and salts thereof, cyclodextrins, pentasodium pantetate, and mixtures thereof.

Such suitable chelating agents can comprise 0.001 wt.% to 3 wt.%, such as 0.01 wt.% to 2 wt.%, or 0.01 wt.% to 1 wt.% of the total weight of the hair modifying composition.

### Auxiliary Antioxidants

Examples of antioxidants include, but are not limited to, water-soluble antioxidants such as sulfhydryl compounds and their derivatives (e.g., sodium metabisulfite and N-acetyl-cysteine, glutathione), lipoic acid and dihydrolipoic acid, stilbenoids such as resveratrol and derivatives, lactoferrin, and ascorbic acid and ascorbic acid derivatives (e.g., sodium isoascorbate, ascobyl-2-glucoside, ascorbyl palmitate and ascorbyl polypeptide). Oil-soluble antioxidants suitable for use in the compositions of the disclosed technology include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of the disclosed technology, include, but not limited to, extracts containing flavonoids and isoflavonoids and their derivatives (e.g., genistein and diadzein), extracts containing resveratrol and the like. Examples of such natural extracts include grape seed, green tea, pine bark, feverfew, parthenolide-free feverfew, oat extracts, pomelo extract, wheat germ extract, Hesperidin, Grape extract, Portulaca extract, Licochalcone, chalcone, 2,2'-dihydroxy chalcone, Primula extract, propolis, and the like.

Additional antioxidant may be selected from the following: butylhydroxytoluene (BHT), butylhydroxyanisole (BHA), tert-butylhydroquinone (TBHQ), 2,6,-di-tert-butyl-4-methylphenol, gallic acid esters such as propyl gallate, probucol, polyphenoles, ascorbic acid and its salts, enzymes such as catalase, superoxide dismutase and peroxidases; citric acid, citrates, monoglyceride esters, calcium metabisulfate, lactic acid, malic acid, succinic acid, tartaric acid, vitamin A or β-carotene, vitamins E and C, tocopherols such as vitamin E acetate, ascorbic acid esters such as ascorbyl palmitate and ascorbyl acetate, zinc, copper, mannitol, reduced glutathione, carotenoids such as cryptoxanthin, astaxanthin and lycopene; cysteine, uric acid, carnitine, taurine, tyrosine, lutein, zeaxanthin, N-acetyl-cysteine, carnosine, γ-glutamylcysteine, quercetin, lactoferrin, dihydrolipoic acid, tea catechins, retinyl palmitate and derivatives thereof, bisulfate, metabisulfite and sodium sulfite, chromans, chromens and their analogues, Lipochroman-6 [INCI: Dimethylmethoxy Chromanol], chelating agents of metals such as EDTA, sorbitol, phosphoric acid or dGlyage^{™} [INCI: Lysine HCl, Lecithin, Tripeptide-9 Citrulline]; extract of *Ginkgo Biloba,* plant extracts such as sage, pomegranate, rosemary, oregano, ginger, marjoram, cranberry, grape, tomato, green tea or black tea; oleoresin extract, extract of plants which contain phenols such as vanillin, ellagic acid and resveratrol; tertiary butylhydroquinone or mixtures thereof, metal salts with a valence of 2 such as selenium, cadmium, vanadium or zinc; α-lipoic acid, coenzyme Q, idebenone or derivatives thereof.

In one aspect, the amount of antioxidant present ranges from about 0.001 to 30 wt.%, or from 0.01 to 3 wt.%, based on the weight of the composition.

### Propellants

Where desired, any known aerosol propellant can be utilized to deliver the hair modifying compositions onto the surface of the hair to be straightened. Exemplary propellants include lower boiling hydrocarbons such as C₃-C₆ straight and branched chain hydrocarbons. Exemplary hydrocarbon propellants include propane, butane, isobutene, and mixtures thereof. Other suitable propellants include ethers, such as, dimethyl ether, hydrofluorocarbons, such as, 1,1-difluoroethane, and compressed gases, such as air and carbon dioxide.

In one aspect, these compositions can contain from 0.1 wt.% to 60 wt.%, or 0.5 to 35 wt.% propellant, based on the total weight of the composition.

### Fragrances and Perfumes

Fragrance and perfume components that may be used in the exemplary composition include natural and synthetic fragrances, perfumes, scents, and essences and any other substances which emit a fragrance. As the natural fragrances, there are those of vegetable origin, such as oil extracts from flowers (e.g., lily, lavender, rose, jasmine, neroli, ylang-ylang), stems and leaves (geranium, patchouli, petitgrain, peppermint), fruits (aniseed, coriander, fennel, needle juniper), fruit skin (bergamot, lemon, orange), roots (mace, angelica, celery, cardamom, costus, iris, sweet flag), woods (pine tree, sandalwood, guaiacum wood, cedar, rosewood, cinnamon), herbs and grasses (tarragon, lemongrass, sage, thyme), needles and twigs (spruce, pine, European red pine, stone pine), and resins and balsam (galbanum, elemi, benzoin, myrrh, frankincense, opopanax), and those of animal origin, such as musk, civet, castoreum, ambergris, or the like, and mixtures thereof.

Examples of synthetic fragrances and perfumes are the aromatic esters, ethers, aldehydes, ketones, alcohols, and hydrocarbons including benzyl acetate, phenoxyethyl isobutylate, p-tert-butylcyclohexyl acetate, linalyl acetate, dimethylbenzylcarbinyl acetate, phenylethyl acetate, linalyl benzoate, benzyl formate, ethylmethylphenyl glycinate, allylcyclohexyl propionate, styralyl propionate, and benzyl salicylate; benzylethyl ether; straight chain alkanals having 8 to 18 carbon atoms, citral, citronellal, citronellyloxyaldehyde, cyclamen aldehyde, hydroxycitronellal, lilial, and bougeonal; ionone compounds, α-isomethyl ionone, and methyl cedryl ketone; anethole, citronellol, eugenol, isoeugenol, geraniol, lavandulol, nerolidol, linalool, phenylethyl alcohol, and terpineol, alpha-pinene, terpenes (e.g., limonene), and balsams, and mixtures thereof.

### Botanicals

Suitable botanical agents useful herein may include, for example, extracts from Echinacea (e.g., sp. angustifolia, purpurea, pallida), yucca glauca, willow herb, basil leaves, hibiscus rosa-sinensis flower extract, hibiscus sabdariffa flower extract, Turkish oregano, carrot root, grapefruit, fennel seed, rosemary, tumeric, thyme, blueberry, bell pepper, blackberry, spirulina, black currant fruit, tea leaves, such as for, example, Chinese tea, black tea (e.g., var. Flowery Orange Pekoe, Golden Flowery Orange Pekoe, Fine Tippy Golden Flowery Orange Pekoe), green tea (e.g., var. Japanese, Green Darjeeling), oolong tea, coffee seed, dandelion root, date palm fruit, gingko leaf, green tea, hawthorn berry, licorice, sage, strawberry, sweet pea, tomato, vanilla fruit, comfrey, arnica, centella asiatica, cornflower, horse chestnut, ivy, magnolia, oat, pansy, skullcap, seabuckthorn, white nettle, and witch hazel. Botanical extracts may also include, for example, chlorogenic acid, glutathione, glycrrhizin, neohesperidin, quercetin, rutin, morin, myricetin, absinthe, and chamomile.

### Hair Fixing Agents/Film Formers

Hair fixing agents may be included in the hair coloring compositions, including polymer fixatives such as 3-aminopropyl methyl, dimethyl, reaction products of silicones and siloxanes with 2-ethyl-4,5-dihydrooxazole homopolymer, ethyl sulfates, such as Polysilicone-9.

Other commercially available hair fixative polymers/film former polymers can be used such as nonionic, cationic, and amphoteric hair setting polymers, cationic conditioning polymers, and combinations thereof. Conventional polymeric hair fixative and hair styling polymers, well known in the art, include natural gums and resins and neutral or anionic polymers of synthetic origin. Listings of commercially available hair fixative and conditioning fixative polymers can be readily found in the INCI Dictionary, in supplier websites, and in the trade literature. See, for example, the Polymer Encyclopedia published in Cosmetics & Toiletries®, 117(12), December 2002 (Allured Publishing Corporation, Carol Stream, IL).

Suitable commercially available nonionic polymers (i.e., neutral) used as hair styling or fixative polymers include, without limitation thereto, polyvinylpyrrolidone (PVP), polyvinylpyrrolidone/vinylacetate copolymer (PVP/VA), and the like. Commercially available cationic fixative polymers include, without limitation thereto, polymers having the INCI name, polyquaternium, such as polyquaternium-4, a diallyldimonium chloride/hydroxyethylcellulose copolymer (such as CELQUAT^{®} H-100 from Nouryon), polyquaternium-11, a quaternized vinyl pyrrolidone/dimethylaminoethyl methacrylate copolymer (such as GAFQUAT^{®} 734, 755, 755N, ISP); polyquaternium-16, a quaternized vinyl pyrrolidone/vinylimidazolium chloride copolymer (such as LUVIQUAT^{®} FC-370, BASF); polyquaternium-28, a vinylpyrrolidone/methacrylamidopropyltrimethylammonium chloride copolymer (such as GAFQUAT^{®} HS-100, ISP); polyquaternium-46, a quaternized vinylcaprolactam/vinylpyrrolidone/methylvinylimidazolium methosulfate copolymer; polyquaternium-55, a quaternized vinylpyrrolidone/ dimethylaminopropylmethylacrylamide/lauryldimethylpropylmethacrylamidoa mmonium chloride copolymer (such as STYLEZE^{™} W, ISP), and the like; and amino-substituted polymers which are cationic under acidic pH conditions, such as vinylcaprolactam/PVP/dimethylaminoethylmethacrylate copolymer (such as GAFFIX^{®} VC-713, ISP); PVP/dimethylaminoethylmethacrylate copolymer (such as Copolymer 845, ISP), PVP/DMAPA acrylates copolymer (such as STYLEZE^{™} CC-10, ISP), the pyrrolidone carboxylic acid salt of chitosan, having the INCI name, Chitosan PCA (such as KYTAMER^{®} PC, Amerchol), and the like.

Suitable amphoteric fixative polymers include, without limitation thereto, octylacryamide/acrylates/butylaminoethylmethacrylate copolymer (such as the AMPHOMER^{®} polymers, Nouryon), acrylates/lauryl acrylate/stearyl acrylate/ethylamine oxide methacrylate copolymers (such as the DIAFORMER^{®} polymers, Clariant Corp.), and the like.

Film-forming polymers such as polyacrylic acid and sodium polyacrylate polymer fixatives, such as Fixate^{™} RSP available from Lubrizol Advanced Materials, Inc., Cleveland, Ohio, are also suitable fixatives.

The hair fixing agent may be present in the composition at from 0.001 wt.% to 20 wt.%, such as at least 0.1 wt.%, or up to 5 wt.%.

### Emollients, Humectants and Emulsifiers

Exemplary emulsifiers include but are not limited to C₁₂-C₁₈ fatty alcohols; alkoxylated C₁₂-C₁₈ fatty alcohols;C₁₂-C₁₈ fatty acids; and alkoxylated C₁₂-C₁₈ fatty acids, the alkoxylates each having 10 to 30 units of ethylene oxide, propylene oxide, and combinations of ethylene oxide/propylene oxide; C₈-C₂₂ alkyl mono- and oligoglycosides; ethoxylated sterols; partial esters of polyglycerols; esters and partial esters of polyols having 2 to 6 carbon atoms and saturated and unsaturated fatty acids having 12 to 30 carbon atoms; partial esters of polyglycerols; and organosiloxanes; and combinations thereof.

The fatty alcohols, acids and alkoxylated fatty alcohols and fatty acids are as described in the emollient description above. In one aspect of the disclosed technology, the fatty alcohols and fatty acids each are ethoxylated with 10 to 30 units of ethylene oxide.

The C₈-C₂₂ alkyl mono- and oligoglycoside emulsifiers are prepared by reacting glucose or an oligosaccharide with primary fatty alcohols having 8 to 22 carbon atoms. Products which are obtainable under the trademark Plantacare^{®} comprise a glucosidically bonded C₈-C₁₆ alkyl group on an oligoglucoside residue whose average degree of oligomerization is 1 to 2. Exemplary alkyl glucosides and oligoglycosides are selected from octyl glucoside, decyl glucoside, lauryl glucoside, palmityl glucoside, isostearyl glucoside, stearyl glucoside, arachidyl glucoside and behenyl glucoside, and mixtures thereof.

Exemplary ethoxylated sterols include ethoxylated vegetable oil sterols such as, for example, soya sterols. The degree of ethoxylation is greater than about 5 in one aspect, and at least about 10 in another aspect. Suitable ethoxylated sterols are PEG-10 Soy Sterol, PEG-16 Soy Sterol and PEG-25 Soy Sterol.

The partial esters of polyglycerols have 2 to 10 glycerol units and are esterified with 1 to 4 saturated or unsaturated, linear or branched, optionally hydroxylated C₈-C₃₀ fatty acid residues. Representative partial esters of polyglycerols include diglycerol monocaprylate, diglycerol monocaprate, diglycerol monolaurate, triglycerol monocaprylate, triglycerol monocaprate, triglycerol monolaurate, tetraglycerol monocaprylate, tetraglycerol monocaprate, tetraglycerol monolaurate, pentaglycerol monocaprylate, pentaglycerol monocaprate, pentaglycerol monolaurate, hexaglycerol monocaprylate, hexaglycerol monocaprate, hexaglycerol monolaurate, hexaglycerol monomyristate, hexaglycerol monostearate, decaglycerol monocaprylate, decaglycerol monocaprate, decaglycerol monolaurate, decaglycerol monomyristate, decaglycerol monoisostearate, decaglycerol monostearate, decaglycerol monooleate, decaglycerol monohydroxystearate, decaglycerol dicaprylate, decaglycerol dicaprate, decaglycerol dilaurate, decaglycerol dimyristate, decaglycerol diisostearate, decaglycerol distearate, decaglycerol dioleate, decaglycerol dihydroxystearate, decaglycerol tricaprylate, decaglycerol tricaprate, decaglycerol trilaurate, decaglycerol trimyristate, decaglycerol triisostearate, decaglycerol tristearate, decaglycerol trioleate, decaglycerol trihydroxystearate, and mixtures thereof.

The saturated C₁₂-C₃₀ fatty alcohol emulsifiers are as described in the emollient description set forth above. In one aspect of the disclosed technology, the fatty alcohol emulsifier is selected from but not limited to cetyl alcohol, stearyl alcohol, arachidyl alcohol, behenyl alcohol and lanolin alcohol or mixtures of these alcohols, and as are obtainable in the hydrogenation of unsaturated vegetable oil and animal fatty acids.

The emulsifiers based on the esters and partial esters of polyols having 2 to 6 carbon atoms and linear saturated and unsaturated fatty acids having 12 to 30 carbon atoms are, for example, the monoesters and diesters of glycerol or ethylene glycol or the monoesters of propylene glycol with saturated and unsaturated C₁₂-C₃₀ fatty acids (e.g., PEG-3 Glyceryl Cocoate).

The partially esterified polyglycerol emulsifiers include 2 to about 10 glycerol units and esterified with 1 to 5 saturated or unsaturated, linear or branched, optionally hydroxylated C₈-C₃₀ fatty acid residues.

In one aspect of the disclosed technology, the emulsifier can be present in an amount ranging from about 0.5 wt.% to about 12 wt.%, from about 1 wt.% to about 15 wt.% in another aspect, and from about 5 wt.% to about 10 wt.% in a further aspect, based on the total weight of the personal care, home care, health care, and institutional care composition in which they are included.

Suitable emollients include but are not limited to an emollient selected from silicone fluids (e.g., volatile silicone oils and non-volatile silicone oils described above); mineral oils; petrolatums; vegetable oils; fish oils; fatty alcohols; fatty acids; fatty acid and fatty alcohol esters; alkoxylated fatty alcohols; alkoxylated fatty acid esters; benzoate esters; Guerbet esters; alkyl ether derivatives of polyethylene glycols, such as, for example methoxypolyethylene glycol (MPEG); and polyalkylene glycols; lanolin and lanolin derivatives; and the like.

Mineral oils and petrolatums include cosmetic, USP and NF grades and are commercially available from Penreco under the Drakeol^{®} and Penreco^{®} trade names. Mineral oil includes hexadecane and paraffin oil.

Suitable fatty alcohol emollients include but are not limited to fatty alcohols containing 8 to 30 carbon atoms. Exemplary fatty alcohols include capryl alcohol, pelargonic alcohol, capric alcohol, lauryl alcohol, myristyl alcohol, cetyl alcohol, isocetyl alcohol, stearyl alcohol, isostearyl alcohol, cetearyl alcohol, oleyl alcohol, ricinoleyl alcohol, arachidyl alcohol, icocenyl alcohol, behenyl alcohol, and mixtures thereof.

Suitable fatty acid emollients include but are not limited to fatty acids containing 10 to 30 carbon atoms. Exemplary fatty acids are selected from capric acid, lauric acid, myristic acid, palmitic acid, stearic acid, oleic acid, linoleic acid, arachidic acid, behenic acid, and mixtures thereof. Exemplary of the fatty acid and fatty alcohol ester emollients include but are not limited to hexyl laurate, decyl oleate, isopropyl stearate, isopropyl isostearate, butyl stearate, octyl stearate, cetyl stearate, myristyl myristate, octyldodecyl stearoylstearate, isostearyl hydroxystearate, octylhydroxystearate, diisopropyl adipate, isopropyl myristate, isopropyl palmitate, ethyl hexyl palmitate, isodecyl oleate, isodecyl neopentanoate, diisopropyl sebacate, isostearyl lactate, lauryl lactate, diethyl hexyl maleate, PPG-14 butyl ether and PPG-2 myristyl ether propionate, cetearyl octanoate, and mixtures thereof.

Alkoxylated fatty alcohol emollients are ethers formed from the reaction of a fatty alcohol with an alkylene oxide, generally ethylene oxide or propylene oxide. Suitable ethoxylated fatty alcohols are adducts of fatty alcohols and polyethylene oxide. In one aspect of the disclosed technology, the ethoxylated fatty alcohols can be represented by the formula R'-(OCH₂CH₂)_{n'}-OH, wherein R' represents the aliphatic residue of the parent fatty alcohol and n represents the number of molecules of ethylene oxide. In another aspect of the disclosed technology, R' is derived from a fatty alcohol containing 8 to 30 carbon atoms. In one aspect, n' is an integer ranging from 2 to 50, 3 to 25 in another aspect, and 3 to 10 in a further aspect. In a still further aspect, R' is derived from a fatty alcohol emollient set forth above. Exemplary ethoxylated fatty alcohols are but are not limited to capryl alcohol ethoxylate, lauryl alcohol ethoxylate, myristyl alcohol ethoxylate, cetyl alcohol ethoxylate, stearyl alcohol ethoxylate, cetearyl alcohol ethoxylate oleyl alcohol ethoxylate, and, behenyl alcohol ethoxylate, wherein the number of ethylene oxide units in each of the foregoing ethoxylates can range from 2 and above in one aspect, and from 2 to about 150 in another aspect. It is to be recognized that the propoxylated adducts of the foregoing fatty alcohols and mixed ethoxylated/propoxylated adducts of the foregoing fatty alcohols are also contemplated within the scope of the disclosed technology. The ethylene oxide and propylene oxide units of the ethoxylated/propoxylated fatty alcohols can be arranged in random or in blocky order.

More specific examples of ethoxylated alcohols are but are not limited to Beheneth 5-30 (the 5-30 meaning the range of repeating ethylene oxide units), Ceteareth 2-100 (e.g., ceteareth-20), Ceteth 1-45, Cetoleth 24-25, Choleth 10-24, Coceth 3-10, C9-11 pareth 3-8, C11-15 pareth 5-40, C11-21 Pareth 3-10, C12-13 pareth 3-15, Deceth 4-6, Dodoxynol 5-12, Glycereth 7-26, Isoceteth 10-30, Isodeceth 4-6, Isolaureth 3-6, isosteareth 3-50, Laneth 5-75, Laureth 1-40, Nonoxynol 1-120, Nonylnonoxynol 5-150, Octoxynol 3-70, Oleth 2-50 (e.g., Oleth-20), PEG 4-350, Steareth 2-100, and Trideceth 2-10.

Specific examples of propoxylated alcohols are but are not limited to PPG-10 Cetyl Ether, PPG-20 Cetyl Ether, PPG-28 Cetyl Ether, PPG-30 Cetyl Ether, PPG-50 Cetyl Ether, PPG-2 Lanolin Alcohol Ether, PPG-5 Lanolin Alcohol Ether, PPG-10 Lanolin Alcohol Ether, PPG-20 Lanolin Alcohol Ether, PPG-30 Lanolin Alcohol Ether, PPG-4 Lauryl Ether, PPG-7 Lauryl Ether, PPG-10 Oleyl Ether, PPG-20 Oleyl Ether, PPG-23 Oleyl Ether, PPG-30 Oleyl Ether, PPG-37 Oleyl Ether, PPG-50 Oleyl Ether, PPG-11 Stearyl Ether, PPG-15 Stearyl Ether, PPG-2 Lanolin Ether, PPG-5 Lanolin Ether, PPG-10 Lanolin Ether, PPG-20 Lanolin Ether, PPG-30 Lanolin Ether, and PPG-1 Myristyl Ether.

Specific examples of ethoxylated/propoxylated alcohols are but are not limited to PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

Alkoxylated fatty acid emollients are formed when a fatty acid is reacted with an alkylene oxide or with a pre-formed polymeric ether. The resulting product may be a monoester, diester, or mixture thereof. Suitable ethoxylated fatty acid ester emollients suitable for use in the disclosed technology are products of the addition of ethylene oxide to fatty acids. The product is a polyethylene oxide ester of a fatty acid. In one aspect of the disclosed technology, the ethoxylated fatty acid esters can be represented by the formula R"-C(O)O(CH₂CH₂O)_{n"}-H, wherein R" represents the aliphatic residue of a fatty acid and n represents the number of molecules of ethylene oxide. In another aspect, n" is an integer ranging from 2 to 50, 3 to 25 in another aspect, and 3 to 10 in a further aspect. In still another aspect of the disclosed technology, R" is derived from a fatty acid containing 8 to 24 carbon atoms. In a still further aspect, R" is derived from a fatty acid emollient set forth above. It is to be recognized that propoxylated and ethoxylated/propoxylated products of the foregoing fatty acids are also contemplated within the scope of the disclosed technology. Exemplary alkoxylated fatty acid esters include but are not limited to capric acid ethoxylate, lauric acid ethoxylate, myristic acid ethoxylate, stearic acid ethoxylate, oleic acid ethoxylate, coconut fatty acid ethoxylate, and polyethylene glycol 400 propoxylated monolaurate, wherein the number of ethylene oxide units in each of the foregoing ethoxylates can range from 2 and above in one aspect, and from 2 to about 50 in another aspect. More specific examples of ethoxylated fatty acids are PEG-8 distearate (the 8 meaning the number of repeating ethylene oxide units), PEG-8 behenate, PEG-8 caprate, PEG-8 caprylate, PEG-8 caprylate/caprate, PEG cocoates (PEG without a number designation meaning that the number of ethylene oxide units ranges from 2 to 50), PEG-15 dicocoate, PEG-2 diisononanoate, PEG-8 diisostearate, PEG-dilaurates, PEG-dioleates PEG-distearates, PEG Ditallates, PEG-isostearates, PEG-jojoba acids, PEG-laurates, PEG-linolenates, PEG-myristates, PEG-oleates, PEG-palmitates, PEG-ricinoleates, PEG-stearates, PEG-tallates, and the like. Guerbet ester emollients are formed from the esterification reaction of a Guerbet alcohol with a carboxylic acid. Guerbet ester emollients are commercially available from the Noveon Consumer Specialties Division of Lubrizol Advanced Materials, Inc. under product designations G-20, G-36, G-38, and G-66. Lanolin and lanolin derivatives are selected from lanolin, lanolin wax, lanolin oil, lanolin alcohols, lanolin fatty acids, alkoxylated lanolin, isopropyl lanolate, acetylated lanolin alcohols, and combinations thereof. Lanolin and lanolin derivatives are commercially available from the Lubrizol Advanced Materials, Inc. under the trade names Lanolin LP 108 USP, Lanolin USP AAA, Acetulan^{™}, Ceralan^{™}, Lanocerin^{™}, Lanogel^{™} (product designations 21 and 41), Lanogene^{™}, Modulan^{™}, Ohlan^{™}, Solulan^{™} (product designations 16, 75, L-575, 98, and C-24), Vilvanolin^{™} (product designations C, CAB, L-101, and P). The emollient(s) can be utilized in an amount ranging from about 0.5 wt.% to about 30 wt.% by weight of the total personal care composition in one aspect 0.1 wt.% to 25 wt.% in another aspect, and 5 wt.% to 20 wt.% in a further aspect. While emollients are generally employed in personal care compositions, they can be employed in home care, health care, and institutional care compositions in the same wt. ratios as set forth for personal care compositions so long as they effect a desired physical attribute (e.g., humectant properties) in such compositions.

Suitable humectants include allantoin, pyrrolidonecarboxylic acid and its salts, hyaluronic acid and its salts, sorbic acid and its salts, urea, lysine, arginine, cystine, guanidine, and other amino acids, polyhydroxy alcohols such as glycerin, propylene glycol, hexylene glycol, hexanetriol, ethoxydiglycol, dimethicone copolyol, methyl glucose dioleate, methyl glucose sesquistearate and sorbitol, and the esters thereof, polyethylene glycol, glycolic acid and glycolate salts (e.g. ammonium and quaternary alkyl ammonium), lactic acid and lactate salts (e.g. ammonium and quaternary alkyl ammonium), sugars and starches, sugar and starch derivatives (e.g. alkoxylated glucose), panthenols such as dl-panthenol, lactamide monoethanolamine, acetamide monoethanolamine, and the like, and mixtures thereof. In one embodiment the humectants include the C₃-C₆ diols and triols, such as glycerin, propylene glycol, hexylene glycol, hexanetriol, and the like, and mixtures thereof. Such suitable humectants typically comprise about 1 wt.% to about 10 wt. %, preferably about 2 wt.% to about 8 wt. %, and more preferably about 3 wt.% to about 5 wt.% of the total weight of the personal care compositions of the disclosed technology.

### Buffer Agents

Buffering agents can be used in the exemplary compositions. Suitable buffering agents include alkali or alkali earth metal carbonates, phosphates, bicarbonates, citrates, borates, acetates, acid anhydrides, succinates, and the like, such as sodium phosphate, sodium citrate, sodium acetate, sodium bicarbonate, and sodium carbonate.

### pH adjusters

The pH of the composition can be from to 1.5-9.5, e.g., at least 2.0, or at least 2.5. In some embodiments, the pH is up to 4.0, or up to 6.5, or up to 8. To provide the selected pH, the composition may include one or more pH modifiers selected from organic and inorganic acids and bases.

The pH of the composition can be adjusted with any combination of acidic and/or basic pH adjusting agents known to the art. Acidic materials include organic acids and inorganic acids, in particular, monocarboxylic acids, dicarboxylic acids, and tricarboxylic acids, for example and not limited to, acetic acid, citric acid, tartaric acid, alpha-hydroxy acids, beta-hydroxy acids, salicylic acid, malic acid, itaconic acid, maleic acid, alginic acid, glutamic acid, galacteric acid, fumaric acid, succinic acid, benzoic acid, etidronic acid, and amino acids (glycine, taurine, alanine, cysteine, cystine, creatine, valine, glutamine, leucine, arginine, lysine, etc.) and natural fruit acids, or inorganic acids, for example, hydrochloric acid, nitric acid, sulfuric acid, sulfamic acid, phosphoric acid, and combinations thereof.

Other acids can be used such as carboxylic acids, such as alpha-hydroxy acid (AHA), beta-hydroxy acid (BHA), alpha-amino acid, alpha-keto acids (AKAs), and mixtures thereof. In such cosmeceuticals, AHAs can include, but are not limited to, lactic acid, glycolic acid, fruit acids, such as malic acid, citric acid, tartaric acid, extracts of natural compounds containing AHA, such as apple extract, apricot extract, and the like, honey extract, 2-hydroxyoctanoic acid, glyceric acid (dihydroxypropionic acid), tartronic acid (hydroxypropanedioic acid), gluconic acid, mandelic acid, benzilic acid, azelaic acid, alpha-lipoic acid, salicylic acid, AHA salts and derivatives, such as arginine glycolate, ammonium glycolate, sodium glycolate, arginine lactate, ammonium lactate, sodium lactate, alpha-hydroxybutyric acid, alpha-hydroxyisobutyric acid, alpha-hydroxyisocaproic acid, alpha-hydroxyisovaleric acid, atrolactic acid, and the like. BHAs can include, but are not limited to, 3-hydroxy propanoic acid, beta-hydroxybutyric acid, beta-phenyl lactic acid, beta-phenylpyruvic acid, and the like. Alpha-amino acids include, without being limited thereto, alpha-amino dicarboxylic acids, such as aspartic acid, glutamic acid, and mixtures thereof, sometimes employed in combination with fruit acid. AKAs include pyruvic acid. In some antiaging compositions, the acidic active agent may be retinoic acid, a halocarboxylic acid, such as trichloroacetic acid, an acidic antioxidant, such as ascorbic acid (vitamin C), a mineral acid, phytic acid, lysophosphatidic acid, and the like. Some acidic anti-acne actives, for example, can include salicylic acid, derivatives of salicylic acid, such as 5-octanoylsalicylic acid, retinoic acid, and its derivatives.

Basic materials include inorganic and organic bases, and combinations thereof. Examples of inorganic bases include but are not limited to the alkali metal hydroxides (especially sodium, potassium, and ammonium), and alkali metal salts such as sodium borate (borax), sodium phosphate, sodium pyrophosphate, and the like; and mixtures thereof. Examples of organic bases include triethanolamine (TEA), diisopropanolamine, triisopropanolamine, aminomethyl propanol, aminomethylpropanediol, dimethylaminomethyl propanol, dodecylamine, cocamine, oleamine, morpholine, triamylamine, triethylamine, tetrakis(hydroxypropyl)ethylenediamine, L-arginine, aminomethyl propanol, tromethamine (2-amino 2-hydroxymethyl-1,3-propanediol), and PEG-15 cocamine.

Such pH modifiers may be present at from 0.0001 wt.% to 50 wt.%, based on the active component.

### Pearlescent/Opacifying Agents

Some formulations are often opacified by deliberately incorporating pearlescent materials therein to achieve a cosmetically attractive pearl-like appearance, known as pearlescence. An opacifier often is included in a composition to mask an undesirable aesthetic property, such as to improve the color of a composition that is darkened due to the presence of a particular ingredient, or to mask the presence of a particulate material in the composition. Opacifiers also are included in aqueous compositions to improve the aesthetics and consumer acceptance of an otherwise esthetically unpleasing composition. For example, an opacifier can impart a pearlescent appearance to a clear composition, thereby communicating an appearance of creaminess, mildness and body to the consumer. Persons skilled in the art are aware of problems faced by formulators in consistently preparing a stable pearlescent formulation. A detailed discussion is found in the article "Opacifiers and pearling agents in shampoos" by Hunting, Cosmetic and Toiletries, Vol. 96, pages 65-78 (July 1 981).

The opacifying or pearlescent material includes organic compounds and inorganic compounds. Typical examples of organic compounds are monoesters and/or diesters of ethylene glycol, propylene glycol, diethylene glycol, dipropylene glycol, triethylene glycol or tetraethylene glycol with fatty acids containing from about 6 to about 22 carbon atoms in one aspect, and from about 12 to about 18 carbon atoms in another aspect. Such fatty acids include caproic acid, caprylic acid, 2-ethyhexanoic acid, capric acid, lauric acid, isotridecanoic acid, myristic acid, palmitic acid, palmitoleic acid, stearic acid, isostearic acid, oleic acid, elaidic acid, petroselic acid, linoleic acid, linolenic acid, arachic acid, gadoleic acid, behenic acid, erucic acid, and mixtures thereof. In one aspect, ethylene glycol monostearate (EGMS) and/or ethylene glycol distearate (EGDS) and/or polyethylene glycol monostearate (PGMS) and/or polyethyleneglycol distearate (PGDS) are suitable pearlescent agents used in the composition.

Inorganic pearlescent agents include those selected from the group consisting of mica, metal oxide coated mica, silica coated mica, bismuth oxychloride coated mica, bismuth oxychloride, myristyl myristate, glass, metal oxide coated glass, various aluminum and magnesium salts, guanine, fish scales, glitter (polyester or metallic) and mixtures thereof.

Suitable micas include muscovite or potassium aluminum hydroxide fluoride. The platelets of mica are can be coated with a thin layer of metal oxide. Metal oxides are selected from the group consisting of rutile, titanium dioxide, ferric oxide, tin oxide, alumina, and mixtures thereof.

A representative listing of opacifiers is found in the CTFA Cosmetic Ingredient Handbook, J. Nikitakis, ed., 1988, at page 75. Other pearlescent or opacifying materials are disclosed in U.S. Pat. No. 4,654,207; U.S. Pat. No. 5,019,376; and U.S. Pat. No. 5,384,114.

In one aspect, the amount of the pearlescent or opacifying material can be used in amounts ranging from about 0.01 to about 10 wt.% in one aspect, from about 0.1% to about 5 wt.% in another aspect, and from 0.5 to about 3 wt.% in a further aspect, based upon the total weight of the composition.

### Other Insoluble Components

Other generally insoluble components suitable for use in the present compositions, UV absorbers, antibacterial compositions, anti-wrinkling and anti-aging compositions, microsponges, cosmetic beads and flakes. Cosmetic beads, flakes and capsules can be included in a composition for aesthetic appearance or can function as micro- and macro-encapsulants for the delivery of benefit agents to the hair and skin. Exemplary bead components include, but are not limited to, agar beads, alginate beads, jojoba beads, gelatin beads, Styrofoam ^{™} beads, polyacrylate, polymethylmethacrylate (PMMA), polyethylene beads, Unispheres^{™} and Unipearls^{™} cosmetic beads (Induchem USA, Inc., New York, NY), Lipocapsule^{™} Liposphere^{™}, and Lipopearl^{™} microcapsules (Lipo Technologies Inc., Vandalia, OH), and Confetti II^{™} dermal delivery flakes (United-Guardian, Inc., Hauppauge, NY).

The nature of the hydrophilic cosmetic active ingredient and/or adjuvant can be synthetic or natural, or come from a biotechnological procedure or from a combination of a synthetic procedure and a biotechnological procedure. Preferably, the hydrophilic active ingredient of the nanocapsules is thermolabile. A thermolabile active ingredient is understood to be that which presents a degradation equal or greater to 0.5% after having been subjected to a temperature of 80°C for two hours.

The cosmetic active ingredient is selected, without restriction, from the group formed by amino acids, peptides, proteins, hydrolized proteins, enzymes, coenzymes, hormones, vitamins, mineral salts, nucleotides, nucleic acids, molecules and extracts of biological and biotechnological origin, synthetic or partially synthetic hydrophilic molecules and/or mixtures thereof.

The amino acids, their salts and/or derivatives, as well as the commercial mixtures which contain them, are selected, for example and not restricted to, from the group formed by serine, proline, alanine, glutamate, arginine, glycine, methionine, citrulline, sodium methylglycine diacetate (TRILON^{®} M marketed by BASF), derivatives of amino acids which contain cysteine, in particular N-acetyl cysteine, ergothioneine or S-carboxymethylcysteine, and/or mixtures thereof.

The peptides or the commercial mixtures which contain them are selected, for example and not restricted to, from the group formed by peptides of cosmetic use, such as GHK [INCI: Tripeptide-1], acetyl-glutamyl-methionyl-alanyl-isoleucine, acetyl-arginyl-phenylglycyl-phenylglycine, Bodyfensine^{™} [INCI: Acetyl Dipeptide-3 Aminohexanoate], Relistase^{™} [INCI: Acetylarginyltriptophyl Diphenylglycine], acetyl-arginyl-phenylglycyl-valyl-glycine, acetyl-arginyl-phenylglycyl-valyl-phenylglycine, diaminopropionyl-alanyl-asparaginyl-histidine, acetyl-arginyl-asparaginyl-histidyl-citrulline-amide, Aldenine^{®} [INCI: Hydrolized Wheat Protein, Hydrolized Soy Protein, Tripeptide-1], Decorinyl^{®} [INCI: Tripeptide-10 Citrulline], Serilesine^{®} [INCI: hexapeptide-10], Peptide AC29 [INCI: Acetyl Tripeptide-30 Citrulline], Vilastene^{™} [INCI: Lysine HCl, Lecithin, Tripeptide-10 Citrulline], dGlyage^{™} [INCI: Lysine HCl, Lecithin, Tripeptide-9 Citrulline], Eyeseryl^{®} [INCI: Acetyl Tetrapeptide-5], Preventhelia^{®} [INCI: Diaminopropionoyl Tripeptide-33], Argireline^{®} [INCI: Acetyl Hexapeptide-8], SNAP-7 [INCI: Acetyl Heptapeptide-4], SNAP-8 [INCI: Acetyl Octapeptide-3], Leuphasyl^{®} [INCI: Pentapeptide-18], Trylagen^{®} [INCI: Pseudoalteromonas Ferment Extract, Hydrolyzed Wheat Protein, Hydrolyzed Soy Protein, Tripeptide-10 Citrulline, Tripeptide-1], Inyline^{™} [INCI: Acetyl Hexapeptide-30], Melatime^{™} [INCI: Acetyl Tripeptide-40], Thermostressine^{™} [INCI: Acetyl Tetrapeptide-22] or Liporeductyl^{®} [INCI: Caffeine, Butcherbroom (Ruscus Aculeatus) Root Extract, TEA-Hydroiodide, Carnitine, Ivy (Hedera Helix) Extract, Escin, Tripeptide-1] marketed by Lipotec, Matrixyl^{®} [INCI: Palmitoyl Pentapeptide-4], Matrixyl^{®} 3000 [INCI: Palmitoyl Tetrapeptide-7, Palmitoyl Oligopeptide], Dermaxyl^{®} [INCI: Palmitoyl Oligopeptide], Calmosensine^{™} [INCI: Acetyl Dipeptide-1], Biopeptide CL^{™} [INCI: Glyceryl Polymethacrylate, Propylene Glycol, Palmitoyl Oligopeptide] or Biopeptide EL^{™} [INCI: Palmitoyl Oligopeptide] marketed by Sederma, pseudodipeptides, IP 2000 [INCI: Dextran, Trifluoroacetyl Tripeptide-2] marketed by IEB and Atrium, Pepha^{®}-Timp [INCI: Human Oligopeptide-20], ECM-Protect^{®} [INCI: Water (Aqua), Dextran, Tripeptide-2] or Melanostatine^{®}-5 [INCI: Dextran, Nonapeptide-1] marketed by Atrium Innovations, Timp-Peptide [proposed INCI: Acetyl Hexapeptide], Bronzing S.F. [proposed INCI: Butiryl Pentapeptide], BONT-L-Peptide [INCI: Palmitoyl Hexapeptide-19] or ECM Moduline [proposed INCI: Palmitoyltripeptide] marketed by Infinitec Activos, IP2000 [INCI: Dextran, Trifluoroacetyl tripeptide-2] marketed by Institut Europeen de Biologie Cellulaire, Syn^{®}-Coll [INCI: Palmitoyl Tripeptide-5] marketed by Pentapharm, Neutrazen^{™} [INCI: Water, Butylene Glycol, Dextran, Palmitoyl Tripeptide-8], ChroNOline^{™} [INCI: Caprooyl Tetrapeptide-3] or Thymulen-4 [INCI: Acetyl Tetrapeptide-2] marketed by Atrium Innovations/Unipex Group, Meliprene^{®} [INCI: Dextran, Acetyl Heptapeptide-1] or Melitane^{®} [INCI: Acetyl Hexapeptide-1] marketed by Institut Européen de Biologie Cellulaire/Unipex Group, Skinasensyl^{™} [INCI: Acetyl Tetrapeptide-15] marketed by Laboratoires Serobiologiques/Cognis, Vialox^{®} [INCI: Pentapeptide-3], Syn^{®}-Ake^{®} [INCI: Dipeptide Diaminobutyroyl Benzylamide Diacetate], Syn^{®}-Coll [INCI: Palmitoyl Tripeptide-5], Syniorage^{™} [INCI: Acetyl Tetrapeptide-11], Dermican^{™} [INCI: Acetyl Tetrapeptide-9] marketed by Laboratoires Sérobiologiques/Cognis, Kollaren^{®} [INCI: Tripeptide-1, Dextran] marketed by Institut Europeen de Biologie Cellulaire, Collaxyl^{®} IS [INCI: Hexapeptide-9], Laminixyl IS^{™} [INCI: Heptapeptide], Quintescine^{™} IS [INCI: Dipeptide-4], UCPeptide^{™} V [INCI: Pentapeptide] or AT Peptide^{™} IS [INCI: Tripeptide-3] marketed by Vincience/ISP, glutathione, carnosine and/or mixtures thereof; and peptides of pharmaceutical use, such as glucagon, leuprolide, goserelin, triptorelin, buserelin, nafarelin, deslorelin, histrelin, avorelin, abarelix, cetrorelix, ganirelix, degarelix, desmopressin, somatostatin and analogues of somatostatin such as octreotide, vapreotide and lanreotide, among others.

The proteins, hydrolyzed protein, enzymes and hormones, as well as the commercial mixtures which contain them, are selected, for example and not restricted to, from the group formed by Elhibin^{®} [INCI: Glycine Soja (Soybean) Protein], Preregen^{®} [INCI: Glycine Soja (soybean) Protein, Oxido Reductases] or Regu^{®}-Age [INCI:Hydrolyzed Rice Bran Protein, Glycine Soja (Soybean) Protein, Oxido Reductases] marketed by Pentapharm/DSM, cadherins, integrins, selectins, hyaluronic acid receptors, immunoglobulins, fibroblast growth factor, connective tissue growth factor, platelet-derived growth factor, vascular endothelial growth factor, epidermal growth factor, insulin-like growth factor, keratinocyte growth factors, colony-stimulating growth factors, transforming growth factor-beta, tumor necrosis factor-alpha, interferons, interleukins, matrix metalloproteinases, receptor protein tyrosine phosphatases, protein hydrolysates, hydrolyzed vegetable proteins such as hydrolyzed wheat protein, hydrolyzed soy protein or hydrolyzed whey protein, Lipeptide [INCI: Hydrolized vegetable protein] by Lipotec, Collalift^{®} [INCI: Hydrolyzed Malt Extract ] marketed by Coletica/Engelhard, Colhibin [INCI: Hydrolyzed Rice Protein] marketed by Pentapharm, Cytokinol^{®} LS [INCI: Hydrolyzed Casein, Hydrolyzed Yeast Protein, Lysine HCL] marketed by Laboratoires Serobiologiques/Cognis, Liftline^{®} [INCI: Hydrolyzed wheat protein] or Ridulisse C^{®} [Hydrolyzed Soy Protein] marketed by Silab, catalase, superoxide dismutase, lactoperoxidase, glutathione peroxidase, lactoprotein, casein, lactoperoxidase, lysozyme, glycosidases, stratum corneum chymotryptic enzyme or SCCE, proteases such as trypsin, chymotrypsin, sutilain, papain or bromelain, DNA repair enzymes such as photolyase or T4 endonuclease V, lipase, luteinizing hormone (LH), follicle-stimulating hormone (FSH), growth hormone, insulin and/or mixtures thereof.

The vitamins are selected, for example and not restricted to, from the group formed by hydrosoluble vitamins, such as vitamin C, vitamin B1, vitamin B2, vitamin B3, vitamin B5, vitamin B6, vitamin B7, vitamin B9, vitamin B12, carnitine and/or mixtures thereof.

The extracts of biological or biotechnological origin, which can be chemically modified, as well as the commercial mixtures which contain them, are selected, for example and not restricted to, from the group formed by vegetable extracts, marine extracts, cell extracts and extracts produced by microorganisms.

The vegetable extracts are selected from the group formed by hydrosoluble vegetable extracts, for example and not restricted to, hydrosoluble extracts of chamomile, ivy, lemon, ginseng, raspberry, Roast *amaranth, Rehmannias radix,* gardenia, carrot, orange, peach, pineapple, gentian, hibiscus flower, walnut leaf, pumpkin, peony, quinoa, boldo, rough bindweed, salvia, pomegranate, oregano, ginger, marjoram, cranberry, grape, tomato, green tea, black tea, aloe vera (*Aloe Barbadensis*)*, Saphora japonica,* papaya, pineapple, pumpkin, sweet potato, *Bupleurum Chinensis, Cecropia Obtusifolia, Celosia Cristata, Centella Asiatica, Chenopodium Quinoa, Chrysanthellum Indicum, Citrus Aurantium Amara, Coffea Arabica, Coleus Forskohlii, Commiphora Myrrha, Crithmum Maritimum, Eugenia Caryophyllus, Ginkgo Biloba, Hedera Helix* (*ivy*)*, Hibiscus Sabdariffa, Ilex Paraguariensis, Laminaria Digitata, Nelumbium Speciosum, Paullinia Cupana, Peumus Boldus, Phyllacantha Fibrosa, Prunella Vulgaris, Prunus Amygdalus Dulcis, Ruscus Aculeatus* (Butcherbroom extract), *Sambucus Nigra, Spirulina Platensis Algae, Uncaria Tomentosa, Verbena Officinalis, Opuntia ficus indica, Salix alba, Lupinus spp., Secale cereale, Tussilago farfara, Achillea millefolium, Aradirachta indica, Asmuna japonica, Autocarpus incisus, Bidens pilosa, Broussonetia papyrifera, Chlorella vulgaris, Cimicifuga racemosa, Emblica officinalis, Glycyrrhiza glabra, Glycyrrhiza uralensis, Ilex purpurea, Ligusticum lucidum, Ligusticum wallichii, Mitracarpus scaber, Morinda citrifolia, Morus alba, Morus bombycis, Naringi crenulata, Prunus domesticus, Pseudostellariae radix, Rumex crispus, Rumex occidentalis, Sapindus mukurossi, Saxifragia sarmentosa, Scutellaria Galericulate, Sedum sarmentosum Bunge, Stellaria medica, Triticum Vulgare, Uva ursi, Whitania somnifera, Aristoloquia clematis, Rosa moschata, Echinacea angustifolia, Symphytum officinale, Equisetum arvense, Hypericum perforatum, Mimosa tenuiflora, Persea gratissima, Prunus africanum, Tormentilla erectea, Solanum tuberosum, Rosmarinus officinalis, Vaccinium angustifolium, Macrocystis pyrifera* algae, *Padina pavonica, Malpighia punicitolia, Cynara scolymus, Gossypium herbaceum, Panicum miliaceum, Morus nigra, Sesamum indicum, Glycine soja, Triticum vulgare, Glycine Max* (soy), malt, flax, red clover, kakkon-to, white lupin, hazelnut, maize, beech tree shoots, *Trifolium pratense* (red clover), *Phormium tenax* (New Zealand flax), *Cinnamommum zeylanicum, Laminaria saccharina, Spiraea ulmaria,* Nettle Root, *Pygeum africanum, Avena Sativa, Arnica montana, Cinchona succirubra, Eugenia caryophyllata, Humulus lupulus, Hypericum perforatum, Mentha piperita, Rosmarinus officinalis, Thymus vulgaricus,* plant extract of the genus *Silybum,* extract of legume seeds, extracts of red algae from the genus *Porphyra,* Phytovityl C^{®} [INCI: Aqua, Zea Mays Extract] marketed by Solabia, Micromerol^{™} [INCI: Pyrus Malus Extract] or Heather Extract [INCI: Calluna Vulgaris Extract] marketed by Coletica/Engelhard/BASF, Proteasyl^{®} TP LS8657 [INCI: Pisum Sativum Extract] marketed by Laboratoires Sérobiologiques/Cognis, Radicaptol [INCI: Propylene Glycol, Water, Passiflora Incarnata Flower Extract, Ribes Nigrum (Blackcurrant) Leaf Extract, Vitis Vinifera (grape) Leaf Extract] marketed by Solabia or ViaPure^{™} Boswellia [INCI: Olivanum (Boswellia Serrata) Extract] marketed by Soliance, EquiStat [INCI Pyrus Malus Fruit Extract, Glycine Soja Seed Extract] marketed by Coletica/Engelhard, Litchiderm^{™} [INCI: Litchi Chinensis pericarp extract] or Arganyl^{™} [INCI: Argania Spinosa Leaf Extract] marketed by Laboratories Sérobiologiques/Cognis, Dakaline [INCI: Prunus amygdalus dulcis, Anogeissus leiocarpus bark extract] marketed by Soliance, Actimp 1.9.3^{®} [INCI: Hydrolyzed Lupine Protein] marketed by Expanscience Laboratorios, Pronalen^{®} Refirming HSC [INCI: Triticum vulgare, Silybum Marianum, Glycine Soy, Equisetum Arvense, Alchemilla Vulgaris, Medicago Sativa, Raphanus Sativus] or Polyplant^{®} Refirming [INCI: Coneflower, Asiatic Centella, Fucus, Fenugreek] marketed by Provital, Lanablue^{®} [INCI: Sorbitol, Algae Extract] marketed by Atrium Innovations, Firmiderm^{®} LS9120 [INCI: Terminalia Catappa Leaf extract, Sambucus Negra Flower Extract, PVP, Tannic Acid] marketed by Laboratoires Serobiologiques/Cognis, among others.

Cell extracts and extracts produced by microorganisms, or commercial mixtures which contain them, are selected from the group formed by hydrosoluble cell extracts and hydrosoluble extracts produced by microorganisms, for example and not restricted to, Antarcticine^{®} [INCI: Pseudoalteromonas Ferment Extract] and Trylagen^{®} [INCI: Pseudoalteromonas Ferment Extract, Hydrolyzed Wheat Protein, Hydrolyzed Soy Protein, Tripeptide-10 Citrulline, Tripeptide-1] marketed by Lipotec, yeast extract, extract of *Saccharomyces cerivisiae* and the product of milk fermentation with *Lactobacillus Bulgaricus,* among others.

The amount of active ingredient contained in the delivery system ranges between 0.00001 and 50% in weight, preferably between 0.0001 and 40% in weight, and more preferably between 0.001 and 30% in weight.

The nanocapsules comprise other cosmetic and/or active ingredients and/or adjuvants of any nature, hydrophobes, hydrophiles and amphiphiles, which can be found inside the nanocapsules in solution or in suspension in the lipid matrix, or in the aqueous phase of the hair color composition. In particular, the cosmetic and/or alimentary active ingredients and/or adjuvants are selected, for example and not restricted to, from the group formed by surfactants, humectants or substances which retain moisture, moisturizers or emollients, agents stimulating healing, coadjuvant healing agents, agents stimulating re-epithelialization, coadjuvant re-epithelialization agents, agents which synthesize dermal or epidermal macromolecules, firming and/or redensifying and/or restructuring agents, cytokine growth factors, agents which act on capillary circulation and/or microcirculation, anti-glycation agents, free radical scavengers and/or anti-atmospheric pollution agents, reactive carbonyl species scavengers, 5α-reductase-inhibiting agents, lysyl- and/or prolyl hydroxylase inhibiting agents, defensin synthesis-stimulating agents, bactericidal agents and/or bacteriostatic agents and/or antimicrobial agents and/or germicidal agents and/or fungicidal agents and/or fungistatic agents and/or germ-inhibiting agents, anti-viral agents, antiparasitic agents, antihistaminic agents, NO-synthase inhibiting agents, desquamating agents or keratolytic agents and/or exfoliating agents, comedolytic agents, anti-psoriasis agents, anti-dandruff agents, anti-inflammatory agents and/or analgesics, anesthetic agents, anti-wrinkle and/or anti-aging agents, cosmetic and/or absorbent and/or body odor masking deodorants, antiperspirant agents, perfuming substances and/or perfumed oils and/or isolated aromatic compounds, anti-oxidizing agents, agents inhibiting vascular permeability, hydrolytic epidermal enzymes, whitening or skin depigmenting agents, agents inhibiting sweat-degrading enzymes, agents capable of filtering UV rays, agents which stimulate or regulate keratinocyte differentiation, anti-itching agents, agents which stimulate or inhibit the synthesis of melanin, propigmenting agents, self-tanning agents, melanocyte proliferation stimulating agent, liquid propellants, vitamins, amino acids, proteins, biopolymers, gelling polymers, skin relaxant agents, agents capable of reducing or treating bags under eyes, agents for the treatment and/or care of sensitive skin, astringent agents, agents regulating sebum production, anti-stretch mark agents, lipolytic agents or agents stimulating lipolysis, venotonic agents, anti-cellulite agents, calming agents, agents acting on cell metabolism, agents to improve dermal-epidermal junction, agents inducing hair growth or hair-loss retardants, body hair growth inhibiting or retardant agents, heat shock protein synthesis stimulating agents, muscle relaxants, muscle contraction inhibitory agents, agents inhibiting acetylcholine receptor clustering, anticholinergic agents, elastase inhibitory agents, matrix metalloproteinase inhibitory agents, chelating agents, vegetable extracts, essential oils, marine extracts, mineral salts, cell extracts, emulsifying agents, agents stimulating the synthesis of lipids and components of the stratum corneum (ceramides, fatty acids, etc.), agents obtained from a bio-fermentation process and/or mixtures thereof. The nature of these active ingredients and/or cosmetic and/or alimentary adjuvants can be synthetic or natural, such as vegetable extracts, or come from a biotechnological process or from a combination of a synthetic process and a biotechnological process. Additional examples can be found in the CTFA International Cosmetic Ingredient Dictionary & Handbook, 12th Edition (2008*).* In the context of the disclosed technology, a biotechnological process is understood to be any process which produces the active ingredient, or part of it, in an organism, or in a part of it.

The humectant or substance that retains moisture, moisturizer or emollient is selected, for example and not restricted to, from the group formed by polyols and polyethers such as glycerin, ethylhexylglycerin, caprylyl glycol, pentylene glycol, propylene glycol and their derivatives, Glycereth-26, Sorbeth-30; panthenol; pyroglutamic acid and its salts and derivatives; amino acids, such as serine, proline, alanine, glutamate or arginine; ectoine and its derivatives; *N*-(2-hydroxyethyl)acetamide; *N*-lauroyl-pyrrolidone carboxylic acid; *N*-lauroyl-L-lysine; *N*-alpha-benzoyl-L-arginine; urea; creatine; α- and β-hydroxy acids such as lactic acid, glycolic acid, malic acid, citric acid or salicylic acid, and their salts; polyglyceryl acrylate; sodium glucuronate, carraghenates (*Chondrus crispus*) or chitosan; glycosaminoglycans such as hyaluronic acid and derivatives thereof; aloe vera in any of its forms; honey; soluble collagen; lecithin and phosphatidylcholine; ceramides; cholesterol and its esters; tocopherol and its esters, such as tocopheryl acetate or tocopheryl linoleate; long-chain alcohols such as cetearyl alcohol, stearyl alcohol, cetyl alcohol, oleyl alcohol, isocetyl alcohol or octadecan-2-ol; long-chain alcohol esters such as lauryl lactate, myristyl lactate or C₁₂-C₁₅ alkyl benzoates; fatty acids such as stearic acid, isostearic acid or palmytic acid; polyunsaturated fatty acids (PUFAs); sorbitans such as sorbitan distearate; glycerides such as glyceryl monoricinoleate, glyceryl monostearate, glyceryl stearate citrate or caprylic and capric acid triglyceride; saccarose esters such as saccarose palmitate or saccarose oleate; butylene glycol esters, such as dicaprylate and dicaprate; fatty acid esters such as isopropyl isostearate, isobutyl palmitate, isocetyl stearate, isopropyl laurate, hexyl laurate, decyl oleate, cetyl palmitate, di-n-butyl sebacate, isopropyl myristate, isopropyl palmitate, isopropyl stearate, butyl stearate, butyl myristate, isopropyl linoleate, 2-ethylhexyl palmitate, 2-ethylhexyl cocoate, decyl oleate, myristyl myristate; squalene; mink oil; lanolin and its derivatives; acetylated lanolin alcohols; silicone derivatives such as cyclomethicone, dimethicone or dimethylpolysiloxane; Antarcticine^{®} [INCI: Pseudoalteromonas Ferment Extract] or acetyl-glutamyl-methionyl-alanyl-isoleucine, acetyl-arginyl-phenylglycyl-phenylglycine or acetyl-arginyl-6-aminohexanoyl-alanine marketed by Lipotec, petrolatum; mineral oil; mineral and synthetic waxes; beeswax (cera alba); paraffin; or waxes and oils with vegetable origins such as candelilla wax (*Euphorbia cerifera*)*,* carnauba wax (*Copernicia cerifera*)*,* shea butter (*Butirospermum parkii*)*,* cocoa butter (*Theobroma cacao*)*,* castor oil (*Ricinus communis*)*,* sunflower oil (*Helianthus annuus*)*,* olive oil (Olea europaea), coconut oil (*Cocos nucifera*)*,* palm oil (*Elaeis guineensis*)*,* wheat germ oil (*Triticum vulgare*)*,* sweet almond oil (*Prunus amygdalus dulces*)*,* musk rose oil *(Rosa moschata),* soya bean oil (*Glycine soja),* grape seed oil (*Vitis vinifera*)*,* calendula oil (*Calendula officinalis),* jojoba oil (*Simmonsis chinensis),* mango oil (*Mangifera indica*)*,* avocado oil (*Persea gratissima*)*,* and/or mixtures thereof, among others.

The bactericidal and/or bacteriostatic agent and/or antimicrobial and/or germicidal agent and/or fungicidal agent and/or fungistatic agent and/or germ inhibiting agent is selected, for example and not restricted to, from the group formed by macrolides, pyranosides, calcium channel blockers, for example and not restricted to, cinnarizine and diltiazem; hormones, for example and not restricted to, estril, analogues thereof or thyroxine and/or its salts, caprylyl glycol, imidazolidinyl urea, methyl 4-hydroxybenzoate [INCI: methylparaben], ethyl 4-hydroxybenzoate [INCI: ethylparaben], propyl 4-hydroxybenzoate [INCI: propylparaben], butyl 4-hydroxybenzoate [INCI: butylparaben], isobutyl 4-hydroxybenzoate [INCI: isobutylparaben], 1,3-bis(hydroxymethyl)-5,5-dimethylimidazolidine-2,4-dione [INCI: DMDM Hydantoin], benzyl 4-hydroxybenzoate [INCI: benzylparaben], benzyl alcohol, dehydroacetic acid, benzoic acid, sorbic acid, salicylic acid, formic acid, propionic acid, 2-bromo-2-nitropropane-1,3-diol, 3-*p*-chlorophenoxy-1,2-propanodiol [INCI: chlorphenesin], dichlorobenzyl alcohol, iodopropynyl butylcarbamate, benzalkonium chloride, odor-absorbing fungicides such as zinc ricinoleate, cyclodextrins, benzethonium chloride, chlorhexidine, ethanol, propanol, 1,3-butanediol, 1,2- propylene glycol, undecylenic acid, dehydroacetic acid, *N*-methylmorpholine acetonitrile (MMA), isopropanol, methanol, 1,2-hexanediol, 1,2-octanediol, pentylene glycol, glycerin laurate, glycerin caprilate, glycerin caprate, benzoyl peroxide, chlorhexidine gluconate, triclosan and derivatives thereof, phenoxyethanol, terpinen-4-ol, α-terpineol, resorcinol, stiemycin, erythromycin, neomycin, clindamycin and its esters, tetracyclines, metronidazole, azelaic acid, tolnaftate, nystatin, clotrimazole, ketoconazole, derivatives of zinc such as zinc piritionate or trithionate, zinc oxide and zinc undecylenate, piroctone olamine, isothiazolinones, selenium sulfur, benzyl hemiformal, boric acid, sodium borate, 6,6-dibromo-4,4-dichloro-2,2'-methylenediphenol [INCI: bromochlorophene], 5-bromo-5-nitro-1,3-dioxane, tosylchloramide sodium [INCI: chloramine T], chloroacetamide, p-chloro-m-cresol, 2-benzyl-4-chlorophenol [INCI: chlorophene], dimethyl oxazolidine, dodecyl dimethyl-2-phenoxyethyl ammonium bromide [INCI: domiphen bromide], 7-ethyl bicyclooxazolidine, hexetidine, glutaraldehyde, *N*-(4-chlorophenyl)-*N*-[4-chloro-3-(trifluoromethyl)phenyl]-urea [INCI: cloflucarban], 2-hydroxy-4-isopropyl-2,4,6-cycloheptatriene-1-one [INCI: Hinokitiol], isopropylmethylphenol, mercury salts, aluminum salts, nisin, phenoxyisopropanol, o-phenylphenol, 3-heptyl-2-[(3-heptyl-4-methyl-3*H*-thiazole-2-ylidene)methyl]-4-methylthiazole iodide [INCI: Quaternium-73], silver chloride, sodium iodide, thymol, undecylenic acid, diethylenetriaminepentaacetic acid, ethylenediaminetetraacetic acid and ethylenediaminetetraacetates, lactoperoxidase, glucose oxidase, lactoferrin, alkylaryl sulfonates, halogenated phenols, phenol mercury acetate and/or mixtures thereof, benzamidines, isothiazolines, derivatives of phthalimide, derivatives of pyridine, guanidines, quinolines, 1,2-dibromo-2,4-dicyanobutane, iodine-2-propylbutyl carbamate, iodine, tamed iodines, peroxo compounds, 4-chloro-3,5-dimethylphenol, 2,2'-methylene-bis(6-bromo-4-chlorophenol), 3-methyl-4-(1-methylethyl)phenol, 3-(4-chlorophenoxy)-1,2-propanediol, 3,4,4'-trichlorocarbanilide (TTC), thiamine essence, eugenol, farnesol, glycerin monolaurate, diglycerin monocaprinate, *N*-alkyl salicylic acid amides such as *n*-octyl salicylic acid amide or *n*-decyl salicylic acid amide, derivatives of halogenated xylene and cresol, such as *p*-chloro-meta-cresol or *p*-chloro-meta-xylene, extracts of *Allium sativum, Calendula officinalis, Chamomilla recutita, Echinacea Purpura, Hyssopus Officinalis, Melaleuca alternifolia* or tea tree oil, carnation essence, menthol and mint essence, among others.

The hair growth inducing agent, the agent which acts on capillary circulation and/or microcirculation, or the hair loss retardant agent is selected, for example and not restricted to, from the group formed by the extracts of *Tussilago farfara* or *Achillea millefolium,* nicotinic acid esters such as C₃-C₆ alkyl nicotinates such as methyl or hexyl nicotinate, benzyl nicotinate, or tocopheryl nicotinate; biotin, 5α-reductase-inhibiting agents, anti-inflammatory agents, retinoids, for example and not restricted to, all-trans-retinoic acid or tretinoin, isotretinoin, retinol or vitamin A, and derivatives thereof, such as zinc salt of acetate, palmitate, propionate, motretinide, etretinate and trans-retinoate; anti-bacterial agents, calcium channel blockers, for example and not restricted to, cinnarizine and diltiazem; hormones, for example and not restricted to, estriol, its analogues or thyroxine, its analogues and/or salts; antiandrogenic agents, for example and not restricted to, oxendolone, spironolactone or diethylstilbestrol; anti-radical agents, esterified oligosaccharides, for example and not restricted to, those described in documents EP 0 211 610 and EP 0 064 012; derivatives of hexosaccharic acids, for example and not restricted to, glucosaccharic acid or those described in document EP 0 375 388; glucosidase inhibitors, for example and not restricted to, D-glucaro-1,5-lactam or those described in document EP 0 334 586; glycosaminoglycanase and proteoglycanase inhibitors, for example and not restricted to L-galactono-1,4-lactone or those described in document EP 0 277 428; tyrosine kinase inhibitors, for example and not restricted to, 1-amido-1-cyano(3,4-dihydroxyphenyl)ethylene or those described in document EP 0403238, diazoxides, for example and not restricted to, 7-(acetylthio)-4',5'-dihydrospiro[androst-4-ene-17,2'-(3H)furan]-3-one, 1,1-dioxide of 3-methyl-7-chloro[2H]-1,2,4-benzothiadiazine or spirooxazine; phospholipids, for example and not restricted to, lecithin; salicylic acid and derivatives thereof, hydroxycarboxylic or keto carboxylic acids and esters thereof, lactones and their salts; anthralin, eicosa-5,8,11-trienoic acids and esters thereof or amides among others, minoxidil and derivatives or mixtures thereof.

The antioxidant is selected, for example and not restricted to, from the group formed by butylhydroxytoluene (BHT), butylhydroxyanisole (BHA), tert-butylhydroquinone (TBHQ), 2,6,-di-tert-butyl-4-methylphenol, gallic acid esters such as propyl gallate, probucol, polyphenoles, ascorbic acid and its salts, enzymes such as catalase, superoxide dismutase and peroxidases; citric acid, citrates, monoglyceride esters, calcium metabisulfate, lactic acid, malic acid, succinic acid, tartaric acid, vitamin A or β-carotene, vitamins E and C, tocopherols such as vitamin E acetate, ascorbic acid esters such as ascorbyl palmitate and ascorbyl acetate, zinc, copper, mannitol, reduced glutathione, carotenoids such as cryptoxanthin, astaxanthin and lycopene; cysteine, uric acid, carnitine, taurine, tyrosine, lutein, zeaxanthin, N-acetyl-cysteine, carnosine, γ-glutamylcysteine, quercetin, lactoferrin, dihydrolipoic acid, tea catechins, retinyl palmitate and derivatives thereof, bisulfate, metabisulfite and sodium sulfite, chromans, chromens and their analogues, Lipochroman-6 [INCI: Dimethylmethoxy Chromanol], chelating agents of metals such as EDTA, sorbitol, phosphoric acid or dGlyage^{™} [INCI: Lysine HCl, Lecithin, Tripeptide-9 Citrulline]; extract of *Ginkgo Biloba,* plant extracts such as sage, pomegranate, rosemary, oregano, ginger, marjoram, cranberry, grape, tomato, green tea or black tea; oleoresin extract, extract of plants which contain phenols such as vanillin, ellagic acid and resveratrol; tertiary butylhydroquinone or mixtures thereof, metal salts with a valence of 2 such as selenium, cadmium, vanadium or zinc; α-lipoic acid, coenzyme Q, idebenone or derivatives thereof.

The agent capable of filtering UV rays is selected, for example and not restricted to, from the group formed by organic or mineral photoprotective agents active against A and/or B ultraviolet rays such as substituted benzotriazoles, substituted diphenylacrylates, organic nickel complexes, umbelliferone, urocanic acid, biphenyl derivatives, stilbene, 3-benzylidene camphor, and derivatives thereof such as 3-(4-methylbenzylidene)camphor; derivatives of 4-aminobenzoic acid, 2-ethylhexyl 4-(dimethylamino)benzoate, 2-octyl 4-(dimethylamino)benzoate and amyl 4-(dimethylamino)benzoate; cinnamic acid esters, such as 2-ethylhexyl 4-methoxycinnamate or diethylamino hydroxybenzoyl hexyl benzoate, propyl 4-methoxycinnamate, isoamyl 4-methoxycinnamate, 2-ethylhexyl (octocrylenes) 2-cyano-3,3-phenyl cinnamate; salicylic acid esters, such as 2-ethylhexyl salicylate, 4-isopropylbenzyl salicylate, homomenthyl salicylate; benzophenone derivatives, such as 2-hydroxy-4-methoxybenzophenone, 2-hydroxy-4-methoxy-4'-methylbenzophenone, 2,2'-dihydroxy-4-methoxybenzophenone; benzalmalonic acid esters, such as di-2-ethylhexyl 4-methoxybenzalmalonate; triazine derivatives, such as 2,4,6-trianilino, p-carbo-2'-ethyl-1'-hexyloxy-1,3,5-triazine, octyl triazone or dioctyl butamido triazones; propane-1,3-diones, such as 1-(4-tert-butylphenyl)-3-(4'-methoxyphenyl)propane-1,3-dione; ketotricyclo(5.2.1.0)decane derivatives; 2-phenylbenzimidazole-5-sulfonic acid; benzophenone sulfonic acid derivatives, such as 2-hydroxy-4-methoxybenzophenone-5-sulfonic acid and its salts; 4-(2-oxo-3-bornylidenemethyl)benzenesulfonic acid, benzoyl methane derivatives, such as benzoyl methane 2-methyl-5-(2-oxo-3-bornylidene)sulfonic acid, such as 1-(4'-tert-butylphenyl)-3-(4'-methoxyphenyl)propane-1,3-dione, 4-tert-butyl-4'-methoxydibenzoylmethane, 1-phenyl-3-(4'-isopropylphenyl)-propane-1,3-dione, enamine compounds, anthranilates, silicons, benzimidazole derivatives, imidazolines, benzoyl derivatives, Chromabright^{™} [INCI: Dimethylmethoxy Chromanyl Palmitate] or Preventhelia^{®} [INCI: Diaminopropionoyl Tripeptide-33] both marketed by Lipotec, metal oxides such as zinc oxide, titanium, iron, zirconium, silicon, manganese, aluminum and cerium; silicates, talc, barium sulfate, zinc stearate, carbon nanotubes and/or mixtures thereof.

The following examples further describe and demonstrate embodiments within the scope of the present technology. These examples are presented solely for illustration and are not to be construed as limitations of the present technology since many variations thereof are possible without departing from the scope thereof. Unless otherwise specified weight percent (wt.%) is given in weight percent, based on the weight of the total composition. The amount of all ingredients reported in the tables in the Examples is "as supplied" by the manufacturer. Any ingredient not supplied as 100 percent active material is identified by the active material percentage supplied by the manufacturer. To calculate the amount of active ingredient utilized in the exemplified composition, multiply the active material percent by the total amount for an ingredient (as supplied). For example, if an ingredient as supplied by the manufacturer contains 30 wt. % of active polymer material the remainder is inert carrier component(s).

### Example 1 (PUD Synthesis)

Polytetrahydrofuran (PTHF) 1000 (1,536 grams), dicyclohexylmethane diisocyanate (H₁₂MDI) (747 grams) and isophorone diisocyanate (IPDI) (633 grams) were reacted at 215-225 °F (102-107 °C) under a blanket of dry nitrogen for about an hour. The reaction mixture was cooled to 170 °F (77 °C), and 314 grams Jeffcat^{™} DPA tethered amine monomer was added. The reaction mixture was stirred at 175-185 °F (79-85 °C) for 40 minutes to produce an NCO-terminated prepolymer. The mixture was cooled to 145 °F (63 °C), and 69 grams of glacial acetic acid were added over 15 minutes with agitation. A portion (3,060 grams) of the partially neutralized prepolymer was added with mixing to 4,000 g water at 65 °F (18 °C), which contained 98 grams of glacial acetic acid and 5 grams of DeeFo^{™} 97-3 defoamer, over the course of about 10-15 minutes to form an aqueous dispersion of cationic NCO-terminated polyurethane prepolymer. The remaining NCO was allowed to react with water overnight thereby producing a clean (no coagulum and floc) stable aqueous dispersion of cationic polyurethane having the following properties: total solids content - 43.6%, a pH 4.7, and Brookfield viscosity - 70 cP. Mean diameter of particle size distribution was 26 nm (measured by Malvern and reported as intensity-average Gaussian distribution). Weight-average molecular weight was measured at 42,500 g/mol. Ultimate tensile was measured at 3,750 psi (standard deviation = 170 psi), elongation at break - 620% (standard deviation = 30%), and modulus at 100% elongation - 1,025 psi (standard deviation = 35 psi).

### Example 2 (Permanent Hair Coloring Composition)

An oxidative hair coloring agent comprising oxidative dye precusors and dye couplers was formulated from the ingredients and amounts listed in Table 1. Formulation A was prepared with the tethered tertiary amine polyurethane of the present technology, while Formulations B and C were prepared with a nonionic polyurethane and cationic polyurethane, respectively. Formulation D was prepared using a benchmark ampholytic polymer commonly used in oxidative hair coloring system. Formulation D is a blank formulation containing no polymer.

**TABLE 1**

| Formulation | | A (wt.%) | B¹ (wt.%) | C¹ (wt.%) | D¹ (wt.%) | E¹ (wt.%) |
|---|---|---|---|---|---|---|
| Phase A | | | | | | |
| 1 | Deionized Water | 50.25 | 50.25 | 50.25 | 50.25 | 50.25 |
| 2 | Tetrasodium EDTA | 0.15 | 0.15 | 0.15 | 0.15 | 0.15 |
| 3 | Sodium Isoascorbate | 0.3 | 0.3 | 0.3 | 0.3 | 0.3 |
| 4 | Sodium Sulfite | 0.4 | 0.4 | 0.4 | 0.4 | 0.4 |
| 5 | PEG-3 Glyceryl Cocoate | 1 | 1 | 1 | 1 | 1. |
| 6 | Deionized Water | 10 | 10 | 10 | 10 | 10 |
| 7 | p-aminophenol, 3-methyl-4-aminophenol, m-aminophenol, resorcinol, 4-amino-2-hydroxytoluene | 3 | 3 | 3 | 3 | 3 |
| 8 | Deionized Water | 10 | 10 | 10 | 10 | 10. |
| 9 | Polymer of Example 1 (total solids) | 0.4 | 0 | 0 | 0 | 0 |
| 10 | NonionicPolyurethane (total solids) | 0 | 0.4 | 0 | 0 | 0 |
| 11 | Cationic Polyurethane (total solids) | 0 | 0 | 0.4 | 0 | 0 |
| 12 | Polyquaternium-22 (total solids) | 0 | 0 | 0 | 0.4 | 0 |
| | No Polymer | -- | -- | -- | -- | 0 |

| Phase B | | | | | | |
|---|---|---|---|---|---|---|
| 13 | Vegetable Oil | 4 | 4 | 4 | 4 | 4 |
| 14 | Cetearyl Alcohol | 4 | 4 | 4 | 4 | 4 |
| 15 | Cetyl Alcohol | 4.5 | 4.5 | 4.5 | 4.5 | 4.5 |
| 16 | Cetearyl Alcohol (and) Ceteareth-20 | 1 | 1 | 1 | 1 | 1 |
| 17 | Methyl Glucose Dioleate | 0.5 | 0.5 | 0.5 | 0.5 | 0.5 |
| 18 | Oleth-20 | 1.5 | 1.5 | 1.5 | 1.5 | 1.5 |

| Phase C | | | | | | |
|---|---|---|---|---|---|---|
| Formulation | | A (wt.%) | B¹ (wt.%) | C¹ (wt.%) | D¹ (wt.%) | E¹ (wt.%) |
| 19 | Acrylates/Beheneth-25 Methacrylate Copolymer | 3 | 3 | 3 | 3 | 3 |
| 20 | Parfum | 0.50 | 0.50 | 0.50 | 0.50 | 0.5 |
| 21 | Monoethanolamine (99.5%) | 5 | 5 | 5 | 5 | 5 |
| 22 | Glycerin (and) Water (and) Hibiscus Sabdariffa Flower Extract (and) Phenoxyethanol | 0.5 | 0.5 | 0.5 | 0.5 | 0.5 |
| pH | | 9.75 | 9.62 | 9.81 | 9.72 | 9.67 |
| Brookfield Viscosity² (mPa·s) | | 40,000 | 38,200 | 41,000 | 40,500 | 41,000 |

| | | | | | | |
|---|---|---|---|---|---|---|
| ¹Comparative formulations ²Brookfield^{®} viscometer Model DV-II+ Pro (at 25 °C at 10 rpm, Spindle 6) | | | | | | |

The ingredients for the permanent hair color composition were formulated according to the following procedure:
Add Deionized water to a beaker and start mixer at medium speed (500rpm).

Add ingredients No. 2, No. 3 and No. 4 and mix until homogenous.

In a separate beaker add PHASE A ingredients Nos. 5-7 and mix well. Add this to the batch.

Use deionized water ingredient No. 8 to rinse the beaker and add washings to the main batch.

Cover the batch with foil and do not let air enter. Blanket the batch with nitrogen to prevent oxidation.

Add PHASE A ingredients Nos. 9-12 to the main batch. Cover the batch to seal out air after addition of the ingredients. Mix until homogenous.

Start heating the batch to 65-70°C.

In a separate beaker add PHASE B ingredients and heat to 65-70°C.

Add this to the main batch and mix for 15 minutes at a high speed (900 rpm) until homogenous maintaining the temperature at 65-70°C. Cover the batch to seal out air after addition of PHASE B.

Start to cool the batch to 50°C. Keep the batch covered with foil.

Add PHASE C ingredient No. 19 at 50°C and mix until homogenous.

Cool the batch to 30°C.

Add PHASE C ingredients Nos. 20-22 one at a time and mix until homogenous. Cover the batch to seal out air after each addition. Blanket the batch with nitrogen to prevent oxidation.

Homogenize the batch with a laboratory homogenizer at 1000 rpm for 1-2 minutes.

### Dyeing Protocol

Each of the color formulations set forth in Table 1 were each mixed with a commercially available developer (Matrix 20V Developer) in a 1:1 weight ratio and immediately applied to pure white blond hair swatches (International Hair Importers) each measuring 6 inches long x ¾ inch wide and weighing 2.5g including the soft swatch binding. The swatches were prepared according to the following proceedure:
1. The hair swatches were placed on plastic wrap so as to not overlap one another. Five grams of the coloring composition was uniformly applied to one side of the swatch, followed by 5g applied to the opposite side. After applying the hair color composition to the swatches, the composition was worked into the hair using a color application brush.
2. The treated swatches were wrapped in plastic and developed for 30-minutes.
3. After 10-minutes the wrapped swatches were gently smoothed from root to tip with the fingers for 2 repetitions.
4. After 30-minutes (step 2) the treated swatches were rinsed with tap water running at 3.8L/min for 1-minute at a temperature of 38-40°C.
5. The rinsed swatches were then shampooed with a 10% sodium lauryl ether sulfate (SLES-2) solution in accordance with the following procedure:
6. Measure and record the dry weight of each pre-washed swatch. Place a weigh boat on the balance and tare. Wet swatch under deionized water with 10 strokes from root to tip. Lightly squeeze out excess water and place the swatch on the weigh boat. The dilution should be 1:5. Note the weight of the swatch.
7. Apply 0.4g of SLES-2 shampoo. Use 2-3 strokes with the fingers to distribute product along the length of the swatch, on both sides of the swatch.
8. Gently massage the product into the hair swatch for 40-seconds with 20 strokes (two-seconds per stroke). Massage the product unidirectionally from root to tip. Use horizontal as well as vertical shear to distribute the product. Flip the swatch after the first twenty seconds and massage to ensure proper distribution of the product.
9. Rinse the hair swatch thoroughly under tap water (shower mode) for 45-seconds. Set the water temperature at 38°C (± 2°C) and the flow rate at 3.8L/minute or 1gal/min. Massage the swatch during rinse by gently holding the swatch between the index finger and middle finger and passing the index finger and middle finger from the root of the swatch to the tip. Use twenty strokes to massage during rinse at the rate of 2-2.5 seconds per stroke.
10. Dry overnight in a humidity chamber at room temperature and 55-60% relative humidity.

### Color Measurement

Three swatches for each formulation were measured for color employing a Hunter LabScan^{™} XE colorimeter using the L, a, b scale. The color was measured on swatches treated with Formulations A, B, C, and D formulated in Table 1. In the Hunter L, a, b scale "L" is a measure of lightness and varies from 100 for perfect white to 0 for perfect black. Delta L corresponds to the difference color saturation (intensity) of the treated hair swatch before and after washing(s). A lower ΔL corresponds to a lesser amount of color dulling. "a" is a measure of redness, when the value of "a" is positive, and a measure of greenness, when negative. Delta "a" is a measure of how light or dark a color is before and after washing(s). A lower Δa is indicative of better color protection. "b" is a measure of yellowness, when the value of "b" is positive, and a measure of blueness, when negative. E (total color value) can be calculated as E = (L²+a²+b²)^{1/2}, a consequent ΔE is the total color change or difference in color in the treated hair before and after washing(s).

The L values of swatches after one wash were measured and recorded to check improvement in color intensity and saturation. The results are given in Table 2.

**TABLE 2**

| Delta E | Formulation A¹ | Formulation B¹ | Formulation C¹ | Formulation D' |
|---|---|---|---|---|
| 1 wash | 27.5 | 30.23 | 32.54 | 30.56 |

| | | | | |
|---|---|---|---|---|
| ¹Tested formulation was mixed at a 1:1 ratio (wt:wt) with developer | | | | |

Permanent hair color formulated with Formulation A comprising a polymer of the disclosed technology improves color intensity and saturation of colored hair. Without wishing to be bound by theory, it is believed that polymers of the disclosed technology assists in the penetration of dyes deep inside the hair cortex. A lower L value means that the colors are intense and vivid.

The permanent hair color formulated with the polymer of Example 1 protects color for up to 30 washes. Lower ΔE values are indicative that hair color is retained.

The ΔE, ΔL, and Δa were measured after 5, 10, 15, 20, 25 and 30 shampoo washes following the shampoo and drying procedures outlined in steps 5 and 6 in the foregoing dyeing protocol. The results are reported in Tables 3, 4, and 5.

**TABLE 3**

| Delta E | Formulation A¹ | Formulation B¹ | Formulation C¹ | Formulation D¹ |
|---|---|---|---|---|
| 5 washes | 2.61 | 4.5 | 5.7 | 6.5 |
| 10 washes | 4.2 | 7.2 | 6.9 | 7.3 |
| 15 washes | 5.4 | 8.9 | 10 | 8.96 |
| 20 washes | 4.92 | 9.1 | 10.5 | 9.4 |
| 25 washes | 5.71 | 10.2 | 10.8 | 11.04 |
| 30 washes | 6.2 | -- | -- | 12.46 |

| | | | | |
|---|---|---|---|---|
| ¹Tested formulation was mixed at a 1:1 ratio (wt:wt) with developer | | | | |

Permanent hair color formulated with Formulation A comprising a polymer of the disclosed technology improves color protection of colored hair. The permanent hair color formulated with the polymer of Example 1 protects color for up to 30 washes. Lower ΔE values mean that hair color is retained.

**TABLE 4**

| Delta L | Formulation A¹ | Formulation B¹ | Formulation C¹ | Formulation D' |
|---|---|---|---|---|
| 5 washes | 1.76 | 3.4 | 4.6 | 5.28 |
| 10 washes | 0.62 | 4.5 | 5.2 | 4.7 |
| 15 washes | 1.24 | 5.6 | 5.8 | 4.80 |
| 20 washes | 0.97 | 6.7 | 7.1 | 6.00 |
| 25 washes | 3.04 | 7.90 | 8.76 | 7.90 |
| 30 washes | 4.2 | -- | -- | 8.94 |

| | | | | |
|---|---|---|---|---|
| ¹Tested formulation was mixed at a 1:1 ratio (wt:wt) with developer | | | | |

The results indicate that color protection is achieved with Formulation A containing the polymer of Example 1 when compared to Formulation B containing a nonionic polyurethane, Formulation C containing a cationic polyurethane and formulation D containing a benchmark ampholytic polymer. Permanent hair color formulated with the polymer of Example 1 protects intensity and saturation of colored hair for up to 30 washes. Lower ΔL means that hair color intensity is retained.

**TABLE 5**

| Delta a | Formulation A' | Formulation B¹ | Formulation C¹ | Formulation D' |
|---|---|---|---|---|
| 5 washes | 1.09 | 2.7 | 3.10 | 2.15 |
| 10 washes | 3.43 | 4.86 | 4.90 | 4.18 |
| 15 washes | 4.11 | 5.72 | 6.11 | 5.17 |
| 20 washes | 3.94 | 6.54 | 7.34 | 5.11 |
| 25 washes | 3.78 | 7.76 | 8.12 | 5.21 |
| 30 washes | 4.03 | -- | -- | 5.90 |

| | | | | |
|---|---|---|---|---|
| ¹Tested formulation was mixed at a 1:1 ratio (wt:wt) with developer | | | | |

Better color protection is achieved with Formulation A containing the polymer of Example 1 when compared to Formulation B containing a nonionic polyurethane, Formulation C containing a cationic polyurethane and formulation D containing a benchmark ampholytic polymer. Permanent hair color formulated with the polymer of Example 1 protects tonality of colored hair for up to 30 washes. A lower Δa means that hair color tonality and value is retained.

The treated hair swatches were evaluated for conditioning properties in the wet and dry state. In wet state, wet detangling and combing tests were conducted to measure conditioning, and in dry state softness and smoothness of hair was measured by a co-efficient of friction test.

### Wet Detangling and Combing Test

Resistance to wet hair combing and detangling was tested on the treated swatches using a Dia-Stron MTT175 Mini Tensile Tester equipped with MTTWIN Version 5.0 software. Six swatches per treatment and 5 runs per swatch were run at a combing speed of 300mm/min and a maximum combing force (gmf) of 2000. A lower value of mean total work is indicative of an improvement in wet combing. A lower mean break load value represents improvement in wet detangling properties. Results are presented in Table 6.

**TABLE 6**

| | Formulation A¹ | Formulation D¹ | Formulation E¹ |
|---|---|---|---|
| Wet detangling (Mean break load) | 934.27 | 748 | 5300 |
| Wet combing (mean total work in joules) | 75.19 | 64.23 | 181 |

| | | | |
|---|---|---|---|
| ¹Tested formulation was mixed at a 1:1 ratio (wt:wt) with developer | | | |

Better wet detangling attributes was demonstrated by hair colored with Formulation A containing the polymer of Example 1 and Formulation D containing a benchmark ampholytic conditioning polymer when compared to control Formulation E containing no polymer. Lower mean break load values show improvement in wet detangling properties indicating that is easier to detangle hair following color treatment with a formulation of the present technology.

The results indicate better wet combing with Formulation A and the benchmark ampholytic polymer when compared to control formulated without a polymer. Lower values of mean total work are indicative of an improvement in wet combing as it is easier to comb hair following color treatment.

### Hair Softness and Smoothness Test

Hair softness was measured by the reduction of friction on the hair fiber surface with a Bruker Universal Mechanical Tester (UMT). The instrument facilitates sensitive testing of the coefficient of friction on a dry hair surface utilizing a sledging mode of the apparatus. Before testing swatches are washed twice utilizing the methodology set forth in steps 5 and 6 of the dyeing protocol. Measurements are taken on 3 treated swatches and compared to a control formulation (no polymer) and a benchmark polymer. Probe speed: 0.1mm/sec., probe travels against the hair cuticles comprising 1 cycle, 2 cylces are completed in 1 run and 2 runs are conducted on each swatch. The coefficient of friction is measured and recorded after each run. The results are presented in Table 7.

**TABLE 7**

| | Formulation A' | Formulation D' | Formulation E¹ |
|---|---|---|---|
| Softness of hair (Mean Coefficient of friction) | 0.015 | 0.024 | 0.05 |

| | | | |
|---|---|---|---|
| ¹Tested formulation was mixed at a 1:1 ratio (wt:wt) with developer | | | |

Permanent color formulated by adding the polymer of the present technology and benchmark polymer showed better improvement in dry properties compared to the control formulated with no polymer. Lower coefficient of friction values is indicative that the treated hair swatch is softer, smoother, and more moisturized.

### Example 3 (Semi-Permanent Hair Coloring composition: not within the scope of the invention)

A semi-permanent hair coloring agent comprising a semi-permanent acid dye was formulated from the ingredients and amounts listed in Table 8. Formulation F was prepared with the tethered tertiary amine polyurethane of the present technology, while Formulations G was prepared using a benchmark ampholytic polymer commonly used in a semi-permanent hair coloring system.

**TABLE 8**

| | Formulation | F | G¹ | H² |
|---|---|---|---|---|
| Phase A | | | | |
| 1 | Aqua | 63.89 | 63.89 | 63.89 |
| 2 | Acid Red 52 | 0.2 | 0.2 | 0.2 |
| 3 | Oleth-20 | 2 | 2 | 2 |
| 4 | Aqua | 5 | 5 | 5 |
| 5 | Aqua | 5 | 5 | 5 |
| 6 | Cocamidopropyl Betaine | 10 | 10 | 10 |
| 7 | DL-Panthenol | 0.05 | 0.05 | 0.05 |
| 8 | Polyurethane-10 | 0.4% | 0 | 0 |
| 9 | Polyquaternium-22 | 0 | 0.4% | 0 |
| 10 | PEG-20 Methyl Glucose Ether | 0.5 | 0.5 | 0.5 |

| Phase B | | | | |
|---|---|---|---|---|
| 11 | Cetyl Alcohol | 2 | 2 | 2 |
| 12 | Cetearyl Alcohol | 1.5 | 1.5 | 1.5 |
| 13 | Methyl Glucose Sesquistearate | 0.6 | 0.6 | 0.6 |
| 14 | PEG-20 Methyl Glucose Sesquistearate | 0.75 | 0.75 | 0.75 |
| 15 | Isostearyl Hydroxystearate | 1.5 | 1.5 | 1.5 |

| Phase C | | | | |
|---|---|---|---|---|
| 16 | Polyacrylate-1 Crosspolymer | 5 | 5 | 5 |
| 17 | Parfum | 0.5 | 0.5 | 0.5 |
| 18 | Tocopheryl Acetate | 0.01 | 0.01 | 0.01 |
| 19 | Hibiscus Rosa-Sinensis Flower Extract | 0.5 | 0.5 | 0.5 |
| 20 | Glycolic acid | 0.2 | 0.2 | 0.2 |
| | pH | 4.50 | 4.62 | 4.56 |
| | Brookfield Viscosity³ (mPa·s) | 9,000 | 8,500 | 9,200 |

| | | | | |
|---|---|---|---|---|
| ¹Benchmark formulation ²Control formulation ³Brookfield^{®} viscometer Model DV-II+ Pro (at 25 °C at 10rpm, Spindle 6) | | | | |

The ingredients for the semi-permanent hair color composition were formulated according to the following procedure:
Add ingredient No.1 to the main beaker and mix using a lightnin mixer.
Mix ingredient No. 2 with ingredient No. 3 and part of No. 4 in a small beaker and add it to the batch.

Use rest of DI water ingredient No. 4 to wash the beaker and add washings to the batch.

Add ingredients Nos. 5-10 one at a time and mix until homogenous.

Heat PHASE A to 70 °C.

In a separate container, mix PHASE B ingredients Nos. 11-15 and heat to 70 °C.

When both phases A and B are at 70 °C add PHASE B to PHASE A and mix for 10 minutes.

Cool to 50 °C and add PHASE C ingredient #16. Mix until homogenous.

Cool the batch to 30 °C.

Add PHASE C ingredients Nos. 17-19 one at a time and mix until homogenous.

Adjust pH to 4.5-4.8 using ingredient No. 20. Mix until homogenous.

### Color Measurement

Pure white blond hair swatches were treated with the semi-permanent hair coloring compositions formulated from the ingredients identified in Table 8 in a manner substantially similar to the treatment and color measurement protocols outlined in Example 2. Three treated swatches from each formulation were measured for color. The ΔE, L and a values after 5 and 10 washes are reported in Tables 9, 10, and 11, respectively.

**TABLE 9**

| Delta E | Formulation F | Formulation G |
|---|---|---|
| 5 washes | 10.65 | 12.91 |
| 10 washes | 20.28 | 25.33 |

Formulation F containing the polymer of Example 1 demonstrates better color protection when compared to the benchmark ampholytic polymer. Semi-permanent hair color formulated with the polymer of Example 1 improves color protection of colored hair. Semi-permanent hair color with the polymer of Example 1 protects color for up to at least 10 washes. Conventional semi-permanent hair coloring compositions generally protect up to 5 to 7 washes. The lower ΔE values indicate that more hair color is retained when compared to the benchmark standard.

**TABLE 10**

| Delta L | Formulation F | Formulation G |
|---|---|---|
| 5 washes | 7.61 | 10.33 |
| 10 washes | 12.21 | 16.1 |

Semi-permanent hair color compositions formulated with the polymer of Example 1 improves color protection of colored hair. Lower ΔL values are indicative of better hair color intensity and color fastness.

**TABLE 11**

| Delta a | Formulation F | Formulation G |
|---|---|---|
| 5 washes | 6.42 | 6.39 |
| 10 washes | 16.57 | 18.85 |

Semi-permanent hair color compositions formulated with the polymer of Example 1 improves color protection of colored hair. Formulation F protects color for at least 10 washes. Lower Δa values for Formulation F are indicative that hair color tonality is preserved and does not fade.

### Wet Detangling and Combing Test

Resistance to wet hair combing and detangling was tested on the treated swatches using the protocol described in Example 2. The results are set forth in Table 12.

**TABLE 12**

| | Formulation F | Formulation G | Formulation H |
|---|---|---|---|
| Wet detangling (Mean break load) | 298 | 136 | 530 |
| Wet combing (mean total work in joules) | 134 | 77 | 181 |

The results demonstrate that the Formulation F and G containing the polymer of Example 1 and the benchmark polymer, respectively, have better wet combing attributes than control Formulation H containing no polymer. Lower mean total work values indicate improvement in wet combing, as it is easier to comb the hair following the color treatment with Formulation F.

Treated hair using the color Formulations F and G containing the polymer of Example 1 and the benchmark polymer, respectively, demonstrate an improvement in wet detangling over hair colored with Formulation H (no polymer). Lower mean break load values are indicative of improvement in wet detangling attributes as it is easier to detangle hair following a color treatment with Formulations F and G compared with control Formulation H.

### Hair Softness and Smoothness Test

Hair softness of the colored hair swatches was evaluated by the protocol set forth in Example 2. The results are presented in Table 13.

**TABLE 13**

| | Formulation F | Formulation G | Formulation H |
|---|---|---|---|
| Softness of hair (Mean Co-efficient of friction) | 0.02 | 0.01 | 0.05 |

Semi-permanent color formulated with the polymer of Example 1 (Formulation F) and the benchmark polymer (Formulation G) showed better improvement in dry properties compared to the control (Formulation H). Lower coefficient of friction values is indicative that the treated hair swatch is softer, smoother, and more moisturized.

### Example 4

A temporary hair color was formulated from the ingredients and amounts shown in Table 14.

**TABLE 14**

| Ingredient | INCI Name | Wt. % |
|---|---|---|
| Deionized water | Aqua | 57.3 |
| Carbopol^{™} Style 2.0 Polymer | Carbomer | 1 |
| Propylene Glycol | Propylene Glycol | 20 |
| Phenoxyethanol | Phenoxyethanol | 0.5 |
| Cova pearl fire red 333 | Mica/Red Iron Oxide | 2.5 |
| SunCROMÀ^{™} red iron oxide C33-128 | Iron Oxide (CI77491) | 7.5 |
| Bentone^{™} 38 | Disteardimonium Hectorite | 5 |
| Silsense^{™} DW18 silicone | Dimethicone PEG-7 Isostearate | 2 |
| Schercemol 318 ester | Isopropyl Isostearate | 2 |
| Polymer | Polymer of Ex. 1 | 1 |
| AMP-ultra PC 2000 | Aminomethyl Propanol | 0.7 |
| Fragrance Herbal Essence | Parfum | 0.5 |
| pH | 6.5 | |
| Brookfied Viscosity¹ (mPa·s) | 20,000 | |

| | | |
|---|---|---|
| ¹Brookfield^{®} viscometer Model DV-II+ Pro (at 25 °C at 10 rpm, Spindle 6) | | |

### Example 5

An oxidative hair dye developer composition was formulated from the components listed in Table 15.

**TABLE 15**

| Ingredient | INCI Name | wt. % |
|---|---|---|
| Water Phase | | |
| Deionized Water | Aqua | 56.5 |
| Sodium Citrate | Sodium citrate | 0.4 |
| Sodium Stannate | Sodium Stannate | 0.4 |
| Sodium Pyrophosphate | Sodium Pyrophosphate | 0.3 |
| Pentasodium Pantetate | Pentasodium pantetate | 0.4 |
| Gluquat^{™} 125 | Lauryl Methyl Gluceth-10 Hydroxypropyldimonium Chloride | 1 |
| Schercodine^{™} C Amido-Amine | Cocamidopropyl Dimethylamine | 1 |
| Glucam^{™} P-20 Humectant | PPG-20 Methyl Glucose Ether | 0.5 |
| Phosphoric Acid | Phosphoric acid | 1 |
| Polymer | Polymer of Ex. 1 | 1 |

| Oil Phase | | |
|---|---|---|
| Coconut Oil | Cocus Nucifera oil | 1 |
| Cetearyl Alcohol | Cetearyl Alcohol | 4.5 |
| Cetyl Alcohol | Cetyl Alcohol | 5 |
| Glucate^{™} SS | Methyl Glucose Sesquistearate | 0.75 |
| Glucate DO | Methyl Glucose Dioleate | 0.75 |
| Chemonic^{™} OE-20 Ethoxylated Alcohol | Oleth-20 | 1.5 |

| Additives | | |
|---|---|---|
| Hydrogen Peroxide (50%) | Hydrogen peroxide | 24 |
| pH | 3.5 - 4 | |
| Brookfied Viscosity¹ (mPa·s) | 5,000 - 9,000 | |

| | | |
|---|---|---|
| ¹Brookfield^{®} viscometer Model DV-II+ Pro (at 25 °C at 10 rpm, Spindle 6) | | |

## Claims

1. A composition for coloring hair and retaining color in colored hair, said composition comprising:
a) at least one hair coloring agent; and
b) at least one polyurethane comprising a polyurethane backbone with one or more tethered tertiary amino groups laterally attached to said polyurethane backbone, wherein said tethered tertiary amino groups are situated away from said polyurethane backbone by a tethering moiety containing at least two intervening atoms, and wherein said tethered tertiary amino group(s) are optionally partially or fully neutralized and/or quaternized; and
wherein the at least one polyurethane comprises the reaction product of:
(i) at least one polyisocyanate;
(ii) at least one main chain polyamine, polyol, polythiols, and mixtures thereof having about two isocyanate reactive hydrogens; and
(iii) at least one compound containing a tertiary nitrogen having two isocyanate reactive hydrogen-containing substituents and a tethered tertiary amino group substituent, wherein said tertiary amino group is situated away from said tertiary nitrogen by a tethering moiety containing at least two intervening atoms.

2. A composition of claim 1, wherein said reaction product is partially or fully neutralized and/or quaternized, preferably wherein said partially or fully neutralized and/or quaternized reaction product is dispersed in water to form a polyurethane prepolymer dispersion.

3. A composition of claim 1 or claim 2, wherein said at least one compound containing a tertiary nitrogen (iii) is selected from 2,2'-((3-dimethylamino)propyl)azanediyl)bis(ethan-1-ol) and 1,1'-((3-dimethylamino)propyl)azanediyl)bis(propan-2-ol), and mixtures thereof.

4. A composition of any of the preceding claims, wherein the at least one polyurethane comprises the reaction product of:
(i) at least one aliphatic polyisocyanate;
(ii) at least one main chain polyol having about two isocyanate reactive hydrogens selected from a polyether; and
(iii) at least one compound containing a tertiary nitrogen having two isocyanate reactive hydrogen-containing substituents and a tethered tertiary amino group substituent, wherein said tertiary amino group is situated away from said tertiary nitrogen by a tethering moiety containing at least three intervening atoms; preferably, wherein said at least one aliphatic polyisocyanate is selected from hexamethylene-1,6-diisocyanate, 1,12-dodecane diisocyanate, 2,2,4-trimethyl-hexamethylene diisocyanate, 2,4,4-trimethyl-hexamethylene diisocyanate, 2-methyl-1,5-pentamethylene diisocyanate, lysine diisocyanate, dicyclohexylmethane diisocyanate, isophorone diisocyanate, cyclohexane diisocyanate, bis-(isocyanatomethyl) cyclohexane, methylcyclohexane diisocyanate, cyclohexane triisocyanate, and mixtures thereof.

5. A composition of any of the preceding claims, wherein said at least one main chain polyol is selected from polytetrahydrofuran, poly(propylene glycol) derived from 1,2-propane diol, poly(propylene glycol derived from 1,3-propane diol, and mixtures thereof.

6. A composition of any of the preceding claims, wherein said tethered tertiary amino groups are neutralized with an acid or wherein said tethered tertiary amino groups are quaternized with a quaternizing agent selected from alkyl halides, aralkyl halides, dialkyl carbonates, dialkyl sulphates, and epoxides.

7. A composition of any of the preceding claims, wherein at least 25%, or at least 50%, or at least 75%, or at least 80%, or at least 85%, or at least 90%, or at least 95% of said tethered tertiary amino groups are neutralized and/or quaternized.

8. A composition of any of the preceding claims, wherein the at least one polyurethane comprises the reaction product of at least one cycloaliphatic diisocyanate, at least one polyether polyol, and at least one tethered tertiary amino group monomer containing two active hydrogen groups, wherein said tertiary amino groups are neutralized with an acid; or wherein the at least one polyurethane comprises the reaction product of a first cycloaliphatic diisocyanate, a second cycloaliphatic diisocyanate different from the first cycloaliphatic diisocyanate, at least one polyether polyol, and at least one tethered tertiary amino group containing two active hydrogen groups, wherein said tertiary amino groups are neutralized with an acid.

9. A composition of any of the preceding claims, wherein the at least one polyurethane comprises the reaction product of dicyclohexylmethane diisocyanate (H₁₂MDI) and isophorone diisocyanate (IPDI), polytetrahydrofuran, at least one tethered tertiary amino group selected from 2,2'-((3-dimethylamino)propyl)azanediyl)bis(ethan-1-ol) and 1,1'-((3-dimethylamino)propyl)azanediyl)bis(propan-2-ol), and mixtures thereof, wherein said tertiary amino groups are neutralized with an acid.

10. A composition of any of the preceding claims, wherein said at least one polyurethane is a polyurethane dispersion (PUD).

11. A composition of any of the preceding claims, wherein said hair coloring agent is a temporary hair dye, a semi-permanent hair dye or comprises at least one permanent hair dye component, and wherein said hair coloring agent is present in an amount ranging from 0.0005 to 20 wt.%, or from 0.005 to 10 wt.%, or from 0.05 to 6 wt.%, based on the total weight of the composition.

12. A composition of any of the previous claims, comprising at least one additional cationic polymer, at least one ampholytic polymer, and mixtures thereof, wherein said at least one additional cationic polymer or at least one amphoteric polymer is present in an amount ranging from 0.001 to 20 wt.% based on the total weight of the composition; or comprising at least one surfactant selected from anionic, cationic, amphoteric, and nonionic surfactants, and mixtures thereof, wherein said at least one surfactant is present in an amount ranging from 0.001 to 40 wt. % based on the total weight of the composition; or comprising at least one thickening agent selected from cellulose derivatives, guar derivatives, gums of microbial origin, casein, alginates, Carbomers, crosslinked acrylic acid copolymers, and mixtures thereof, wherein said at least one thickening agent is present in an amount ranging from 0.001 to 30 wt. % based on the total weight of the composition.

13. A composition of any of the previous claims, wherein said at least one water dispersible polyurethane is present in an amount ranging from 0.004 to 4 wt.%, or from 0.2 to 2.4 wt.%, or from 0.3 to 1.2 wt. % based on the total weight of the composition.

14. A process for coloring hair and retaining color fastness in colored hair, said process comprising contacting hair with the composition of any one of claims 1 to 13.

15. Use of a composition of claims 1 to 13 for coloring hair and prolonging the color fastness of said colored hair.

## Patentansprüche

1. Zusammensetzung zum Färben von Haaren und zum Erhalten von Farbe in gefärbten Haaren, die Zusammensetzung umfassend:
(a) mindestens ein Haarfärbemittel; und
(b) mindestens ein Polyurethan, umfassend ein Polyurethan-Rückgrat mit einer oder mehreren angebundenen tertiären Aminogruppen, die seitlich an das Polyurethan-Rückgrat angehängt sind, wobei die angebundenen tertiären Aminogruppen von dem Polyurethan-Rückgrat durch einen Anbindungsrest entfernt angeordnet sind, der mindestens zwei dazwischenliegende Atome enthält, und wobei die angebundene(n) tertiäre(n) Aminogruppe(n) optional teilweise oder vollständig neutralisiert und/oder quaternisiert sind; und
wobei das mindestens eine Polyurethan das Reaktionsprodukt umfasst von:
(i) mindestens einem Polyisocyanat;
(ii) mindestens einem Hauptketten-Polyamin, Polyol, Polythiol und Mischungen davon, die etwa zwei isocyanatreaktive Wasserstoffe aufweisen; und
(iii) mindestens einer Verbindung, die einen tertiären Stickstoff enthält, der zwei Isocyanat-reaktive Wasserstoff enthaltende Substituenten und einen angebundenen tertiären Aminogruppensubstituenten aufweist, wobei sich die tertiäre Aminogruppe von dem tertiären Stickstoff entfernt befindet, durch einen Anbindungsrest, der mindestens zwei dazwischenliegende Atome enthält.

2. Zusammensetzung nach Anspruch 1, wobei das Reaktionsprodukt teilweise oder vollständig neutralisiert und/oder quaternisiert ist, vorzugsweise wobei das teilweise oder vollständig neutralisierte und/oder quaternisierte Reaktionsprodukt in Wasser dispergiert wird, um eine Polyurethan-Prepolymer-Dispersion auszubilden.

3. Zusammensetzung nach Anspruch 1 oder 2, wobei die mindestens eine Verbindung, die einen tertiären Stickstoff enthält, (iii) ausgewählt ist aus 2,2'-((3-Dimethylamino)propyl)azanediyl)bis(ethan-1-ol) und 1,1'-((3-Dimethylamino)propyl)azanediyl)bis(propan-2-ol) und Mischungen davon.

4. Zusammensetzung nach einem der vorstehenden Ansprüche, wobei das mindestens eine Polyurethan das Reaktionsprodukt umfasst von:
(i) mindestens einem aliphatischen Polyisocyanat;
(ii) mindestens einem Hauptketten-Polyol, das etwa zwei Isocyanat-reaktive Wasserstoffe aufweist, ausgewählt aus einem Polyether; und
(iii) mindestens einer Verbindung, die einen tertiären Stickstoff enthält, der zwei Isocyanat-reaktive Wasserstoff enthaltende Substituenten und einen angebundenen tertiären Aminogruppensubstituenten aufweist, wobei sich die tertiäre Aminogruppe von dem tertiären Stickstoff entfernt befindet, durch einen Anbindungsrest, der mindestens drei dazwischenliegende Atome enthält; vorzugsweise, wobei das mindestens eine aliphatische Polyisocyanat ausgewählt ist aus Hexamethylen-1,6-diisocyanat, 1,12-Dodecandiisocyanat, 2,2,4-Trimethyl-hexamethylendiisocyanat, 2,4,4-Trimethyl-hexamethylendiisocyanat, 2-Methyl-1,5-pentamethylendiisocyanat, Lysindiisocyanat, Dicyclohexylmethandiisocyanat, Isophorondiisocyanat, Cyclohexandiisocyanat, Bis-(isocyanatomethyl)cyclohexan, Methylcyclohexandiisocyanat, Cyclohexantriisocyanat und Mischungen davon.

5. Zusammensetzung nach einem der vorstehenden Ansprüche, wobei das mindestens eine Hauptketten-Polyol ausgewählt ist aus Polytetrahydrofuran, Poly(propylenglykol), das von 1,2-Propandiol abgeleitet ist, Poly(propylenglykol), das von 1,3-Propandiol abgeleitet ist, und Mischungen davon.

6. Zusammensetzung nach einem der vorstehenden Ansprüche, wobei die angebundenen tertiären Aminogruppen mit einer Säure neutralisiert sind oder wobei die angebundenen tertiären Aminogruppen mit einem Quaternisierungsmittel quaternisiert sind, das ausgewählt ist aus Alkylhalogeniden, Aralkylhalogeniden, Dialkylcarbonaten, Dialkylsulfaten und Epoxiden.

7. Zusammensetzung nach einem der vorstehenden Ansprüche, wobei mindestens 25 %, oder mindestens 50 %, oder mindestens 75 %, oder mindestens 80 %, oder mindestens 85 %, oder mindestens 90 %, oder mindestens 95 % der angebundenen tertiären Aminogruppen neutralisiert und/oder quaternisiert sind.

8. Zusammensetzung nach einem der vorstehenden Ansprüche, wobei das mindestens eine Polyurethan das Reaktionsprodukt von mindestens einem cycloaliphatischen Diisocyanat, mindestens einem Polyetherpolyol und mindestens einem angebundenen tertiären Aminogruppen-Monomer umfasst, das zwei aktive Wasserstoffgruppen enthält, wobei die tertiären Aminogruppen mit einer Säure neutralisiert sind; oder wobei das mindestens eine Polyurethan das Reaktionsprodukt eines ersten cycloaliphatischen Diisocyanats, eines zweiten cycloaliphatischen Diisocyanats, das sich von dem ersten cycloaliphatischen Diisocyanat unterscheidet, mindestens eines Polyetherpolyols und mindestens einer angebundenen tertiären Aminogruppe umfasst, die zwei aktive Wasserstoffgruppen enthält, wobei die tertiären Aminogruppen mit einer Säure neutralisiert sind.

9. Zusammensetzung nach einem der vorstehenden Ansprüche, wobei das mindestens eine Polyurethan das Reaktionsprodukt von Dicyclohexylmethandiisocyanat (H₁₂MDI) und Isophorondiisocyanat (IPDI), Polytetrahydrofuran, mindestens einer angebundenen tertiären Aminogruppe ausgewählt aus 2,2'-((3-Dimethylamino)propyl)azanediyl)bis(ethan-1-ol) und 1,1'-((3-Dimethylamino)propyl)azanediyl)bis(propan-2-ol), und Mischungen davon umfasst, wobei die tertiären Aminogruppen mit einer Säure neutralisiert sind.

10. Zusammensetzung nach einem der vorstehenden Ansprüche, wobei das mindestens eine Polyurethan eine Polyurethandispersion (PUD) ist.

11. Zusammensetzung nach einem der vorstehenden Ansprüche, wobei das Haarfärbemittel ein temporärer Haarfarbstoff, ein semipermanenter Haarfarbstoff ist oder mindestens eine permanente Haarfarbstoffkomponente umfasst, und wobei das Haarfärbemittel in einer Menge in einem Bereich von 0,0005 bis 20 Gew.-%, oder von 0,005 bis 10 Gew.-%, oder von 0,05 bis 6 Gew.-% vorhanden ist, bezogen auf das Gesamtgewicht der Zusammensetzung.

12. Zusammensetzung nach einem der vorstehenden Ansprüche, umfassend mindestens ein zusätzliches kationisches Polymer, mindestens ein ampholytisches Polymer und Mischungen davon, wobei das mindestens eine zusätzliche kationische Polymer oder mindestens eine amphotere Polymer in einer Menge in einem Bereich von 0,001 bis 20 Gew.-% vorhanden ist, bezogen auf das Gesamtgewicht der Zusammensetzung; oder umfassend mindestens ein Tensid ausgewählt aus anionischen, kationischen, amphoteren und nichtionischen Tensiden und Mischungen davon, wobei das mindestens eine Tensid in einer Menge in einem Bereich von 0,001 bis 40 Gew.-% vorhanden ist, bezogen auf das Gesamtgewicht der Zusammensetzung; oder umfassend mindestens ein Verdickungsmittel ausgewählt aus Cellulosederivaten, Guarderivaten, Gummis mikrobiellen Ursprungs, Casein, Alginaten, Carbomeren, vernetzten Acrylsäure-Copolymeren und Mischungen davon, wobei das mindestens eine Verdickungsmittel in einer Menge in einem Bereich von 0,001 bis 30 Gew.-% vorhanden ist, bezogen auf das Gesamtgewicht der Zusammensetzung.

13. Zusammensetzung nach einem der vorstehenden Ansprüche, wobei das mindestens eine wasserdispergierbare Polyurethan in einer Menge von 0,004 bis 4 Gew.-% oder von 0,2 bis 2,4 Gew.-% oder von 0,3 bis 1,2 Gew.-% vorhanden ist, bezogen auf das Gesamtgewicht der Zusammensetzung.

14. Prozess zum Färben von Haaren und zum Erhalten von Farbechtheit in gefärbtem Haar, der Prozess umfassend ein Inberührungbringen von Haaren mit der Zusammensetzung nach einem der Ansprüche 1 bis 13.

15. Verwendung einer Zusammensetzung nach den Ansprüchen 1 bis 13 zum Färben von Haaren und zum Verlängern der Farbechtheit der gefärbten Haare.

## Revendications

1. Composition permettant de colorer des cheveux et de conserver la couleur dans les cheveux colorés, ladite composition comprenant :
(a) au moins un agent de coloration des cheveux ; et
(b) au moins un polyuréthane comprenant un squelette de polyuréthane avec un ou plusieurs groupes amino tertiaires attachés, fixés latéralement audit squelette de polyuréthane, dans laquelle lesdits groupes amino tertiaires attachés sont situés à l'écart dudit squelette de polyuréthane par un groupement d'attache contenant au moins deux atomes intermédiaires, et dans laquelle ledit ou lesdits groupes amino tertiaires attachés sont facultativement partiellement ou totalement neutralisés et/ou quaternisés ; et
dans laquelle l'au moins un polyuréthane comprend le produit de réaction de :
(i) au moins un polyisocyanate ;
(ii) au moins une polyamine à chaîne principale, un polyol, des polythiols, et des mélanges de ceux-ci ayant environ deux hydrogènes réactifs aux isocyanates ; et
(iii) au moins un composé contenant un azote tertiaire ayant deux substituants contenant de l'hydrogène réactif aux isocyanates et un substituant de groupe amino tertiaire attaché, dans laquelle ledit groupe amino tertiaire est situé à l'écart dudit azote tertiaire par un groupement d'attache contenant au moins deux atomes intermédiaires.

2. Composition selon la revendication 1, dans laquelle ledit produit de réaction est partiellement ou totalement neutralisé et/ou quaternisé, de préférence dans laquelle ledit produit de réaction partiellement ou totalement neutralisé et/ou quaternisé est dispersé dans de l'eau afin de former une dispersion de prépolymère de polyuréthane.

3. Composition selon la revendication 1 ou la revendication 2, dans laquelle ledit au moins un composé contenant un azote tertiaire (iii) est choisi parmi 2,2'-((3-diméthylamino)propyl)azanediyl)bis(éthan-1-ol) et 1,1'-((3-diméthylamino)propyl)azanediyl)bis(propan-2-ol), et mélanges de ceux-ci.

4. Composition selon l'une quelconque des revendications précédentes, dans laquelle l'au moins un polyuréthane comprend le produit de réaction de :
(i) au moins un polyisocyanate aliphatique ;
(ii) au moins un polyol de chaîne principale ayant environ deux hydrogènes réactifs aux isocyanates choisis parmi un polyéther ; et
(iii) au moins un composé contenant un azote tertiaire ayant deux substituants contenant de l'hydrogène réactif aux isocyanates et un substituant de groupe amino tertiaire attaché, dans laquelle ledit groupe amino tertiaire est situé à l'écart dudit azote tertiaire par un groupement d'attache contenant au moins trois atomes intermédiaires ; de préférence, dans lequel ledit au moins un polyisocyanate aliphatique est choisi parmi 1,6-diisocyanate d'hexaméthylène, diisocyanate de 1,12-dodécane, diisocyanate de 2,2,4-triméthyl-hexaméthylène, diisocyanate de 2,4,4-triméthyl-hexaméthylène, diisocyanate de 2-méthyl-1,5-pentaméthylène, diisocyanate de lysine, diisocyanate de dicyclohexylméthane, diisocyanate d'isophorone, diisocyanate de cyclohexane, bis-(isocyanatométhyl)cyclohexane, diisocyanate de méthylcyclohexane, triisocyanate de cyclohexane, et mélanges de ceux-ci.

5. Composition selon l'une quelconque des revendications précédentes, dans laquelle ledit au moins un polyol de chaîne principale est choisi parmi polytétrahydrofurane, poly(propylène glycol) dérivé de 1,2-propane diol, poly(propylène glycol dérivé de 1,3-propane diol, et mélanges de ceux-ci.

6. Composition selon l'une quelconque des revendications précédentes, dans laquelle lesdits groupes aminés tertiaires attachés sont neutralisés par un acide ou dans laquelle lesdits groupes aminés tertiaires attachés sont quaternisés avec un agent quaternisant choisi parmi halogénures d'alkyle, halogénures d'aralkyle, carbonates de dialkyle, sulfates de dialkyle, et époxydes.

7. Composition selon l'une quelconque des revendications précédentes, dans laquelle au moins 25 %, ou au moins 50 %, ou au moins 75 %, ou au moins 80 %, ou au moins 85 %, ou au moins 90 %, ou au moins 95 % desdits groupes aminés tertiaires attachés sont neutralisés et/ou quaternisés.

8. Composition selon l'une quelconque des revendications précédentes, dans laquelle l'au moins un polyuréthane comprend le produit de réaction d'au moins un diisocyanate cycloaliphatique, d'au moins un polyol de polyéther, et d'au moins un monomère de groupe amino tertiaire attaché contenant deux groupes hydrogène actifs, dans laquelle lesdits groupes amino tertiaires sont neutralisés avec un acide ; ou dans laquelle l'au moins un polyuréthane comprend le produit de réaction d'un premier diisocyanate cycloaliphatique, d'un second diisocyanate cycloaliphatique différent du premier diisocyanate cycloaliphatique, d'au moins un polyol de polyéther, et d'au moins un groupe amino tertiaire attaché contenant deux groupes hydrogène actifs, dans laquelle lesdits groupes amino tertiaires sont neutralisés avec un acide.

9. Composition selon l'une quelconque des revendications précédentes, dans laquelle l'au moins un polyuréthane comprend le produit de réaction de diisocyanate de dicyclohexylméthane (H₁₂MDI) et de diisocyanate d'isophorone (IPDI), de polytétrahydrofurane, d'au moins un groupe amino tertiaire attaché choisi parmi 2,2'-((3-diméthylamino)propyl)azanediyl)bis(éthan-1-ol) et 1,1'-((3-diméthylamino)propyl)azanediyl)bis(propan-2-ol), et mélanges de ceux-ci, dans laquelle lesdits groupes amino tertiaires sont neutralisés avec un acide.

10. Composition selon l'une quelconque des revendications précédentes, dans laquelle ledit au moins un polyuréthane est une dispersion de polyuréthane (PUD).

11. Composition selon l'une quelconque des revendications précédentes, dans laquelle ledit agent de coloration des cheveux est une teinture capillaire temporaire, une teinture capillaire semi-permanente ou comprend au moins un composant de teinture capillaire permanente, et dans laquelle ledit agent de coloration des cheveux est présent en une quantité allant de 0,0005 à 20 % en poids, ou de 0,005 à 10 % en poids, ou de 0,05 à 6 % en poids, sur la base du poids total de la composition.

12. Composition selon l'une quelconque des revendications précédentes, comprenant au moins un polymère cationique supplémentaire, au moins un polymère ampholyte, et des mélanges de ceux-ci, dans laquelle ledit au moins un polymère cationique supplémentaire ou au moins un polymère amphotère est présent en une quantité comprise dans une plage allant de 0,001 à 20 % en poids sur la base du poids total de la composition ; ou comprenant au moins un agent tensioactif choisi parmi des agents tensioactifs anioniques, cationiques, amphotères, et non ioniques, et mélanges de ceux-ci, dans laquelle ledit au moins un agent tensioactif est présent en une quantité comprise dans une plage allant de 0,001 à 40 % en poids sur la base du poids total de la composition ; ou comprenant au moins un agent épaississant choisi parmi dérivés de cellulose, dérivés de guar, gommes d'origine microbienne, caséine, alginates, carbomères, copolymères d'acide acrylique réticulé, et mélanges de ceux-ci, dans laquelle ledit au moins un agent épaississant est présent en une quantité comprise dans une plage allant de 0,001 à 30 % en poids sur la base du poids total de la composition.

13. Composition selon l'une quelconque des revendications précédentes, dans laquelle ledit au moins un polyuréthane dispersible dans l'eau est présent en une quantité comprise dans une plage allant de 0,004 à 4 % en poids, ou de 0,2 à 2,4 % en poids, ou de 0,3 à 1,2 % en poids sur la base du poids total de la composition.

14. Procédé permettant de colorer des cheveux et de conserver la tenue des couleurs des cheveux colorés, ledit procédé comprenant la mise en contact des cheveux avec la composition selon l'une quelconque des revendications 1 à 13.

15. Utilisation d'une composition selon les revendications 1 à 13 permettant de colorer des cheveux et de prolonger la tenue des couleurs desdits cheveux colorés.
